(19) Europäisches Patentamt
European Patent Office
Office européen des brevets

(11) **EP 3 158 336 B1**

(12) **EUROPEAN PATENT SPECIFICATION**

(45) Date of publication and mention
of the grant of the patent:
**22.08.2018 Bulletin 2018/34**

(51) Int Cl.:
***G01N 33/564*** *(2006.01)*   ***G01N 33/574*** *(2006.01)*

(21) Application number: **15736010.8**

(86) International application number:
**PCT/GB2015/051791**

(22) Date of filing: **19.06.2015**

(87) International publication number:
**WO 2015/193678 (23.12.2015 Gazette 2015/51)**

(54) **IMPROVED IMMUNOASSAY METHODS**

VERBESSERTE IMMUNOASSAYVERFAHREN

PROCÉDÉS D'IMMUNODOSAGE AMÉLIORÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.06.2014 GB 201411060**

(43) Date of publication of application:
**26.04.2017 Bulletin 2017/17**

(73) Proprietor: **ONCIMMUNE LIMITED**
**Nottingham NG5 1PB (GB)**

(72) Inventors:
• **MURRAY, Andrea**
**Nottingham**
**Nottinghamshire NG5 1PB (GB)**
• **HAMILTON-FAIRLEY, Geoffrey Neil**
**Nottingham**
**Nottinghamshire NG5 1PB (GB)**
• **ALLEN, Jared**
**Nottingham**
**Nottinghamshire NG5 1PB (GB)**

(74) Representative: **Boult Wade Tennant LLP**
**Verulam Gardens**
**70 Gray's Inn Road**
**London WC1X 8BT (GB)**

(56) References cited:
• **R. W. BARRETTE ET AL: "Quantifying Specific Antibody Concentrations by Enzyme-Linked Immunosorbent Assay Using Slope Correction", CLINICAL AND VACCINE IMMUNOLOGY, vol. 13, no. 7, 1 July 2006 (2006-07-01), pages 802-805, XP055105156, ISSN: 1556-6811, DOI: 10.1128/CVI.00422-05**
• **A. MURRAY ET AL: "Technical validation of an autoantibody test for lung cancer", ANNALS OF ONCOLOGY, vol. 21, no. 8, 2 February 2010 (2010-02-02), pages 1687-1693, XP055213533, ISSN: 0923-7534, DOI: 10.1093/annonc/mdp606**
• **P. BOYLE ET AL: "Clinical validation of an autoantibody test for lung cancer", ANNALS OF ONCOLOGY, vol. 22, no. 2, 30 July 2010 (2010-07-30), pages 383-389, XP055213539, ISSN: 0923-7534, DOI: 10.1093/annonc/mdq361**
• **SHARMA ET AL: "Rapid and sensitive detection of autoantibody in rheumatoid arthritis patients by heat-mediated ELISA", CLINICAL BIOCHEMISTRY, ELSEVIER INC, US, CA, vol. 41, no. 1-2, 11 October 2007 (2007-10-11), pages 97-102, XP022384419, ISSN: 0009-9120, DOI: 10.1016/J.CLINBIOCHEM.2007.09.016**
• **MARIA ERALI ET AL: "ELISA for thyroglobulin in serum: recovery studies to evaluate autoantibody interference and reliability of thyroglobulin values Fax 80l-583", CLINICAL CHEMISTIY, vol. 42, no. 5, 1 January 1996 (1996-01-01), pages 766-770, XP055213547,**

EP 3 158 336 B1

**(Cont. next page)**

- CAROLINE J. CHAPMAN ET AL: "EarlyCDT-Lung test: improved clinical utility through additional autoantibody assays", TUMOR BIOLOGY, vol. 33, no. 5, 11 April 2012 (2012-04-11) , pages 1319-1326, XP055213541, ISSN: 1010-4283, DOI: 10.1007/s13277-012-0379-2

- Unknown: "Theory of Binding Data Analysis", , 10 August 2006 (2006-08-10), XP055213668, Retrieved from the Internet: URL:https://tools.thermofisher.com/downloads/FP7.pdf [retrieved on 2015-09-16]

Description

**Field of the invention**

The invention generally relates to the field of antibody detection, and in particular relates to assays for the detection of antibodies in a sample comprising patient bodily fluid. The scope of protection is defined by the appended claims.

**Background to the invention**

Many diagnostic, prognostic and/or monitoring assays rely on detection of a biological marker of a particular disease state or disease susceptibility. Such biological markers are commonly proteins or polypeptides that are characteristic of a particular disease or associated with susceptibility to disease.
In recent years it has become apparent that antibodies, and in particular autoantibodies, can also serve as biological markers of disease or disease susceptibility. Autoantibodies are naturally occurring antibodies directed to an antigen which an individual's immune system recognises as foreign even though that antigen actually originated in the individual. They may be present in the circulation as circulating free autoantibodies or in the form of circulating immune complexes consisting of autoantibodies bound to their target protein. Differences between a wild type protein expressed by "normal" cells and an altered form of the protein produced by a diseased cell or during a disease process may, in some instances, lead to the altered protein being recognised by an individual's immune system as "non-self" and thus eliciting an immune response in that individual. This may be a humoral (i.e. B cell-mediated) immune response leading to the production of autoantibodies immunologically specific for the altered protein.
WO 99/58978 describes methods for use in the detection/diagnosis of cancer which are based on evaluating the immune response of an individual to two or more distinct tumour markers. These methods generally involve contacting a sample of bodily fluid taken from the individual with a panel of two or more distinct tumour marker antigens, each derived from a separate tumour marker protein, and detecting the formation of complexes of the tumour marker antigens bound to circulating autoantibodies immunologically specific for the tumour marker proteins. The presence of such circulating autoantibodies is taken as an indication of the presence of cancer.
Assays which measure the immune response of the individual to the presence of tumour marker protein in terms of autoantibody production provide an alternative to the direct measurement or detection of tumour marker protein in bodily fluids. Such assays essentially constitute indirect detection of the presence of tumour marker protein. The nature of the immune response means it is likely that autoantibodies can be elicited by a very small amount of circulating tumour marker protein and indirect methods which rely on detecting the immune response to tumour markers will consequently be more sensitive than methods for the direct measurement of tumour markers in bodily fluids. Assay methods based on the detection of autoantibodies may therefore be of particular value early in the disease process and possibly also in relation to screening of asymptomatic patients, for example in screening to identify individuals "at risk" of developing disease amongst a population of asymptomatic individuals, to identify individuals who have developed a disease amongst a population of asymptomatic individuals. In addition, methods based on the detection of autoantibodies may be of particular value early in the disease process and may also be used to identify individuals who have developed a disease amongst a population of symptomatic individuals. Furthermore, they may be useful for earlier detection of recurrent disease. The assay methods may also be of value in selecting or monitoring therapies for a disease.

As well as being indicative of a cancer disease state, antibodies and autoantibodies can also serve as biological markers of other disease states or disease susceptibilities, of which rheumatoid arthritis, systemic lupus erythematous (SLE), primary biliary cirrhosis (PBC), autoimmune thyroiditis (e.g. Hashimoto's thyroiditis), autoimmune gastritis (e.g. pernicious anaemia), autoimmune adrenalitis (eg Addison's disease), autoimmune hypoparathyriodism, autoimmune diabetes (e.g. Type 1 diabetes), myasthenia gravis, paraneoplastic neurological disorders such as Lambert-Eaton Myasthenic Syndrome, inflammatory bowel disease, sarcoidosis and autoimmune hepatitis are but examples.

It was previously recognised that when assays based on detection of antibodies are used diagnostically or prognostically to assess the disease state, disease progression or disease susceptibility of an individual within a population, difficulties can arise in devising a standardised assay methodology appropriate for the whole population of subjects to be screened because the absolute amounts of antibody present vary dramatically from individual to individual. This can produce a high incidence of false negative results, for example amongst individuals having a low amount of antibody. Similarly there is a difficulty in scoring true positive results because the variation in absolute amounts of antibody from individual to individual means that it is difficult to set a threshold for a positive assay result that is appropriate for all individuals within the population screened.

In WO2006/126008 it was determined that the performance, and more specifically the clinical utility and reliability, of assays based on detection of antibodies, particularly autoantibodies, as biological markers of disease can be improved dramatically by inclusion of an antigen titration step. By testing the sample suspected of containing antibodies against a series of different amounts of antigen and constructing a titration curve it is possible to reliably identify true positive screening results independently of the absolute amount of antibody present in the sample. This approach is contrary to previous methods which titrate antigen merely to construct a calibration curve or to allow identification of the most appropriate antigen concentration to be used for detecting antibodies in actual patient samples. In these methods only a single point measurement is proposed for actual diagnosis. Thus, these methods will not allow for variation in amounts of the antibody to be detected from individual to individual resulting in the incidence of false positives and false negatives as discussed above. The antigen titration method of WO2006/126008 provides greater specificity and sensitivity than measuring autoantibody reactivity at a single antigen concentration, or methods in which the serum sample is titrated rather than the antigen.

## Summary of the invention

It has been established that the antigen titration method described in WO2006/126008 provides a sensitivity of around 40% and a specificity of around 90% for the detecting lung cancer. Therefore, about 10% of healthy patients tested had a positive result without presenting with the disease state (false positives). Due to the low incidence of lung cancer in the population, the Positive Predictive Value (PPV), the proportion of positive results which are true positives rather than false positives, is primarily driven by the specificity of the assay. It would therefore be advantageous to increase the specificity, i.e. reduce the number of false positive results. With this in mind, it has surprisingly been determined that the specificity of the antigen titration test method can be improved by assessing both the amount of antigen/antibody binding and a secondary curve parameter. This method will be referred to herein as the "double cut-off method". This method requires assessment of the level of specific binding between the antibody and the antigen and assessment of a secondary curve parameter, with only test results considered positive when compared to cut-off points for both of these metrics being classified as positive.

According to a first aspect of the invention there is provided a method of detecting an antibody in a test sample comprising a bodily fluid from a mammalian subject, the method comprising the steps of:

> (a) contacting said test sample with a plurality of different amounts of an antigen specific for said antibody;
> (b) detecting the amount of specific binding between said antibody and said antigen;
> (c) plotting or calculating a curve of the amount of said specific binding versus the amount of antigen for each amount of antigen used in step (a);
> (d) calculating a secondary curve parameter from the curve plotted or calculated in step (c); and
> (e) determining the presence or absence of said antibody based upon a combination of:

>> (i) the amount of specific binding between said antibody and said antigen determined in step (b); and
>> (ii) said secondary curve parameter determined in step (d).

According to a second aspect of the invention there is provided a method of detecting two or more antibodies in a test sample comprising a bodily fluid from a mammalian subject, the method comprising the steps of:

> (a) contacting said test sample with a panel comprising two or more sets of antigens, wherein each of said antigens in said panel is specific for at least one of said two or more antibodies;
> (b) detecting complexes of said antigens bound to antibodies present in said test sample;
> (c) for each antigen plotting or calculating a separate curve of the amount of said specific binding versus the amount of antigen for each amount of antigen used in step (a);
> (d) calculating a secondary curve parameter for each curve plotted or calculated in step (c); and
> (e) determining the presence or absence of said two or more antibodies based upon a combination of:

>> (i) the amount of specific binding between said antibody and said antigen determined in step (b); and
>> (ii) said secondary curve parameter determined in step (d),

for each of the curves plotted or calculated in step (c).
In all aspects of the invention the mammalian subject is preferably a human.
In all aspects of the invention the method is carried out *in vitro* on a test sample comprising a bodily fluid obtained or prepared from the mammalian subject.
A key feature of the invention in all its aspects is the determination of a secondary curve parameter and the finding that use of this secondary curve parameter, in combination with the amount of specific binding between an antibody in the test sample and the antigen, increases the specificity of the methods of the invention, i.e. reduces the number of false positive results, compared to assays based upon only the amount of specific binding between an antibody in the test sample. Due to the low incidence of lung cancer in the population, the Positive Predictive Value (PPV) is primarily driven by the specificity of the assay and this increase in specificity increases PPV.

In certain embodiments the increase in specificity afforded by the double cut-off method allows a greater number of antigens to be applied to the test sample simultaneously, increasing sensitivity, i.e. decreasing the number of false negative results, relative to prior art methods. In certain embodiments the increase in specificity afforded by the methods of the invention permits additional antigens or multiple antigen variants to be applied to the test sample, increasing the sensitivity of the assay methods. This approach is contrary to previous investigations performed in this area, which focussed on attempting to identify individual antigens demonstrating the highest possible sensitivity. The present method increases the assay specificity, and hence PPV, permitting assay sensitivity to be increased by assaying multiple antigens, with the double cut off method preventing a resulting decrease in specificity which would be expected upon assaying multiple antigens using prior art methods. This avoids the need to identify individual antigens which demonstrate particularly high sensitivity.

**Brief description of Figures**

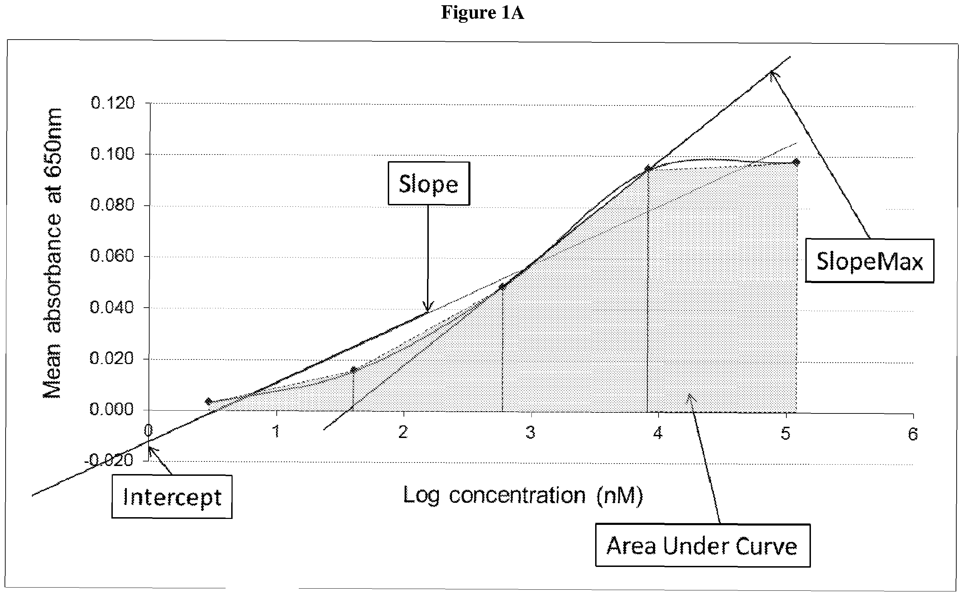

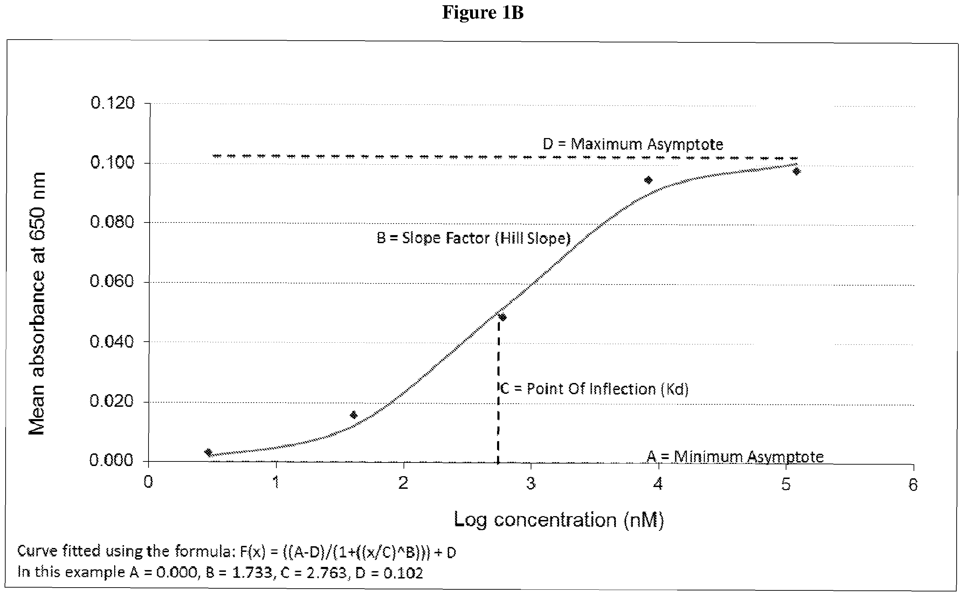

Figure 1. Diagrammatic representation to demonstrate the derivation of secondary curve parameters: A = Slope, Intercept, Area Under the Curve (AUC) and SlopeMax. B = dissociation constant (Kd).

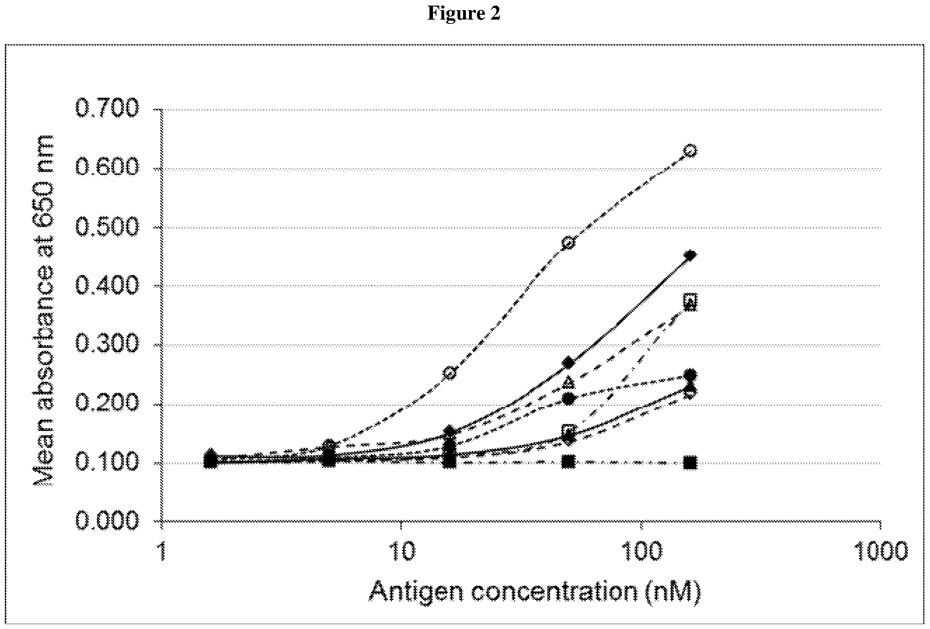

Figure 2. Example of a set of titration curves obtained for binding of antibody to a panel of seven tumour associated antigens: (– – △ – – p53), (– – ◇ – – SOX2), (——▲—— CAGE), (– · -□- · · NY-ESO-1), (——◆——BU4-5), (---- ● --- MAGE A4), (---- ⊕ --- HuD) plus a control (– · -■- · · VOL).

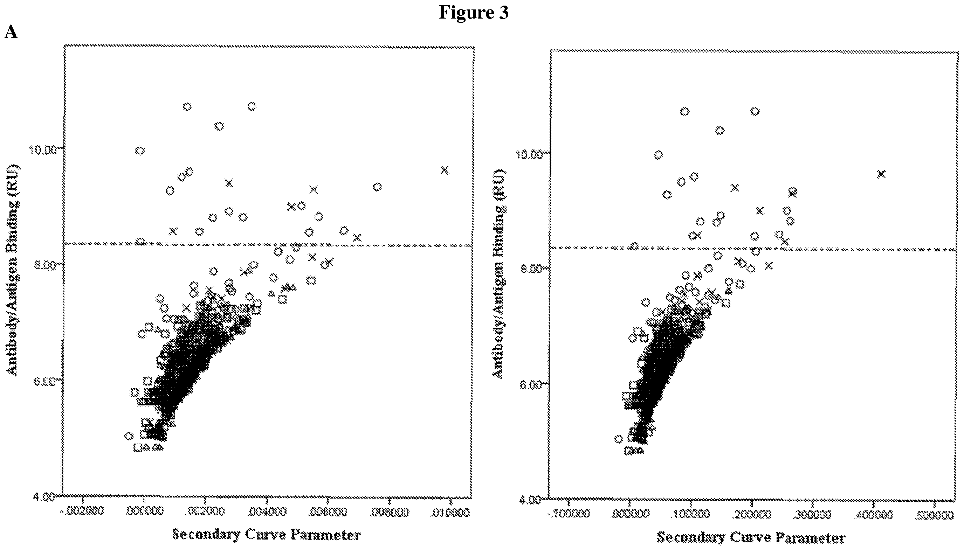

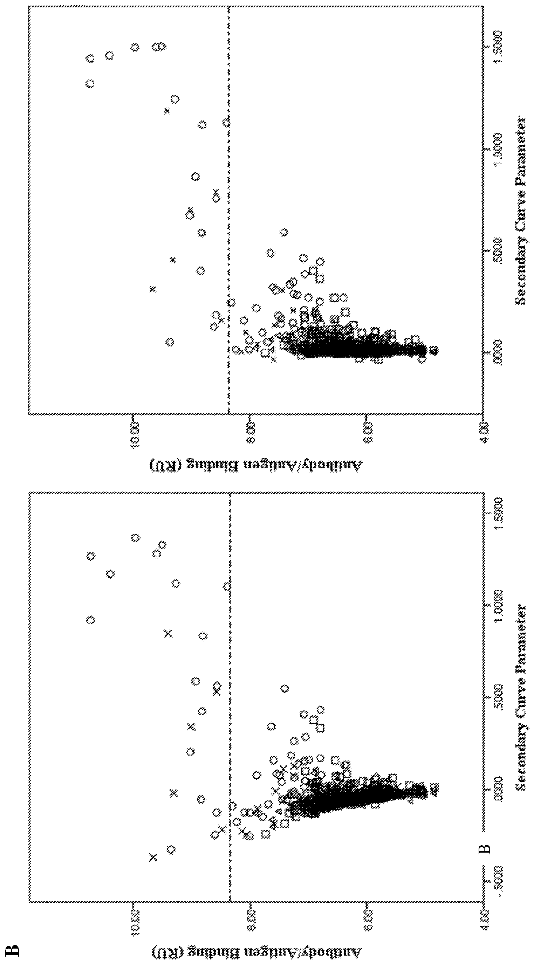

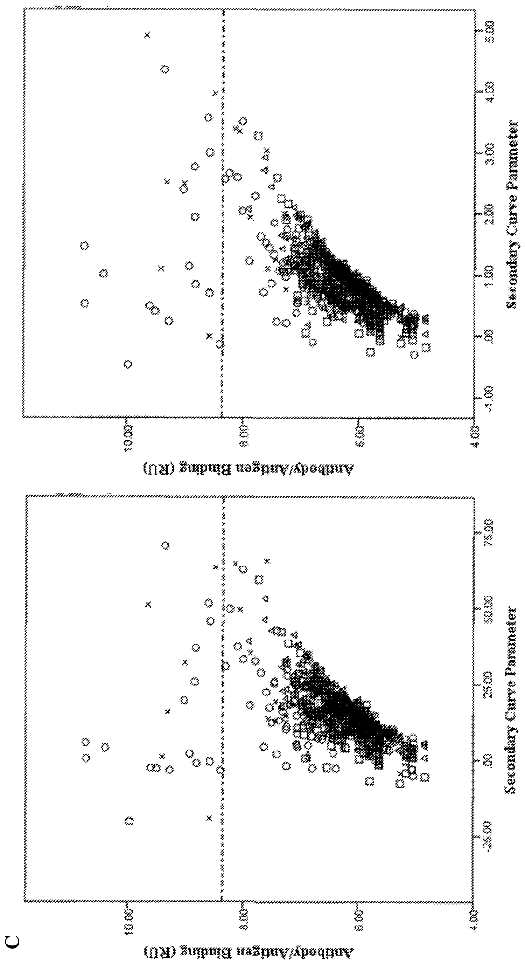

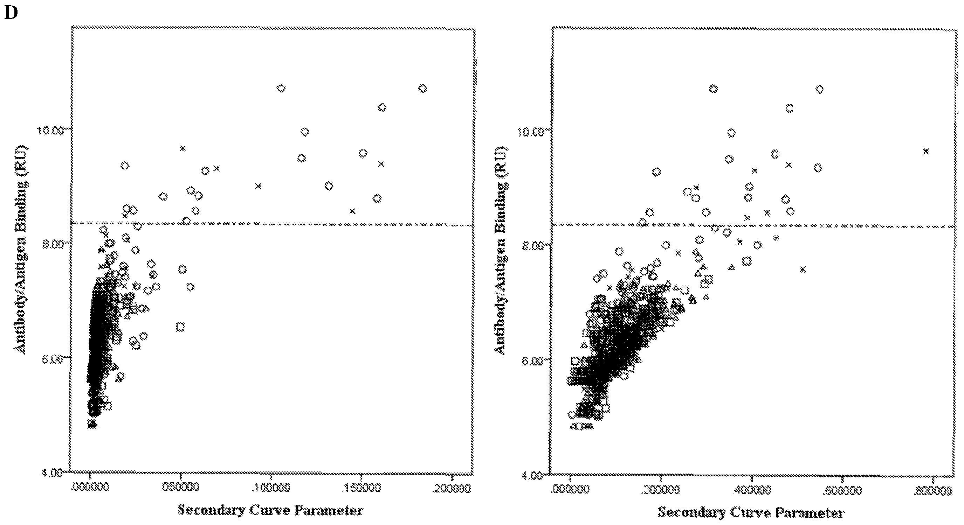

Figure 3. Secondary Curve Parameters. Plots of secondary curve parameter versus antibody / antigen binding (expressed in calibrated reference units (RU)) for p53. Both linear (left hand plot) and log (right hand plot) regression reduction of data are given for each parameter. A = slope, B = intercept, C = AUC, D = slopemax. Seven antigen panel results obtained using only the commercial (i.e. EarlyCDT-Lung) cut-off for antibody / antigen binding are coded as follows: ○ = true positives, x = false positives, ∆ = true negatives, □ = false negatives. -·-·-·- represents the standard cut-off for antibody / antigen binding.

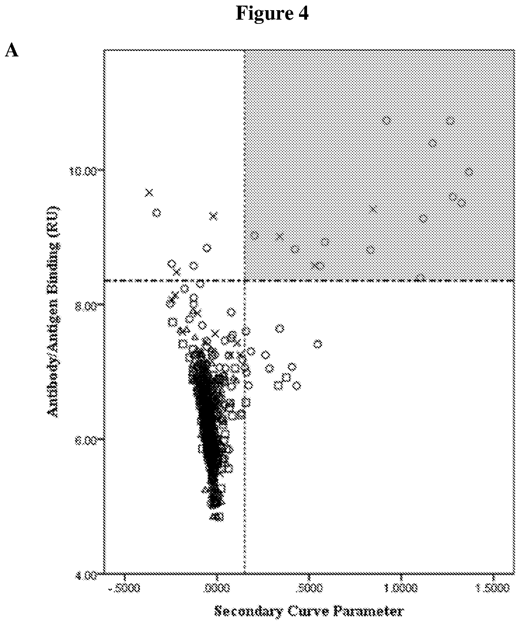

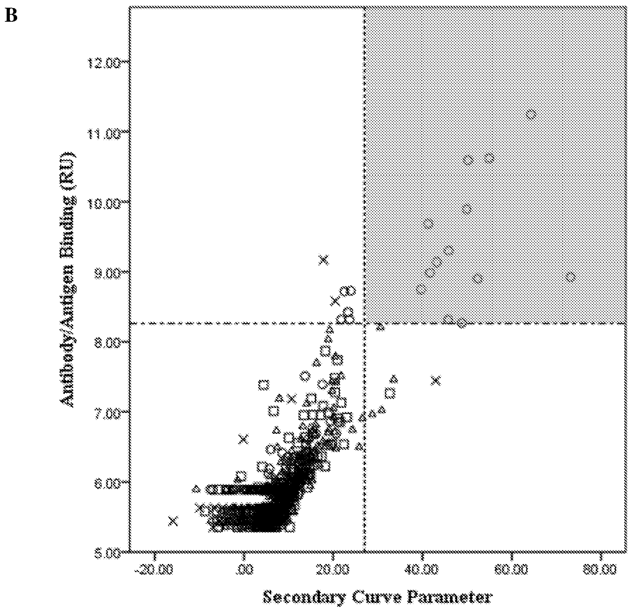

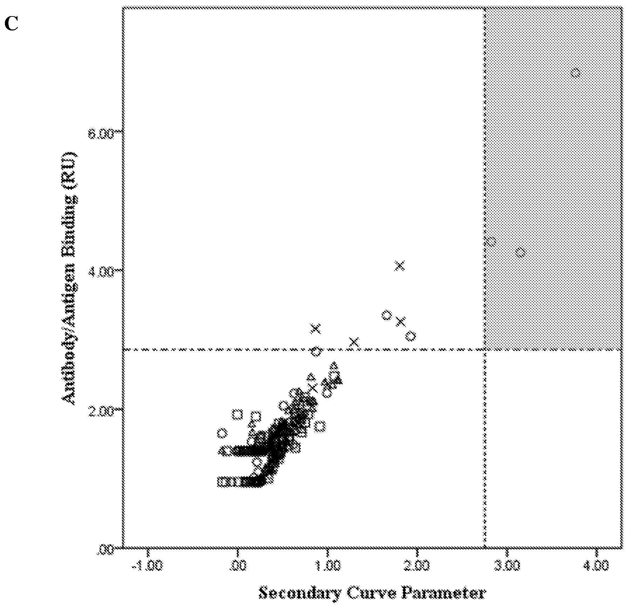

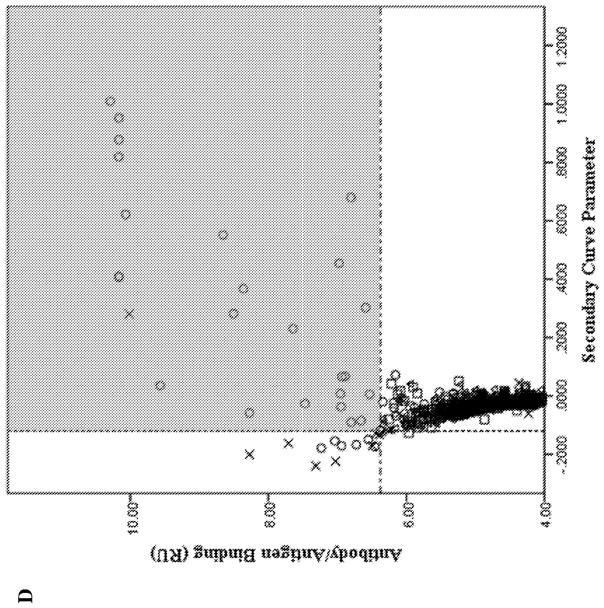

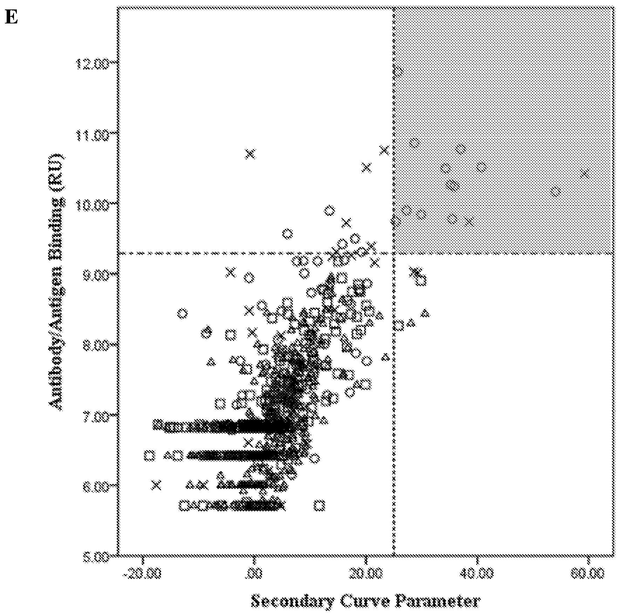

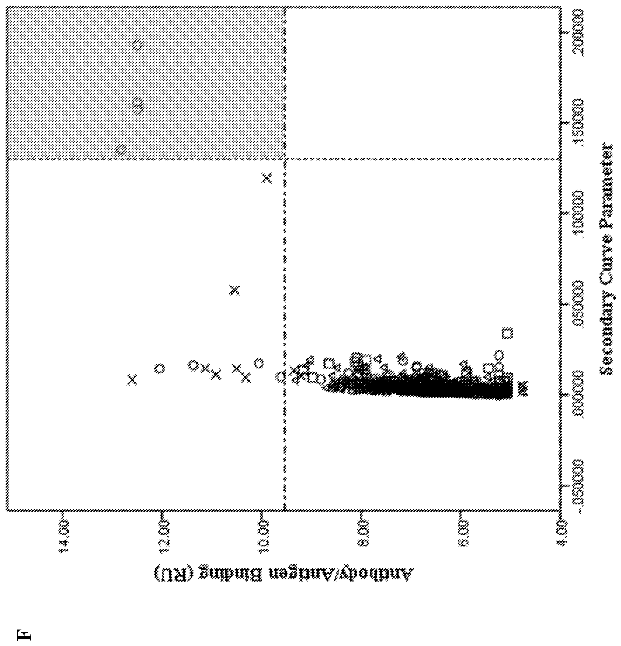

Figure 4. Development of Secondary Optimal Secondary Curve Parameters. Plots of optimal secondary curve parameter versus antibody / antigen binding (expressed in calibrated reference units (RU)) for the seven antigen panel; (A = p53), (B = SOX2), (C = CAGE), (D = NY-ESO-1), (E = MAGE A4), (F = HuD). Seven antigen panel results obtained using only the commercial (i.e. EarlyCDT-Lung) cut-off for antibody / antigen binding are coded as follows: ○ = true positives, X = false positives, A = true negatives, □ = false negatives. -·-·-·- represents the standard cut-off for antibody / antigen binding. --------- represents the secondary curve parameter cut-off. Area shaded in grey represents zone of positivity.

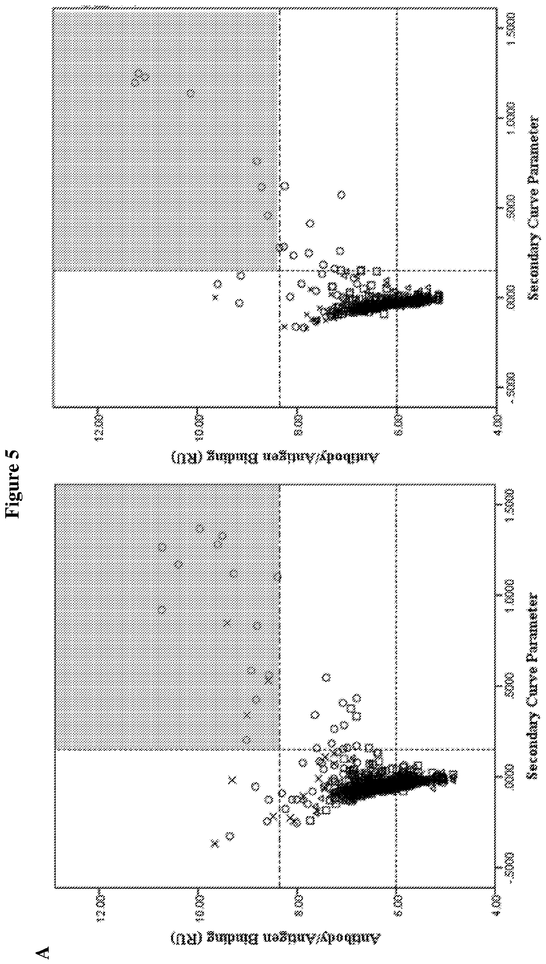

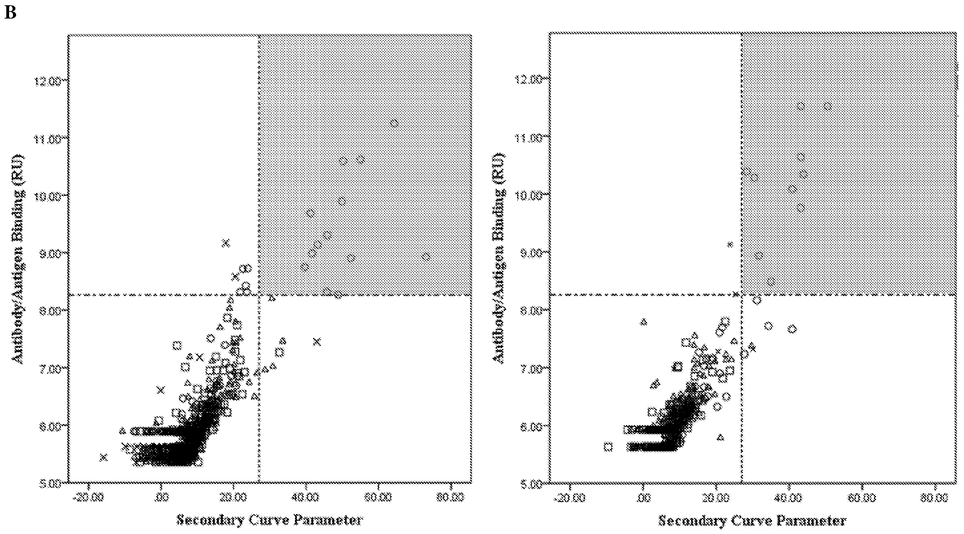

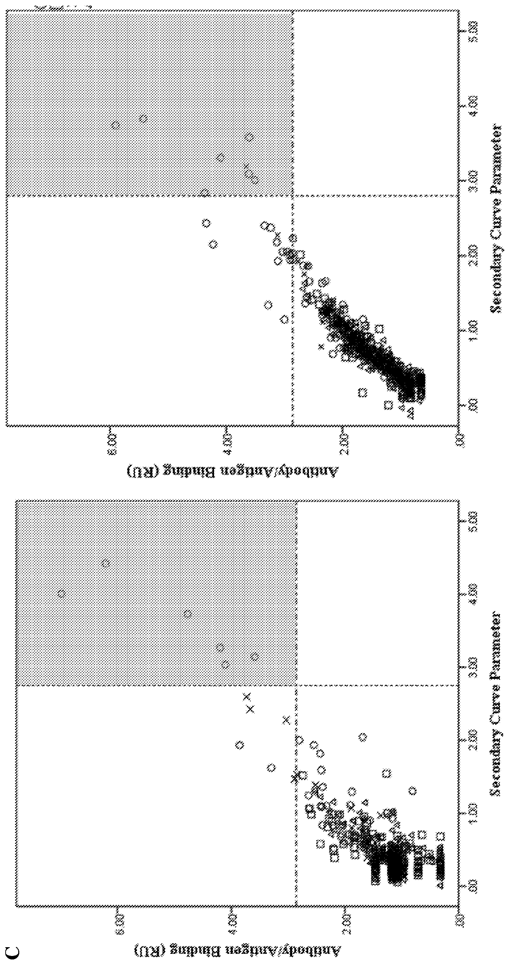

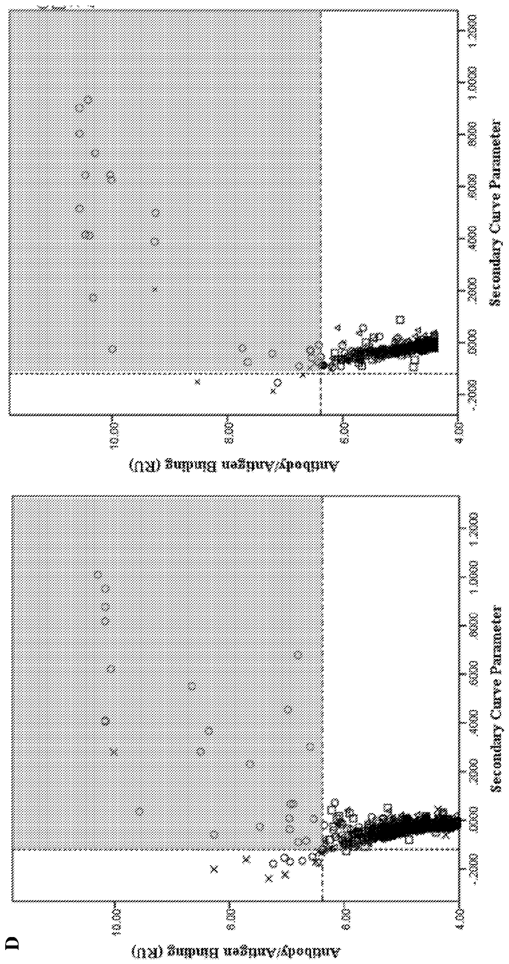

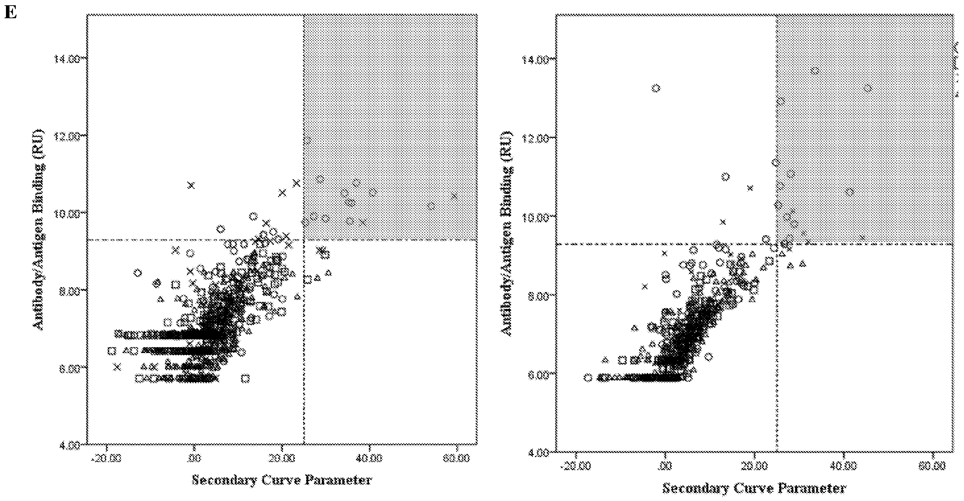

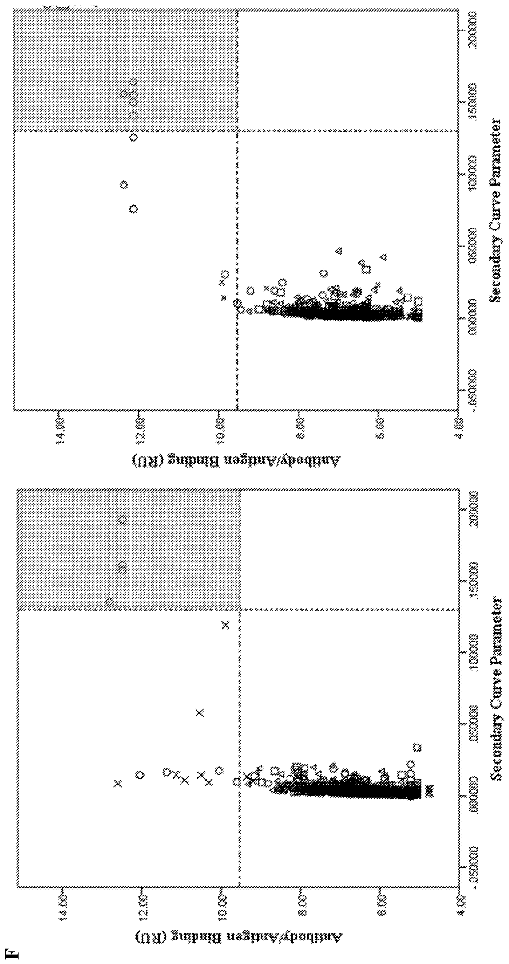

Figure 5. Confirmation on an Independent Sample Set. Plots of optimal secondary curve parameter versus antibody / antigen binding (expressed in calibrated reference units (RU)) for the seven antigen panel; (A = p53), (B = SOX2), (C = CAGE), (D = NY-ESO-1), (E = MAGE A4), (F = HuD). Training set data is shown on the left and confirmatory set data is shown on the right. Seven antigen panel results obtained using only the commercial (i.e. EarlyCDT-Lung) cut-off for antibody / antigen binding are coded as follows: ○ = true positives, X = false positives, ∆ = true negatives, □ = false negatives. -·-·-·- represents the standard cut-off for antibody / antigen binding. --------- represents the secondary curve parameter cut-off. Area shaded in grey represents zone of positivity.

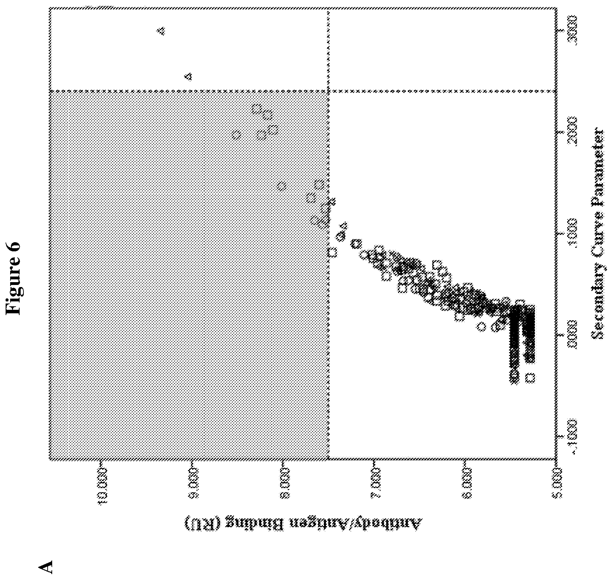

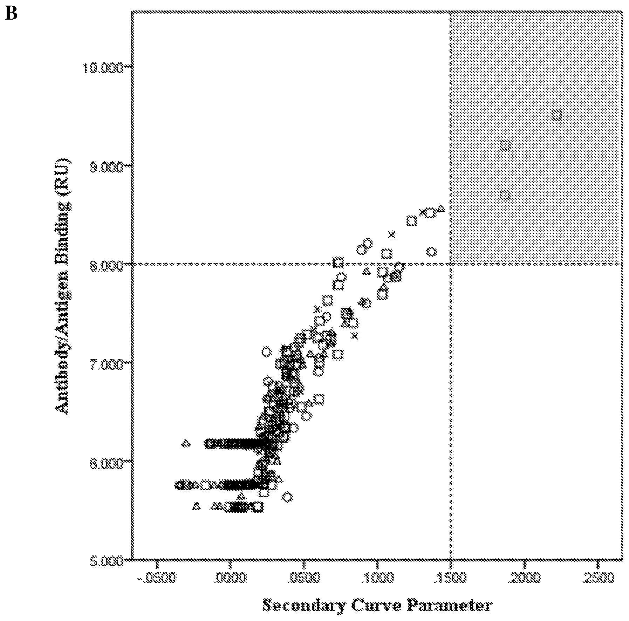

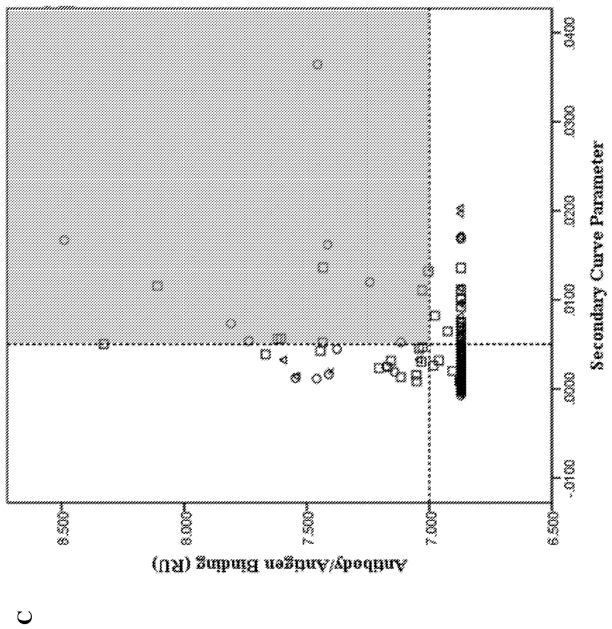

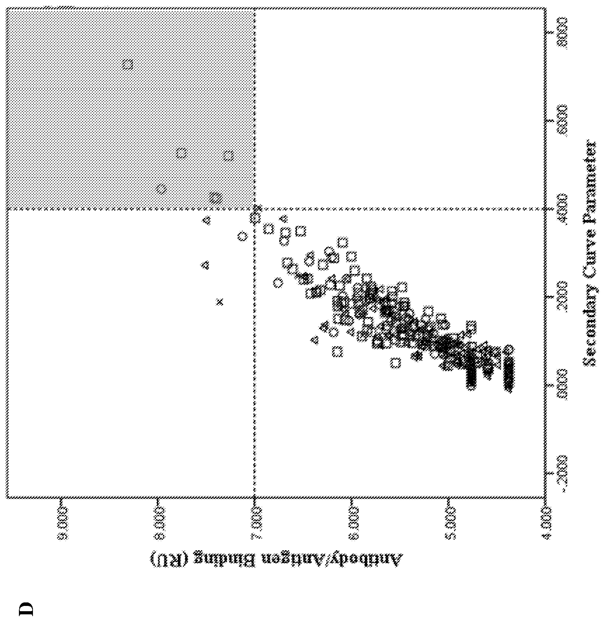

Figure 6. Improvement in Panel Performance by Incorporation of Additonal Antigens. Plots of optimal secondary curve parameter versus antibody / antigen binding (expressed in calibrated reference units (RU)) for four additional antigens; (A = alpha enolase), (B = cytokeratin 8), (C = cytokeratin 20), (D = Lmyc2). Seven antigen panel results obtained using only the commercial (i.e. EarlyCDT-Lung) cut-off for antibody / antigen binding are coded as follows: ○ = true positives, X = false positives, ∆ = true negatives, □ = false negatives. -·-·-·- represents the standard cut-off for antibody / antigen binding. --------- represents the secondary curve parameter cut-off. Area shaded in grey represents zone of positivity.

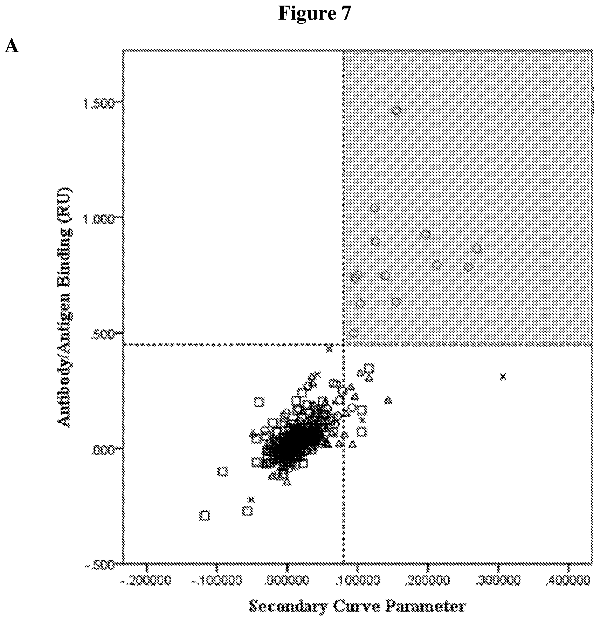

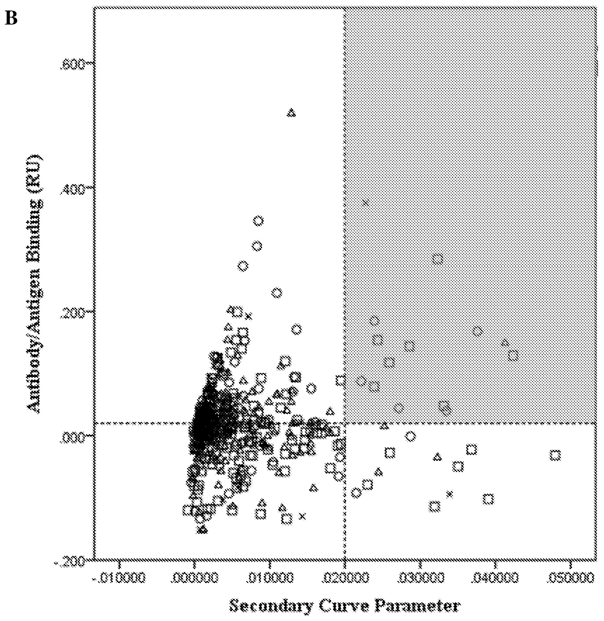

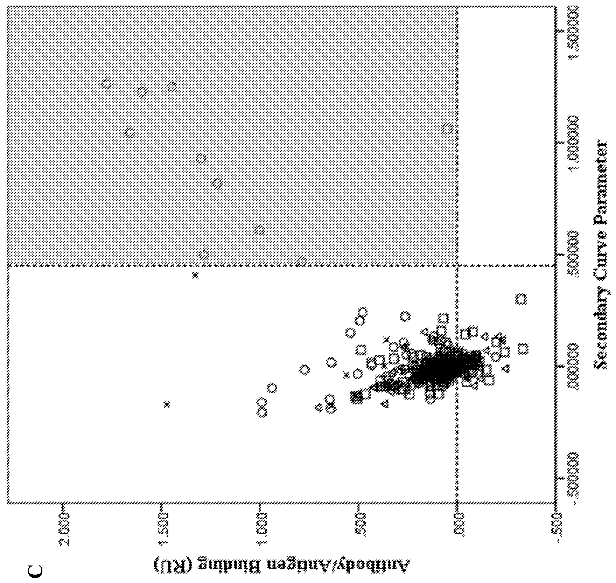

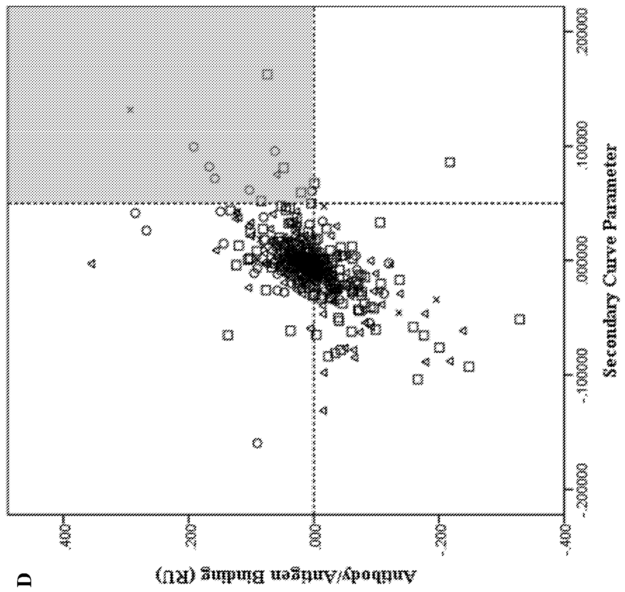

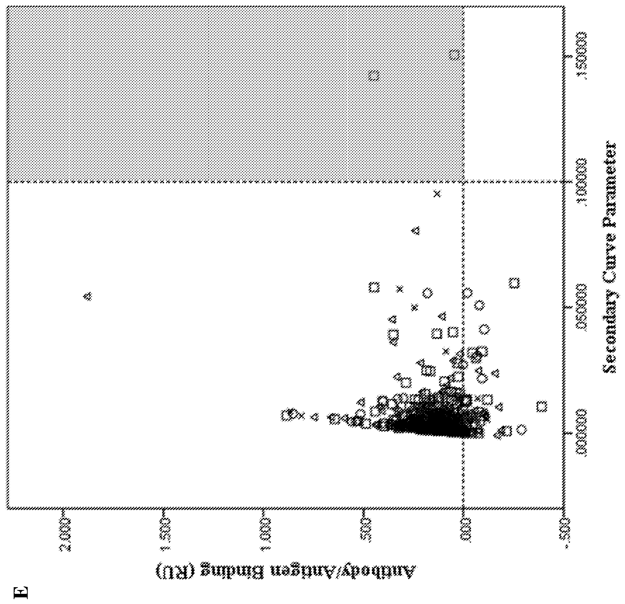

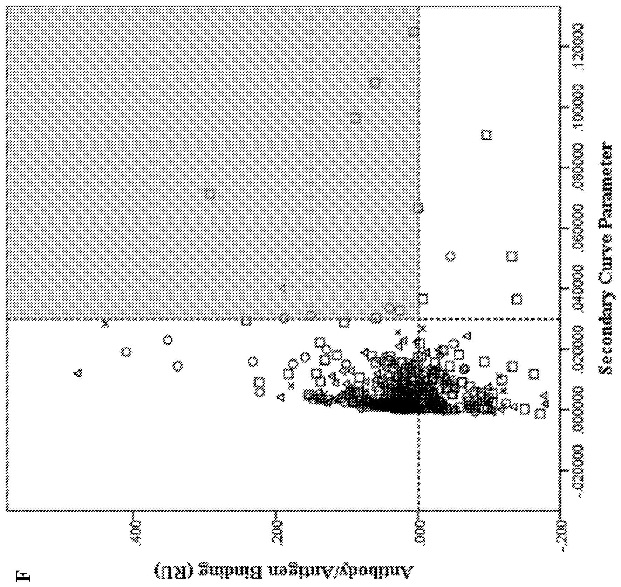

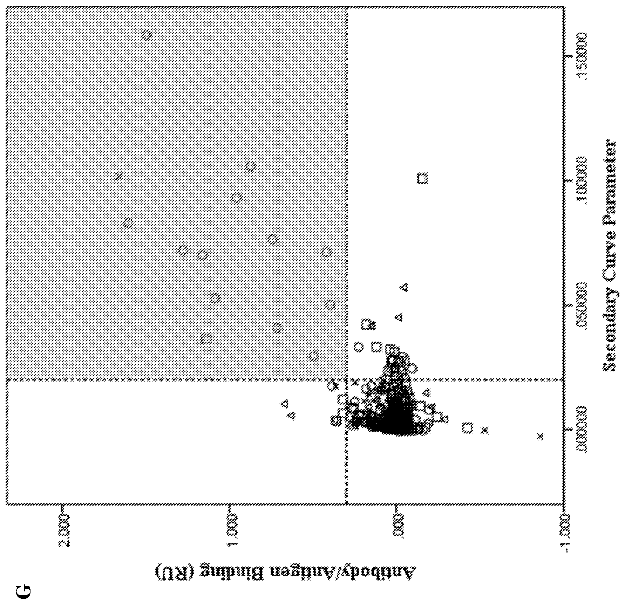

Figure 7. Improvement in Panel Performance by Incorporation of Variants and Mutated Antigens. Plots of optimal secondary curve parameter versus antibody / antigen binding (expressed in absorbance units) for the seven mutations or variants selected to be included in the final panel; (A = p53-C term), (B = Kras G12C/Q61H), (C = p53 C-BirA), (D = Kras Q61H), (E = EGFR1-EP), (F = Kras G13C/Q61H) and (G = p53-95). Seven antigen panel results obtained using only the commercial (i.e. EarlyCDT-Lung) cut-off for antibody / antigen binding are coded as follows: ○ = true positives, x = false positives, Δ = true negatives, □ = false negatives. -·-·-·- represents the standard cut-off for antibody / antigen binding. --------- represents the secondary curve parameter cut-off. Area shaded in grey represents zone of positivity.

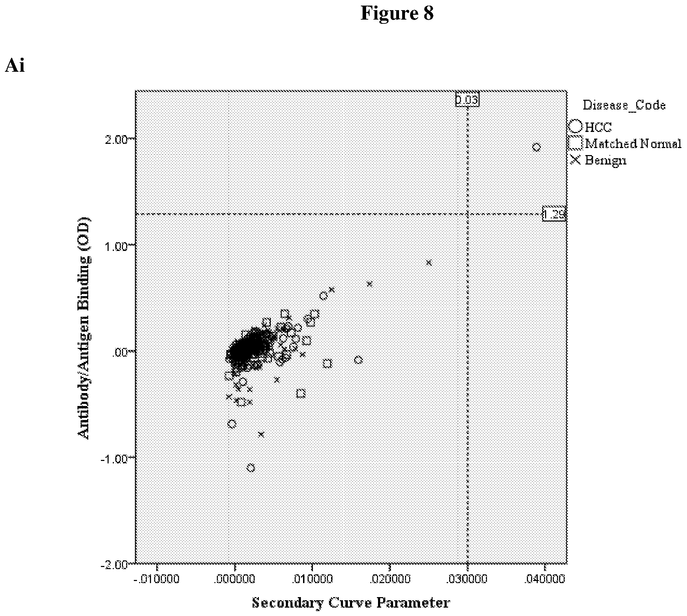

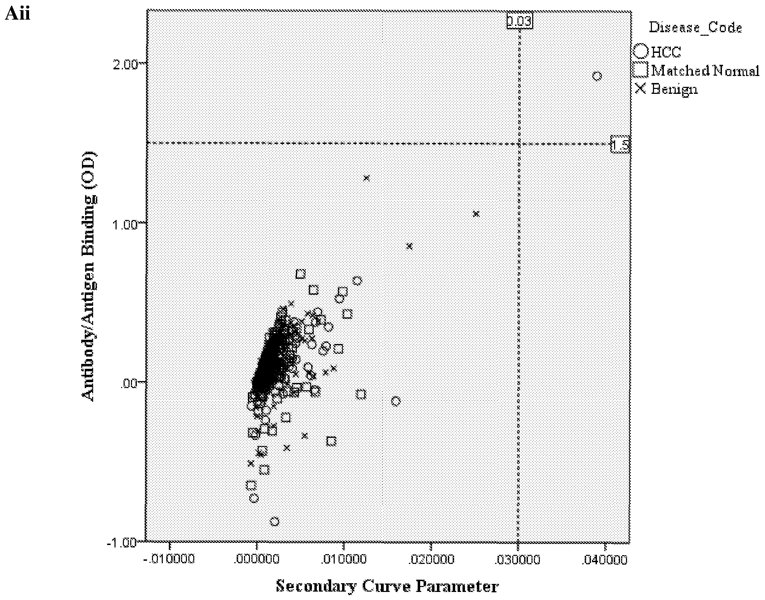

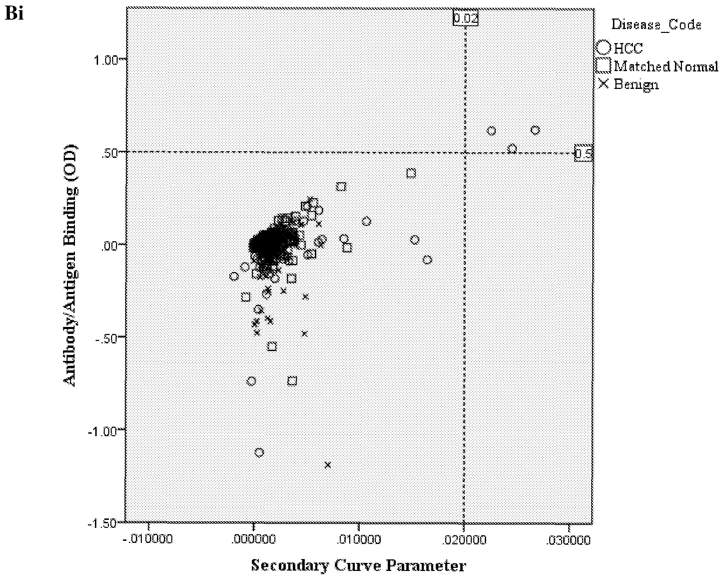

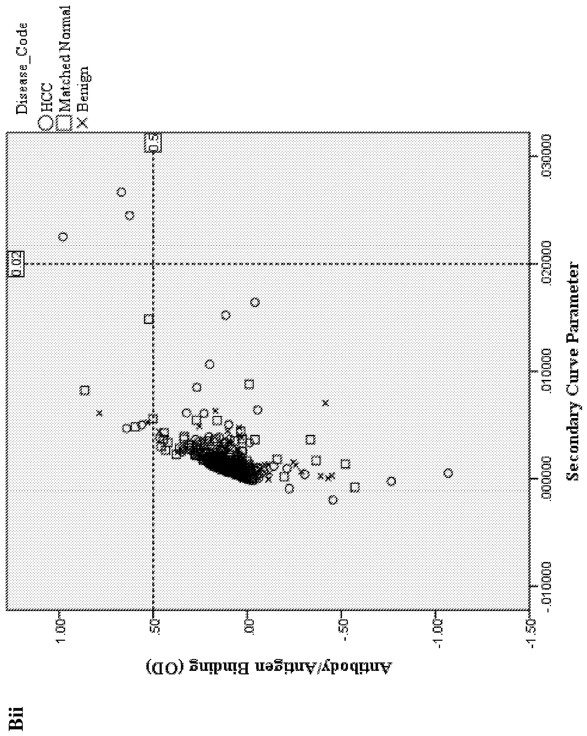

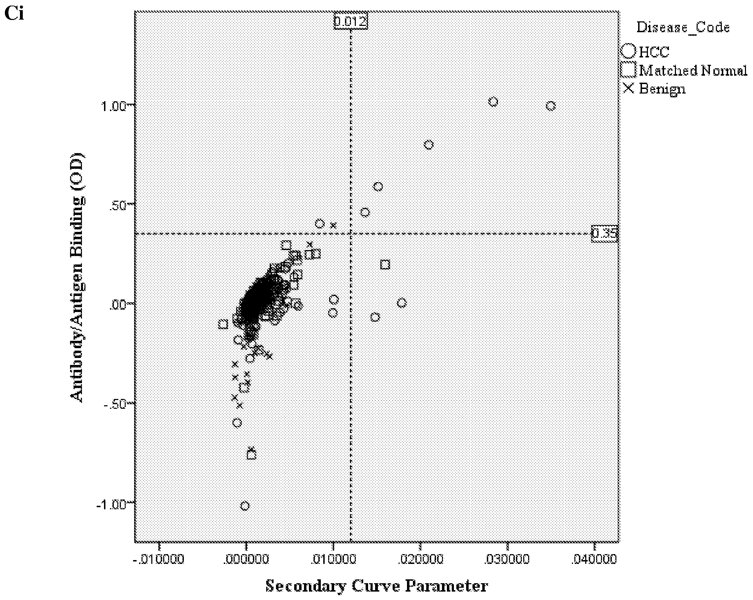

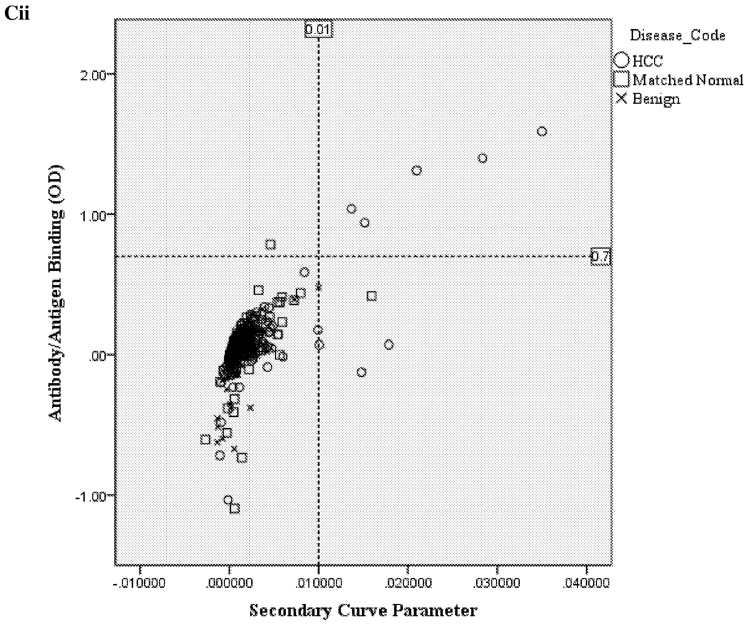

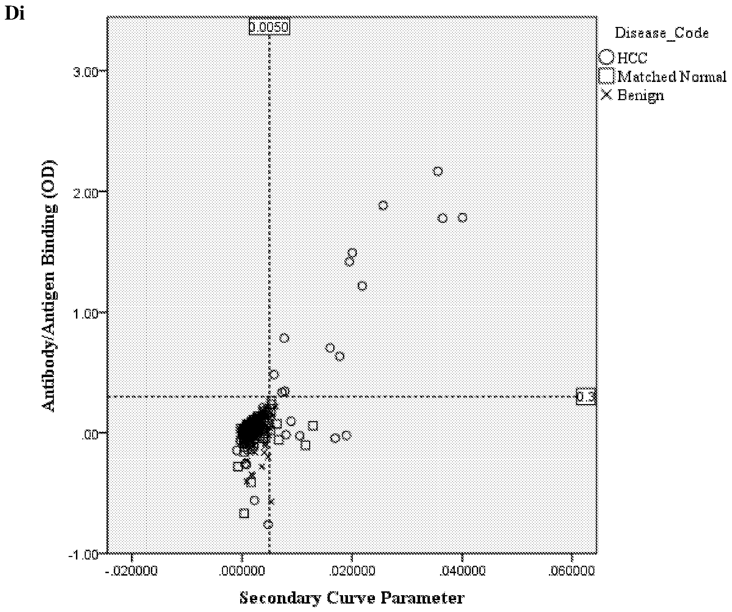

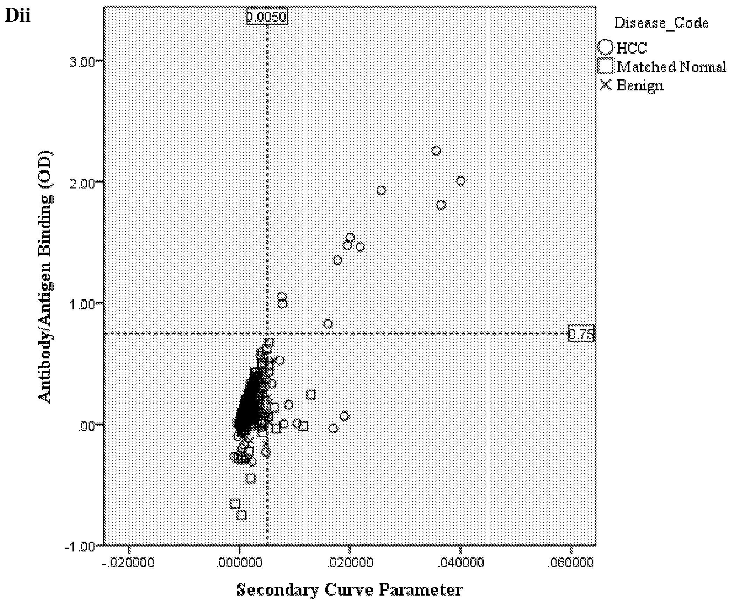

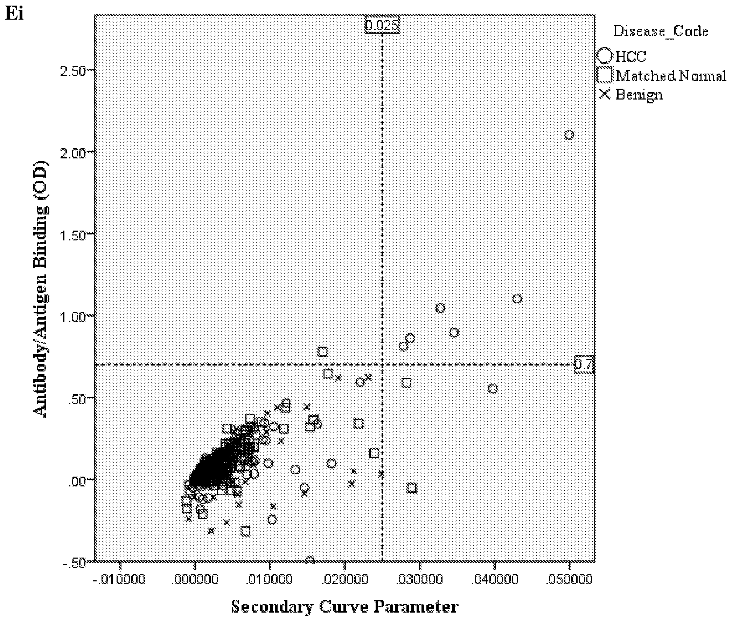

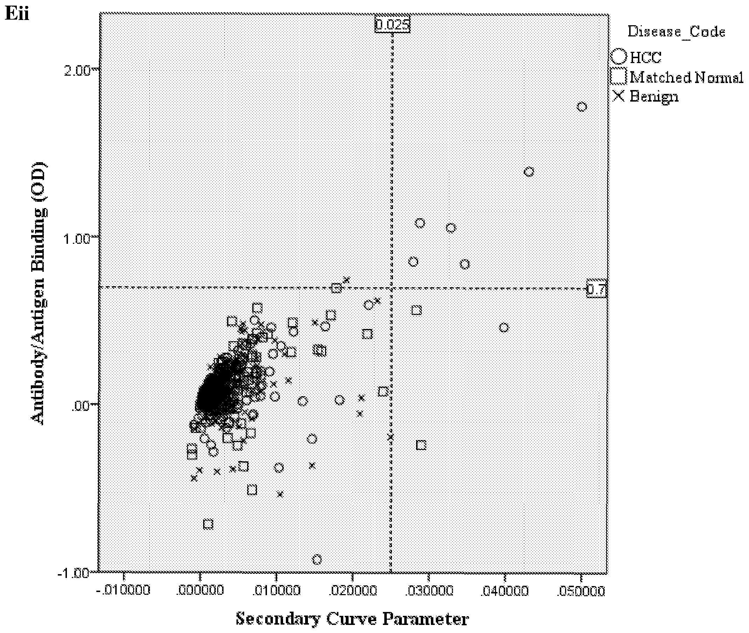

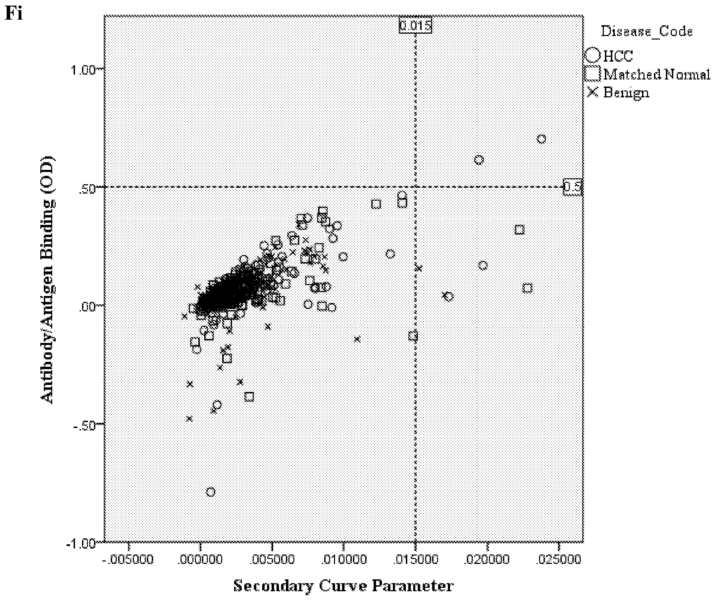

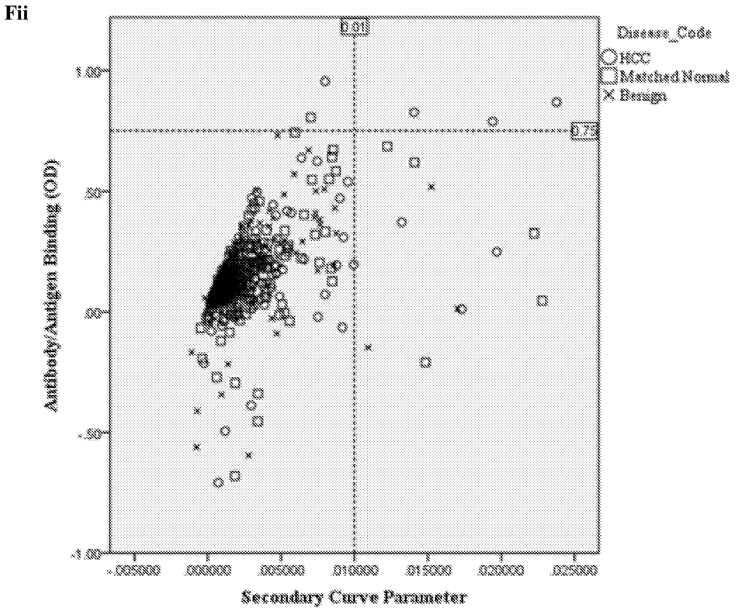

Figure 8: Development of a diagnostic panel for HCC, incorporating Secondary Curve Parameters. Plots of secondary curve parameter versus antibody / antigen binding (expressed in optical density units (OD)) for an initial six antigen panel at two antigen concentrations; (A = p53), (B = SOX2), (C = CAGE), (D = NY-ESO-1), (E = MAGE A4), (F = CK8), (i = antigen at 50nM concentration, ii = antigen at 160nM concentration). Cohort disease class is coded as follows: ○ = HCC, x = benign liver disease, □ = matched healthy normal.represents the relative cut-off. The horizontal cut-off represents the standard antibody / antigen binding cut-off and the vertical cut-off represents the secondary curve parameter cut-off. Area shaded in dark grey represents zone of positivity.

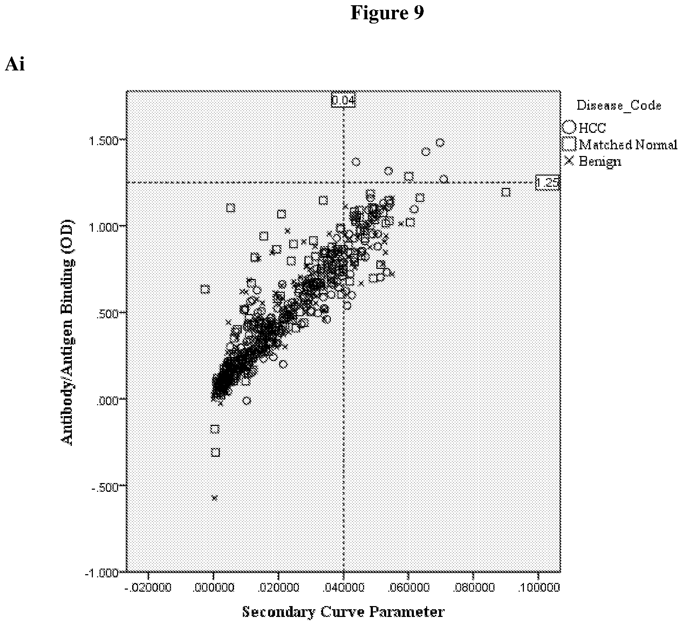

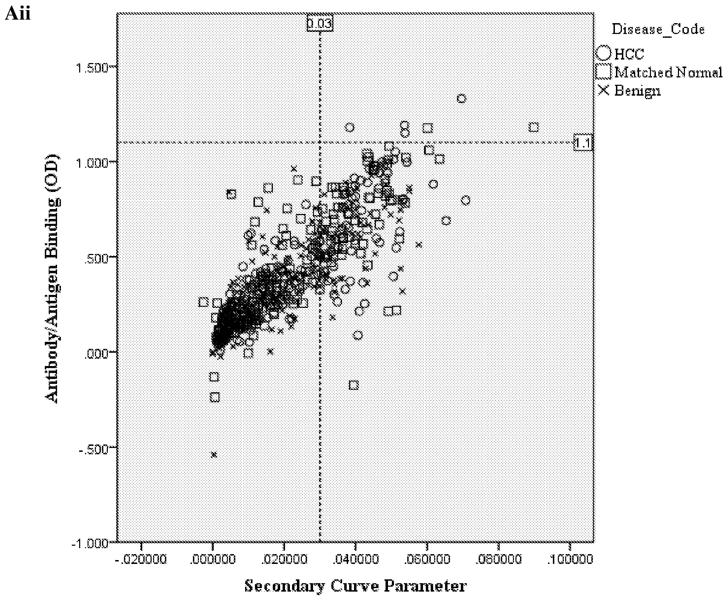

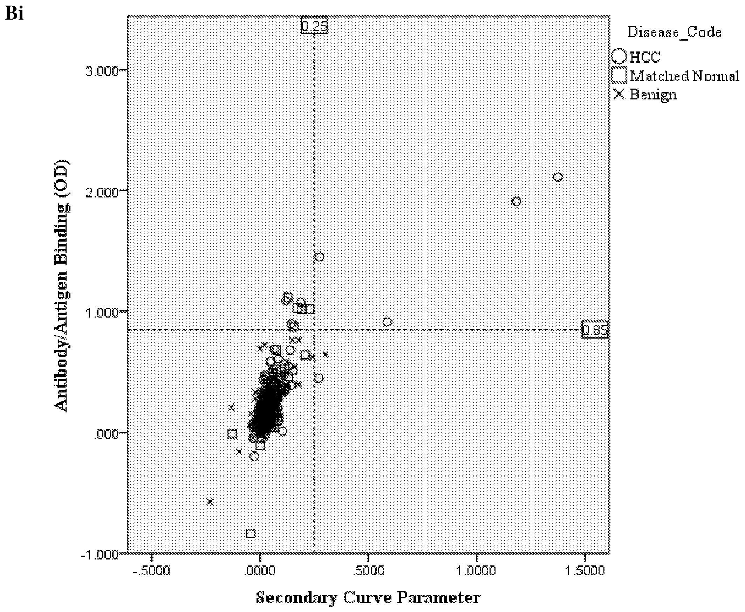

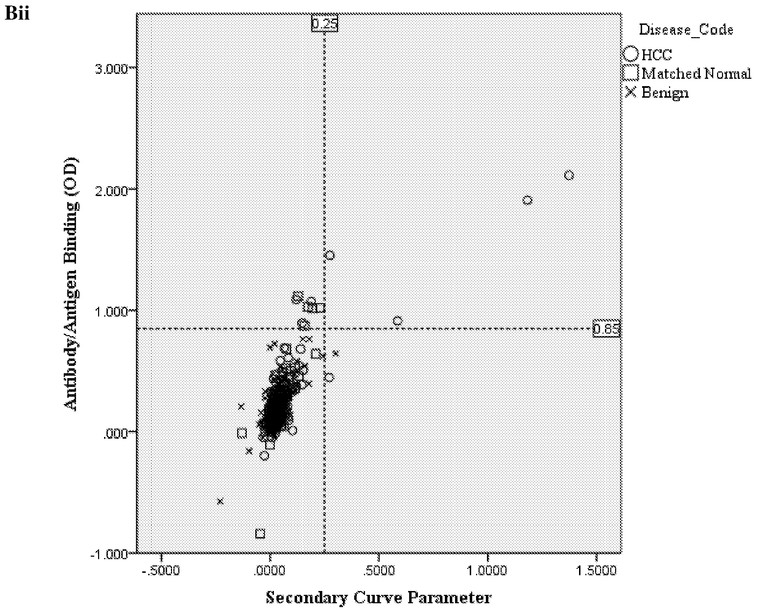

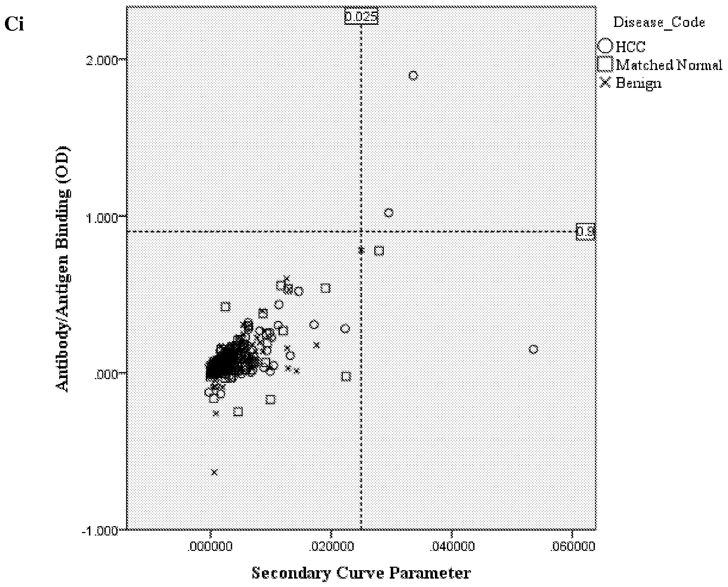

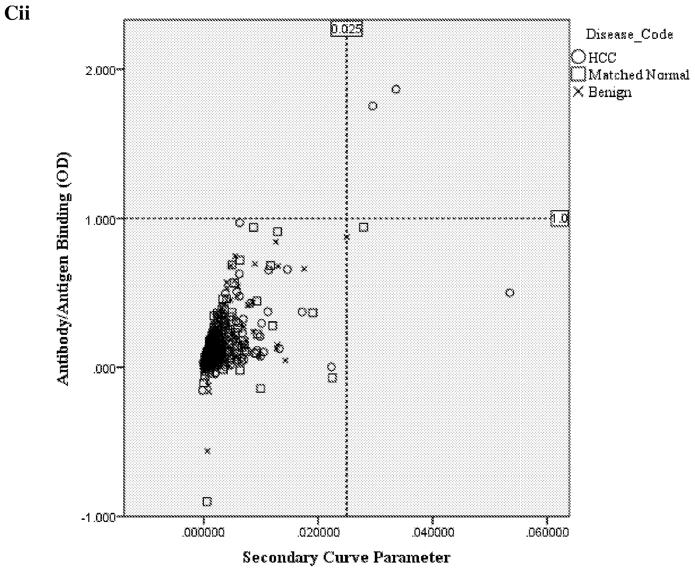

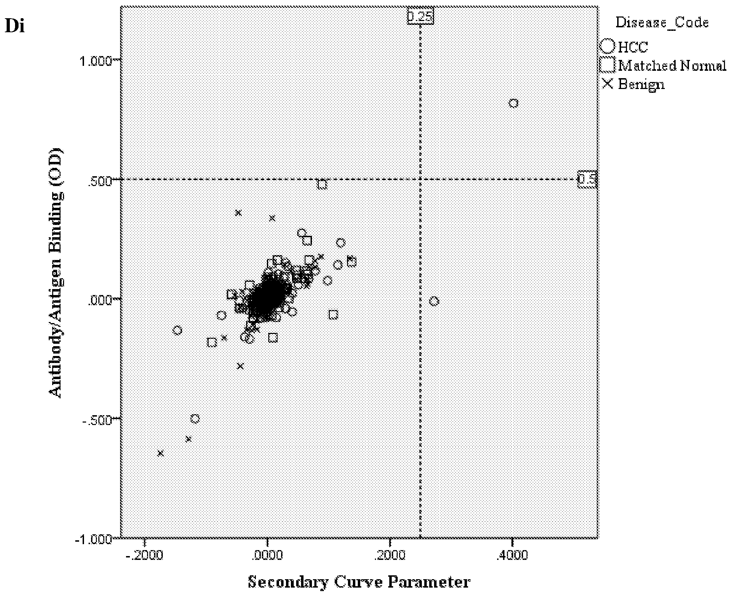

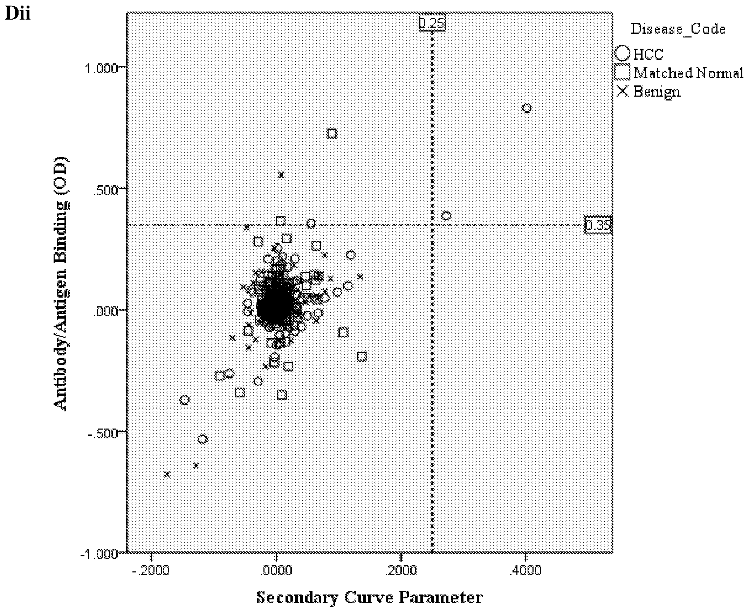

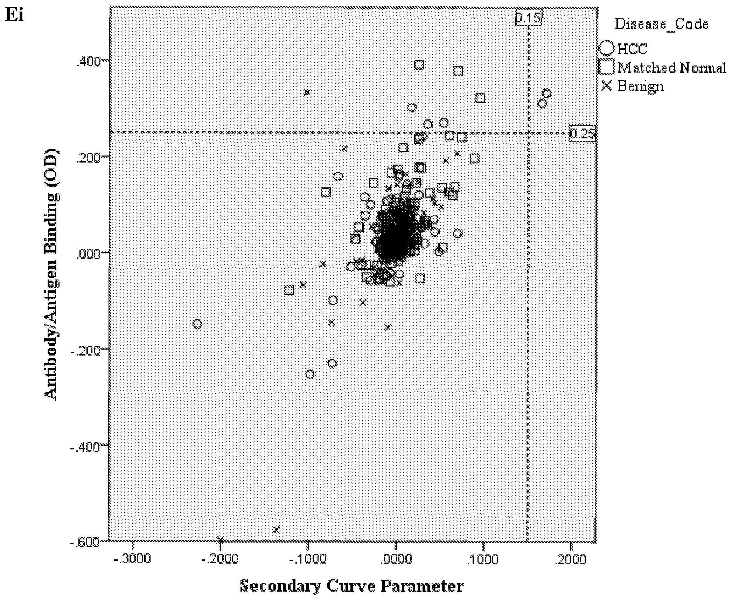

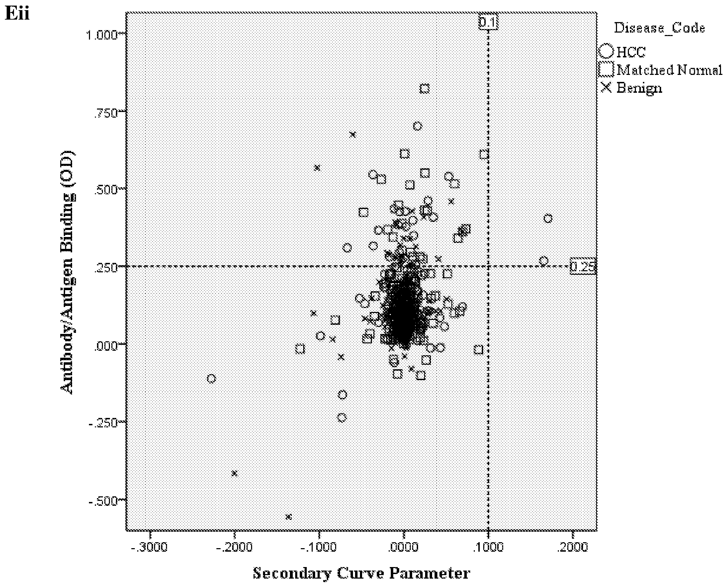

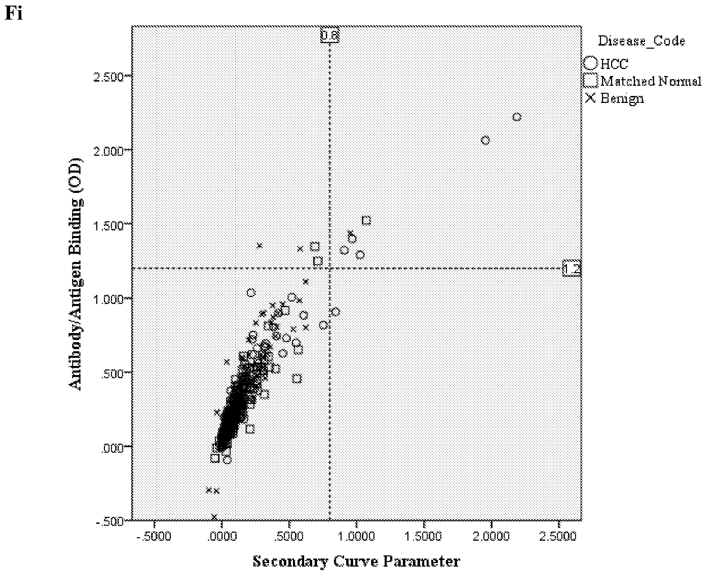

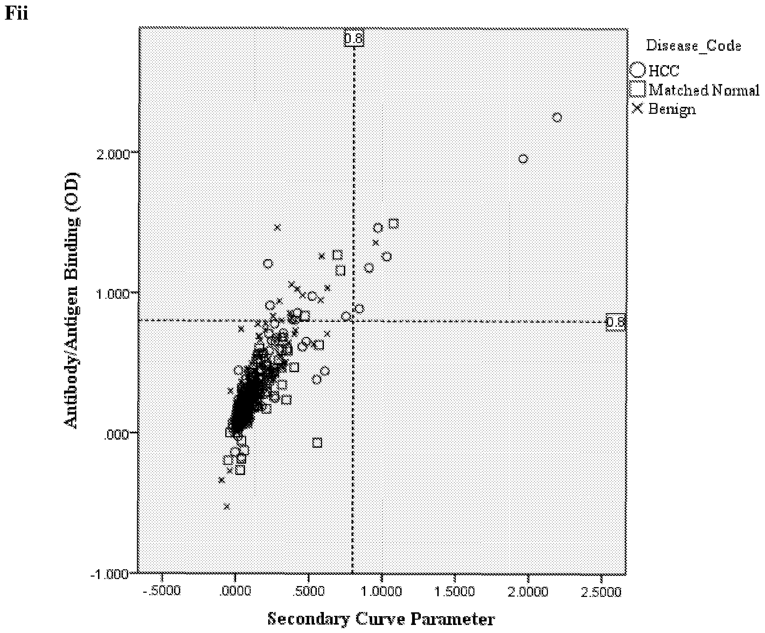

Figure 9: Addition of antigens to a diagnostic panel for HCC, incorporating Secondary Curve Parameters. Plots of secondary curve parameter versus antibody / antigen binding (expressed in optical density units (OD)) for an additional six antigens at two concentrations; (A = AFP), (B = p62), (C = SSX1), (D = HDGF), (E = VTN10), (F = IMP1), (i = antigen at 50nM concentration, ii = antigen at 160nM concentration). Cohort disease class is coded as follows: ○ = HCC, x = benign liver disease, □ = matched healthy normal. --------- represents the relative cut-off. The horizontal cut-off represents the standard antibody / antigen binding cut-off and the vertical cut-off represents the secondary curve parameter cut-off. Area shaded in dark grey represents zone of positivity.

## Detailed description of the invention

The invention in general provides an immunoassay method for detecting an antibody, characterised in that a sample to be tested for the presence of the antibody is tested for specific binding against different amounts of antigen specific for said antibody and a titration curve produced for antibody/antigen binding versus the amount of antigen tested. A secondary curve parameter is then calculated from the curve and the presence of the antibody determined on the basis of both the amount of specific binding between the antibody and the antigen and the secondary curve parameter.

The inventors have established that antigen/antibody binding in negative samples (e.g. samples from normal patients) previously categorised as false positives show different curve characteristics than specific binding in positive samples. The use of a double cut-off method, which looks at both the amount of antigen/antibody binding as well as a secondary curve parameter, therefore reduces the number of false positive results, increasing the specificity. Due to the low incidence of lung cancer in the population, the Positive Predictive Value (PPV) is primarily driven by the specificity of the assay and the method of the invention therefore also increases the PPV relative to assays based upon only the amount of specific binding between an antibody in the test sample.

Therefore, in a non-limiting embodiment the invention provides a method of detecting an antibody in a test sample comprising a bodily fluid from a mammalian subject, the method comprising the steps of:

(a) contacting said test sample with a plurality of different amounts of an antigen specific for said antibody;
(b) detecting the amount of specific binding between said antibody and said antigen;
(c) plotting or calculating a curve of the amount of said specific binding versus the amount of antigen for each amount of antigen used in step (a);
(d) calculating a secondary curve parameter from the curve plotted or calculated in step (c); and
(e) determining the presence or absence of said antibody based upon a combination of:

(i) the amount of specific binding between said antibody and said antigen determined in step (b); and
(ii) said secondary curve parameter determined in step (d).

The general features of immunoassays, for example ELISA, radioimmunoassays and the like, are well known to those skilled in the art (see Immunoassay, E. Diamandis and T. Christopoulus, Academic Press, Inc., San Diego, CA, 1996). Immunoassays for the detection of antibodies having a particular immunological specificity generally require the use of a reagent (antigen) that exhibits specific immunological reactivity with a relevant antibody. Depending on the format of the assay this antigen may be immobilised on a solid support. A sample to be tested for the presence of the antibody is brought into contact with the antigen and if antibodies of the required immunological specificity are present in the sample they will immunologically react with the antigen to form antibody-antigen complexes which may then be detected or quantitatively measured.

The method of the invention is characterised in that a standardised sample to be tested for the presence of the antibody is tested against a plurality of different amounts of antigen, also referred to herein as a set of antigens (this represents a titration series). The sample is tested against at least two, and preferably at least three, four, five, six or seven different amounts of the antigen. Typical assays may also include a negative control which does not contain any antigen.

In the context of the present invention the term "antigen" is used to refer to an immunospecific reagent which complexes with antibodies present in the test sample. An antigen is a substance comprising at least one antigenic determinant or epitope capable of interacting specifically with the target antibody it is desired to detect, or any capture agent interacting specifically with the variable region or complementary determining regions of said antibody. The antigen will typically be a naturally occurring or synthetic biological macromolecule such as for example a protein or peptide, a polysaccharide or a nucleic acid and can include antibodies or fragments thereof such as anti-idiotype antibodies.

Skilled readers will appreciate that in the method of the invention the amount of antigenic determinants or epitopes available for binding to the target antibody is important for establishing a titration series. In many assay formats the amount of antigenic determinants or epitopes available for binding is directly correlated with the amount of antigen molecules present. However, in other embodiments, such as certain solid phase assay systems, the amount of exposed antigenic determinants or epitopes may not correlate directly with the amount of antigen but may depend on other factors, such as attachment to the solid surface. In these embodiments, references herein to "different amounts of antigen" in a titration series may be taken to refer to different amounts of the antigenic determinant or epitope.

During the methods of the invention the relative or absolute amount of specific binding between antibody and antigen is determined for each different amount of antigen (antigenic determinant or epitope) tested and used to plot or calculate a curve of the (relative or absolute) amount of specific binding versus the amount of antigen for each amount of antigen tested. The presence in the test sample of antibody reactive with the antigen used in the assay is determined based upon the amount of specific binding observed at each antigen amount and is generally indicated by a dose-response curve, which is typically S-shaped or sigmoidal. If there is no variation in detectable binding over the different amounts of antigen tested then this can be scored as an absence of a detectable amount of the antibody.

In one embodiment, the presence or absence of antibody is determined by comparison of both the amount of specific binding between the antibody and the antigen and the secondary curve parameter with pre-determined cut-off values. Here, a curve of the amount of specific binding versus the amount of antigen for each amount of antigen used in the titration series is plotted, and the level of binding in known positive samples (e.g. a populations of patients with disease) are compared with the level of binding observed in known negative samples (e.g. normal individuals) in case-controlled studies. Cut-off values for antibody binding at one or more points on the titration curve are chosen that maximise sensitivity (few false negatives) while maintaining high specificity (few false positives). Provided the curve of the amount of specific binding versus the amount of antigen for each amount of antigen used in the titration series is a dose response curve, a measurement is considered to be positive if the amount of specific binding determined for one or more points on the titration curve is above the cut-off point determined.

A measure of the absolute amount of antibody present in a particular sample can, if desired, be derived from the results of antigen titration assays.

Secondary curve parameters

Following detection of the amount of antigen/antibody binding at each amount of antigen used in the titration series, and the plotting of a curve of the amount of specific binding versus the amount of antigen for each amount of antigen used in the titration series, a secondary curve parameter is calculated. The secondary curve parameter may be calculated from either a linear or logarithmic regression curve. Herein a secondary curve parameter is any calculated value which provides an indication of the nature of the curve. For example, the secondary curve parameter may be Slope, Intercept, Area Under the Curve (AUC), SlopeMax or dissociation constant (Kd). These secondary curve parameters are illustrated in Figure 1.

Slope is calculated using the equation:

$$b = \frac{\sum (x - \bar{x})(y - \bar{y})}{\sum (x - \bar{x})^2}$$

where b is the Slope, x refers to the antigen concentration (nM), and y refers to the OD value in absorbance units (AU).

Slope may be calculated from either the linear or logarithmic regression curves, or from both the linear and logarithmic regression curves, for each sample.

Intercept of the regression line is the value of the line at the y-axis when x=0.

Intercept may be calculated from either the linear or logarithmic regression curves, or from both the linear and logarithmic regression curves, for each sample.

Area Under the Curve (AUC) may be calculated using the summed trapezoid rule, which may be accomplished by estimating the definite integral between each set of antigen concentrations following the formula:

$$\int_a^b f(x)\,dx \approx (b-a)\left[\frac{f(a)+f(b)}{2}\right].$$

This calculation is repeated for each pair of consecutive antigen concentrations and the resulting values summed to give a total value for Area Under the Curve (AUC).

Area Under the Curve (AUC) may be calculated from either the linear or logarithmic regression curves, or from both the linear and logarithmic regression curves, for each sample.

SlopeMax may be calculated using the same formula as the Slope, discussed above. However to determine the greatest possible value for the Slope for each sample, a Slope value is obtained for each pair of consecutive antigen concentrations, with the Slope value of the greatest magnitude representing the SlopeMax.

SlopeMax may be calculated from either the linear or logarithmic regression curves, or from both the linear and logarithmic regression curves, for each sample.

Dissociation constant (Kd) may be calculated by fitting a four parameter logistic curve to each set of titration points and an iterative solve method is used to give values for the minimum asymptote (A), slope factor (B), inflection point (C) and maximum asymptote (D) parameters using the formula F(x)=((A-D)/(1+((x/C)^B)))+D, whereby the sum of the squared residuals is minimised. The inflection point for this solve data corresponds to the Kd of the antigen-antibody binding.

In another embodiment the secondary curve parameter may be determined by fitting a logistic curve, such as a 4 parameter logistic curve, to the curve of the amount of specific binding versus the amount of antigen for each amount of antigen used in the titration series. In this embodiment the secondary curve parameter may be Maximum Asymptote, Minimum Asymptote, Hill Slope (or Slope Factor) or Inflection Point.

A 4-Parameter Logistic (4PL) Curve is a curve defined by the formula:

$$F(x) = ((A\text{-}D)/(1+((x/C)^\wedge B))) + D,$$

where A = Minimum Asymptote, B = Hill Slope (or Slope Factor), C = Inflection Point and D = Maximum Asymptote.

To determine secondary curve parameters in this embodiment a 4PL curve is calculated for each sample and antigen using an iterative solve function. Here the 4 parameters are set at values near to the expected value for each, with the following constraints: the value of the Minimum Asymptote is fixed at 0, the value of the Hill Slope is limited to positive values, and the Inflection Point is limited to a maximum value of 1000.

The difference between each point of the titration curve, and the corresponding point on the 4PL curve (returned by the formula F(x) = ((A-D)/(1+((x/C)^B))) + D) can then be calculated, the differences squared, and the values of all the squared differences summed.

The values used for the 4 secondary curve parameters are then adjusted and the sum of the squared means calculated repeatedly in an iterative manner until the sum of the squared means is as close to zero as possible. The iterative solve may be performed using Microsoft Excel's SOLVER function.

Once a secondary curve parameter has been obtained it will be combined with the antigen/antibody binding data in order to determine the presence or absence of the antibody. Here, the amount of specific binding between the antibody and the antigen will be compared with a cut-off value as described above.

The cut-off for the secondary curve parameter is determined using known positive samples (e.g. a set of case-control sample sets consisting of a cohort of patients with disease) and known negative samples (e.g. a cohort of normal individuals in case-controlled studies). For each sample a curve of the amount of specific binding versus the amount of antigen for each amount of antigen used in the titration series is plotted, and the secondary curve parameters observed in the known positive sample (e.g. patients with disease) is compared with the secondary curve parameters observed in the known negative sample (e.g. normal individuals). Cut-off values for the secondary curve parameters are chosen that maximise specificity (few false positives) when used in combination with the cut-off for antibody / antigen binding discussed above.

Upon calculating the cut-off value for the secondary curve parameter, the directionality required for a positive reading, i.e. whether a value above or below the cut-off is considered positive, is also determined. The directionality required for a positive reading will depend upon the antigen and the secondary curve parameter.

A measurement is considered to be ultimately positive, i.e. indicative of the presence of antibody in the test sample, if it is both above the cut-off for antibody / antigen binding and demonstrates the directionality required for a positive reading compared to the cut-off for the secondary curve parameter.

The inventors have determined that including a secondary curve parameter in the assay methodology increases specificity of the immunoassay, increasing the Positive Predictor Value (PPV), compared with prior art methods based upon only the amount of specific binding between an antibody in the test sample.

It should be understood that although the description of the invention included herein is focussed upon the use of a single curve parameter in combination with measurement of the amount of antigen/antibody binding, the use of multiple secondary curve parameters is contemplated. Therefore, in certain embodiments the methods of the invention utilise two, three, four, five, six, seven, eight or more secondary curve parameters.

The method of the invention is advantageous for use in clinical diagnostic, prognostic, predictive and/or monitoring assays where the absolute amounts of target antibody present and the level of binding observed in the absence of the target antibody can vary enormously from patient-to-patient. The inventors have observed that if such assays are based on detection of antibody binding using a single amount/concentration of test antigen, patient samples containing an amount of antibody which is at the very low or the very high end of the normal physiological range of amount of antibody across the population can be missed due to limitations of the assay methodology; samples with a low amount of antibody may be scored as false negative results, whereas those with very high levels of antibody may be off the scale for accurate detection within the chosen assay methodology. Use of the titration method in combination with the calculation of a secondary curve parameter allows the inventors to account for these differences in antibody levels and differences in the level of binding observed.

The double cut-off antigen titration assay method of the invention is also particularly suitable for the detection of antibodies as biological markers of disease state or susceptibility where there is considerable patient-to-patient variation in the specificity and affinity of the antibodies for the target antigen. Antibody responses by their very nature can vary significantly from patient-to-patient, with variation occurring both in the absolute amounts of antibody present and in the specificity and/or affinity of the antibodies. The method of the invention can take account of this patient-to-patient variation, thus enabling a standard assay format to be developed for any given antibody.

Therefore, in a non-limiting embodiment, the invention provides a method of detecting a disease state or disease susceptibility in a mammalian subject, the method comprising the steps of:

(a) contacting a test sample comprising a bodily fluid from said mammalian subject with a plurality of different amounts of an antigen specific for an antibody associated with said disease state or disease susceptibility;
(b) detecting the amount of specific binding between said antibody and said antigen;
(c) plotting or calculating a curve of the amount of said specific binding versus the amount of antigen for each amount of antigen used in step (a);
(d) calculating a secondary curve parameter from the curve plotted or calculated in step (c); and
(e) determining the presence or absence of said disease state or disease susceptibility based upon a combination of:

(i) the amount of specific binding between said antibody and said antigen determined in step (b); and
(ii) said secondary curve parameter determined in step (d).

In a preferred embodiment of each aspect of the invention, the antibody is an autoantibody. Measurement of interactions between autoantibodies and their target antigens is challenging due to non-specific antibody binding in normal patients, which can present as a false positive result. The methods of the invention are particularly advantageous for the detection of autoantibody binding because they permit this low affinity non-specific binding to be distinguished from specific binding between an antigen, such as a tumour antigen, and its autoantibody.

The methods of the invention also provide a safeguard against day-to-day variation in the performance of immunoassays used for detection of autoantibodies/antibodies for diagnostic, prognostic and/or monitoring (disease state or therapy) purposes. It is often observed that there can be considerable day-to-day variation in signal strength when carrying out immunoassays for detection of antibodies in samples comprising patient bodily fluids. Such variation might arise, for example, because of differences in the way in which the samples were obtained and stored prior to testing. Such factors make it difficult to score the results of clinical assays with certainty, for example on the basis of a simple threshold value of antibody/antigen binding. The present invention minimises the effects of such day-to-day variation since the secondary curve parameter is likely to vary less from day to day than the measurement of an absolute signal level.

Still further advantages of the method of the invention over methods utilising only a single antigen concentration are that it permits a broader assay dynamic range than methods utilising a single antigen concentration. This means that further dilution of the patient sample to bring the result within the dynamic range is rarely required, and also that it will generally produce the same qualitative screening result (positive/negative) using bodily fluids from different sources in one individual (e.g. blood or serum versus ascites fluid or pleural effusion), even though the absolute concentration of antibodies may be different in the different fluids. It also means that the analytical sensitivity to low samples with low antibody abundance should be improved.

Assay formats

The methods of the invention may be carried out in any suitable format which enables contact between a sample suspected of containing the antibody and multiple different amounts of an antigen. Conveniently, contact between the sample and different amounts of the antigen may take place in separate but parallel reaction chambers such as the wells of a microtitre plate. Varying amounts of the antigen can be coated onto the wells of the microtitre plate by preparing serial dilutions from a stock of antigen across the wells of the microtitre plate. The stock of antigen may be of known or unknown concentration. Aliquots of the test sample may then be added to the wells of the plate, with the volume and dilution of the test sample kept constant in each well. The absolute amounts of antigen added to the wells of the microtitre plate may vary depending on such factors as the nature of the target antibody, the nature of the sample under test, dilution of the test sample etc. as will be appreciated by those skilled in the art. Generally the amounts of antigen and the dilution of the test sample will be selected so as to produce a range of signal strengths which fall within the acceptable detection range of the read-out chosen for detection of antibody/antigen binding in the method. Conveniently the tested amounts of antigen may vary in the range of from 1.6nM to 160mM.

As aforesaid, it is also possible to construct a titration curve starting with a single stock of antigen even when the absolute concentration of antigen in the stock is unknown. Provided that the same single stock solution is used and serially diluted in the same manner, it is possible to compare the results of separate titration assays for this antigen run on different starting test samples. The double cut-off method of the present application is particularly advantageous in this scenario since the secondary curve parameter will be consistent irrespective of whether the absolute antigen dilution is known.

In a further embodiment of the invention different amounts of the antigen (antigenic determinants or epitopes) may be immobilised at discrete locations or reaction sites on a solid support. The entire support may then be brought into contact with the test sample and binding of antibody to antigen detected or measured separately at each of the discrete locations or reaction sites. Suitable solid supports also include microarrays, for example arrays wherein discrete sites or spots on the array comprise different amounts of the antigen. Microarrays can be prepared by immobilising different amounts of a particular antigen at discrete, resolvable reaction sites on the array. In other embodiments the actual amount of immobilised antigen molecules may be kept substantially constant but the size of the sites or spots on the array varied in order to alter the amount of binding epitope available, providing a titration series of sites or spots with different amounts of available binding epitope. In such embodiments the two-dimensional surface concentration of the binding epitope(s) on the antigen is important in preparing the titration series, rather than the absolute amount of antigen. Techniques for the preparation and interrogation of protein/peptide microarrays are generally known in the art.

It will be understood from the above discussion that in all of the embodiments of the invention variation in the amount of antigen may be achieved by changing the antigen or epitope density against which the sample is tested, or by maintaining antigen or epitope density but increasing the surface area over which antigen is immobilised, or both. Microarrays may be used to perform multiple assays for antibodies of different specificity on a single sample in parallel. This can be done using arrays comprising multiple sets of antigens, each set comprising a particular antigen at multiple different amounts or concentrations.

The term "antigen" is used herein in a broad sense to refer to any substance which exhibits specific immunological reactivity with a target antibody to be detected. Suitable antigens may include, but are not limited to, naturally occurring proteins, recombinant or synthetic proteins or polypeptides, synthetic peptides, peptide mimetics, polysaccharides and nucleic acids. Specifically, where "antigen" is used herein it is intended to encompass any capture agent, whether of human origin, mammalian origin or otherwise, capable of specific immunological interaction with the variable or complementary determining regions of the antibody to be detected. For example anti-idiotypic antibodies may be regarded as an antigen for this purpose as may antigens generated by phage display. A set of antigens (or an antigen set) refers to a single antigen to be tested at different amounts/concentrations in the method of the invention.

Herein, the term "distinct antigens" encompasses antigens derived from different proteins or polypeptides (such as antigens derived from unrelated proteins encoded by different genes) and also antigens which are derived from different peptide epitopes of a single protein or polypeptide. A set of distinct antigens refers to a single antigen to be tested at different amounts/concentrations in the method of the invention. A given microarray may include exclusively sets of distinct antigens derived from different proteins or polypeptides, or exclusively sets of distinct antigens derived from different peptide epitopes of a single protein or polypeptide, or a mixture of the two in any proportion. It should be noted that each individual set of antigens of different amounts or concentrations in any embodiment of the invention will generally comprise just one antigen and not mixtures thereof.

The term, "antigen variant" is used herein to refer to allelic or other variants of a single antigen, such as a single protein antigen as defined above. Antigen variants will generally be derived from a single gene, and different antigen variants may be expressed in different members of the population or in difference disease states. Antigen variants may differ by amino acid sequence or by a post translational modification such as glycosylation, phosphorylation or acetylation. In addition, the term "antigen variant" encompasses antigen mutations such as amino acid substitutions, additions or deletions. Generally an antigen variant will contain less than five (e.g. less than four, less than three, less than two or one) mutations relative to the wild-type antigen. A set of antigen variants refers to a single antigen variant to be tested at different amounts/concentrations in the method of the invention.

An array useful for the methods of the present invention may comprise multiple sets of distinct antigens, multiple sets of antigen variants or multiple sets of both distinct antigens and antigen variants, each set comprising a particular antigen at multiple different amounts or concentrations. Specifically, an array may comprise one, two, three, four, five, six, seven, eight, nine, ten, eleven, twelve, thirteen, fourteen, fifteen, sixteen, seventeen, eighteen, nineteen, twenty or more sets of antigens.

Certain antigens may comprise or be derived from proteins or polypeptides isolated from natural sources, including but not limited to proteins or polypeptides isolated from patient tissues or bodily fluids (e.g. plasma, serum, whole blood, urine, sweat, lymph, faeces, cerebrospinal fluid, ascites, pleural effusion, seminal fluid, sputum, nipple aspirate, post-operative seroma and wound drainage fluid). In such embodiments the antigen may comprise substantially all of the naturally occurring protein, i.e. protein substantially in the form in which it is isolated from the natural source, or it may comprise a fragment of the naturally occurring protein. To be effective as an antigen in the method of the invention any such "fragment" must retain immunological reactivity with the antibodies for which it will be used to test. Suitable fragments might, for example, be prepared by chemical or enzymatic cleavage of the isolated protein.

As used herein the term "bodily fluid", when referring to the material to be tested for the presence of antibodies using the method of the invention, includes *inter alia* plasma, serum, whole blood, urine, sweat, lymph, faeces, cerebrospinal fluid, ascites, pleural effusion, seminal fluid, sputum, nipple aspirate, post-operative seroma, saliva, amniotic fluid, tears or wound drainage fluid. As aforesaid, the methods of the invention are preferably carried out *in vitro* on a test sample comprising bodily fluid removed from the test subject. The type of bodily fluid used may vary depending upon the identity of the antibody to be tested and the clinical situation in which the assay is used. In general, it is preferred to perform the assays on samples of serum or plasma. The test sample may include further components in addition to the bodily fluid, such as for example diluents, preservatives, stabilising agents, buffers etc.

Depending on the precise nature of the assay in which it will be used, the antigen may comprise a naturally occurring protein, or fragment thereof, linked to one or more further molecules which impart some desirable characteristic not naturally present in the protein. For example, the protein or fragment may be conjugated to a revealing label, such as for example a fluorescent label, coloured label, luminescent label, radiolabel or heavy metal such as colloidal gold. In other embodiments the protein or fragment may be expressed as a fusion protein. By way of example, fusion proteins may include a tag peptide at the N- or C-terminus to assist in purification of the recombinantly expressed antigen.

Depending on the format of the assay in which it is to be used the antigen may be immobilised on a solid support such as, for example, the wells of a microtitre plate, microarray beads or chips or magnetic beads. Immobilisation may be effected via noncovalent adsorption, covalent attachment or via tags.

Any suitable attachment means may be used provided this does not adversely affect the ability of the antigen to immunologically react with the target antibody to a significant extent.

The invention is not limited to solid phase assays, but also encompasses assays which, in whole or in part, are carried out in liquid phase, for example solution phase bead assays or competition assays.

In one embodiment, antigens may be labelled with a ligand that would facilitate immobilisation, such as biotin. The antigen can then be diluted to a suitable titration range and allowed to react with antibodies in patient samples in solution. The resulting immune complexes can then be immobilised on to a solid support via a ligand-receptor interaction (e.g. biotin-streptavidin) and the remainder of the assay performed as described below.

To facilitate the production of biotinylated antigens for use in the assay methods of the invention, cDNAs encoding a full length antigen, a truncated version thereof or an antigenic fragment thereof may be expressed as a fusion protein labelled with a protein or polypeptide tag to which the biotin co-factor may be attached, for example via an enzymatic reaction.

Vectors for the production of recombinant biotinylated antigens are commercially available from a number of sources. Alternatively, biotinylated antigens may be produced by covalent linkage of biotin to the antigen molecule following expression and purification.

As aforesaid, the "immunoassay" used to detect antibodies according to the invention may be based on standard techniques known in the art, with the exception that multiple amounts of antigen are used to create a titration curve and the curve is subsequently analysed to calculate a secondary curve parameter. In a most preferred embodiment the immunoassay may be an ELISA. ELISAs are generally well known in the art. In a typical "indirect" ELISA an antigen having specificity for the antibodies under test is immobilised on a solid surface (e.g. the wells of a standard microtiter assay plate, or the surface of a microbead or a microarray) and a sample comprising bodily fluid to be tested for the presence of antibodies is brought into contact with the immobilised antigen. Any antibodies of the desired specificity present in the sample will bind to the immobilised antigen. The bound antibody/antigen complexes may then be detected using any suitable method. In one preferred embodiment a labelled secondary anti-human immunoglobulin antibody, which specifically recognises an epitope common to one or more classes of human immunoglobulins, is used to detect the antibody/antigen complexes. Typically the secondary antibody will be anti-IgG or anti-IgM. The secondary antibody is usually labelled with a detectable marker, typically an enzyme marker such as, for example, peroxidase or alkaline phosphatase, allowing quantitative detection by the addition of a substrate for the enzyme which generates a detectable product, for example a coloured, chemiluminescent or fluorescent product. Other types of detectable labels known in the art may be used with equivalent effect.

Identity of antibodies

In some embodiments the antibody detected by the method of the invention may be associated with a disease or disease susceptibility. That is, the antibody may be a biological marker of a disease state or disease susceptibility. Within this embodiment the methods of the invention may be used to detect a disease state or a disease susceptibility.

In certain embodiments of the invention the antibody may be an autoantibody. The term "autoantibody" refers to a naturally occurring antibody directed to an antigen which an individual's immune system recognises as foreign even though that antigen actually originated in the individual. Autoantibodies include antibodies directed against altered forms of naturally occurring proteins produced by a diseased cell or during a disease process. The altered form of the protein originates in the individual but may be viewed by the individual's immune system as "non-self" and thus elicit an immune response in that individual in the form of autoantibodies immunologically specific to the altered protein. Such altered forms of a protein can include, for example, mutants having altered amino acid sequence, optionally accompanied by changes in secondary, tertiary or quaternary structure, truncated forms, splice variants, altered glycoforms etc. In other embodiments the autoantibody may be directed to a protein which is overexpressed in a disease state, or as a result of gene amplification or abnormal transcriptional regulation. Overexpression of a protein which is not normally encountered by cells of the immune system in significant amounts can trigger an immune response leading to autoantibody production. In still further embodiments the autoantibody may be directed to a foetal form of a protein which becomes expressed in a disease state. If a foetal protein which is normally expressed only in early stages of development before the immune system is functional becomes expressed in a disease state, the foetal form expressed in a disease state in the fully developed human may be recognised by the immune system as "foreign", triggering an immune response leading to autoantibody production.

In one embodiment the antibody may be an autoantibody specific for a tumour marker protein, and more particularly a "cancer-associated" anti-tumour autoantibody.

The term "cancer-associated anti-tumour autoantibody" refers to an autoantibody which is directed against an epitope present on forms of tumour marker proteins which are preferentially expressed in the cancer disease state. The presence of such autoantibodies is characteristic of the cancer disease state, or of pre-disposition to cancer in asymptomatic patients.

In preferred applications the method of the invention will be used to detect the presence of cancer-associated anti-tumour autoantibodies in human subjects or patients, and will most preferably take the form of an *in vitro* immunoassay, performed on a test sample comprising a sample of bodily fluid taken from the subject/patient. The sample of bodily fluid may be diluted in a suitable buffer or may be treated for long term storage or otherwise prior to testing.

*In vitro* immunoassays are non-invasive and can be repeated as often as is thought necessary to build up a profile of autoantibody production in a patient, either prior to the onset of disease, as in the screening of "at risk" individuals, or throughout the course of disease (further discussed below in relation to preferred applications of the method).

Panel assays

As discussed above, the use of a double cut off in the methods of the invention increases specificity, i.e. reduces the number of false positive results. This increase in specificity allows a greater number of antigens to be applied to the test sample simultaneously, increasing sensitivity, i.e. less false negatives than would be expected when additional antigens were applied to a test sample using prior art methods based upon only the amount of specific binding between an antibody in the test sample. In certain embodiments multiple antigens may be applied to the test sample in the form of a panel.

In a particular, but non-limiting, embodiment the invention therefore relates to a method of detecting two or more antibodies in a test sample comprising a bodily fluid from a mammalian subject, the method comprising the steps of:

(a) contacting said test sample with a panel comprising two or more sets of antigens, wherein each of said antigens in said panel is specific for at least one of said two or more antibodies;

(b) detecting complexes of said antigens bound to antibodies present in said test sample;

(c) for each antigen plotting or calculating a separate curve of the amount of said specific binding versus the amount of antigen for each amount of antigen used in step (a);

(d) calculating a secondary curve parameter for each curve plotted or calculated in step (c); and

(e) determining the presence or absence of said two or more antibodies based upon a combination of:

(i) the amount of specific binding between said antibody and said antigen determined in step (b); and

(ii) said secondary curve parameter determined in step (d),

for each of the curves plotted or calculated in step (c).

These methods, which may be hereinafter referred to as "panel assays", utilise a panel of two or more sets of antigens to monitor the overall immune response of an individual. These methods thus detect a "profile" of the immune response in a given individual. In one embodiment "panel assays" utilise a panel of two or more sets of antigens to monitor the overall immune response of an individual to a tumour or other carcinogenic/neoplastic change. These methods thus detect a "profile" of the immune response in a given individual, indicating which tumour markers elicit an immune response resulting in autoantibody production.

The use of a panel of two or more antigens to monitor production of antibodies is generally more sensitive than the detection of antibodies to single markers and gives a much lower frequency of false negative results (see WO 99/58978, WO 2004/044590 and WO2006/126008). It is generally accepted that the sensitivity of an assay will be increased by testing multiple antigens. However, this increased sensitivity is usually associated with a proportional decrease in specificity and prior art methods are therefore limited in the number of antigens which they can use. The methods of the present invention are not so limited because the increased specificity afforded by the double cut-off method permits a disproportionate increase in sensitivity for loss of specificity when multiple antigens are assayed. In some embodiments the methods of the invention therefore contemplate the use of a panel comprising multiple antigens, such as about 2, about 3, about 4, about 5, about 6, about 7, about 8, about 9, about 10, about 11, about 12, about 13, about 14, about 15, about 16, about 17, about 18, about 19, about 20 or more antigens.

In certain embodiments the two or more antigens of the panel may be distinct antigens, as defined previously to encompass antigens derived from different proteins or polypeptides (such as antigens derived from unrelated proteins encoded by different genes) and also antigens which are derived from different peptide epitopes of a single protein or polypeptide. In other embodiments, the two or more antigens of the panel may be antigen variants, as defined above to include allelic or other variants of a single antigen, generally derived from a single gene. The use of multiple antigen variants ensures that the specific antigen variant expressed by the patient and/or the tumour in question will be assayed. This increases the sensitivity of the assay method, with the double cut-off method of the invention ensuring that this increase in sensitivity is not accompanied by a proportional decrease in specificity. The Inventors have observed (Example 4) that individual patients may produce antibodies against a single antigen (e.g. p53) which have different specificities. It therefore appears as though not all patients produce the same altered forms of tumour associated antigens during the course of their disease. The invention therefore contemplates the use of different variant forms, or mutations, of a single antigen in order to improve the performance of the method of the invention.

In still further embodiments, the panel may comprise both distinct antigens and antigen variants. Within this embodiment the panel may comprise immunoassays to (simultaneously) detect one or more antigen variants (such as about 2, about 3, about 4, about 5, about 6, about 7, about 8, about 9, about 10, about 11, about 12, about 13, about 14, about 15, about 16, about 17, about 18, about 19, about 20 or more) of one or more (such as two, three, four, five, six, seven, eight, nine, ten or more) distinct antigens.

For each embodiment of the panel assay it will be apparent that the panel comprises a set of each antigen (i.e. of each distinct antigen and each antigen variant), wherein a set of antigens refers to a single antigen to be tested at different amounts/concentrations in the method of the invention.

Using the panel assays of the invention, the presence or absence of each target antibody is determined using the double cut off method for at least one of the curves produced using the panel assay. Specifically, the presence or absence of each target antibody will be determined based upon a combination of (i) the amount of specific binding between the antibody and the antigen and (ii) the secondary curve parameter for one or more of the curves plotted or calculated, such as one, two, three, four, five, six, seven, eight, nine, ten, eleven, twelve, thirteen, fourteen, fifteen, sixteen, seventeen, eighteen, nineteen, twenty or more of the calculated curves.

As described above in relation to non-panel assays, the presence or absence of antibody is determined by comparison of both the amount of specific binding between the antibody and the antigen and the secondary curve parameter with pre-determined cut-off values for each of the calculated curves. Here, for each antigen in the panel a curve of the amount of specific binding versus the amount of antigen for each amount of antigen used in the titration series is plotted, and the level of binding in known positive samples (e.g. populations of patients with disease) are compared with the level of binding observed in known negative samples (e.g. normal individuals) in case-controlled studies for each antigen in the panel. For each antigen, cut-off values for antibody binding at one or more points on the titration curve are chosen that maximise sensitivity (few false negatives) while maintaining high specificity (few false positives). Provided the curve of the amount of specific binding versus the amount of antigen for each amount of antigen used in the titration series is a dose response curve, a measurement is considered to be positive if the amount of specific binding determined for one or more points on the titration curve is above the cut-off point determined.

Once a secondary curve parameter has been obtained for each antigen it will be combined with the antigen/antibody binding data in order to determine the presence or absence of the antibody. Here, the amount of specific binding between the antibody and the antigen will be compared with a cut-off value as described above.

Here it should be noted that the secondary curve parameter calculated for each antigen within the panel need not necessarily be the same. However, in some embodiments the secondary curve parameter calculated for each antigen within the panel may be the same.

For each antigen, the cut-off for the secondary curve parameter is determined using known positive samples (e.g. a set of case-control sample sets consisting of a cohort of patients with disease) and known negative samples (e.g. a cohort of normal individuals in case-controlled studies). For each antigen, a curve of the amount of specific binding versus the amount of antigen for each amount of antigen used in the titration series is plotted, and the secondary curve parameters observed in the known positive sample (e.g. patients with disease) is compared with the secondary curve parameters for the same antigen observed in the known negative sample (e.g. normal individuals). Cut-off values for the secondary curve parameters are chosen that maximise specificity (few false positives) when used in combination with the cut-off for antibody / antigen binding discussed above.

Upon calculating the cut-off value for the secondary curve parameter, the directionality required for a positive reading, i.e. whether a value above or below the cut-off is considered positive, is also determined. The directionality required for a positive reading will depend upon the antigen and the secondary curve parameter.

A measurement is considered to be ultimately positive, i.e. indicative of the presence of antibody in the test sample, if it is both above the cut-off for antibody / antigen binding and demonstrates the directionality required for a positive reading compared to the cut-off for the secondary curve parameter.

In one embodiment, the panel assay may be used to detect the presence or absence of a disease state or disease susceptibility. Therefore, in one embodiment the invention includes a method of detecting a disease state or disease susceptibility in a mammalian subject, the method comprising the steps of:

(a) contacting a test sample comprising a bodily fluid from said mammalian subject with a panel comprising two or more sets of antigens specific for antibodies associated with a disease state or disease susceptibility;
(b) detecting complexes of said antigens bound to antibodies present in said test sample;
(c) for each antigen plotting or calculating a separate curve of the amount of said specific binding versus the amount of antigen for each amount of antigen used in step (a);
(d) calculating a secondary curve parameter for each curve plotted or calculated in step (c); and
(e) determining the presence or absence of said disease state or disease susceptibility based upon a combination of:

(i) the amount of specific binding between said antibody and said antigen determined in step (b); and
(ii) said secondary curve parameter determined in step (d),

for one or more of the curves plotted or calculated in step (c).

Within this embodiment the presence or absence of the disease state or disease susceptibility is determined based upon a combination of (i) the amount of specific binding between the antibody and the antigen and (ii) the secondary curve parameter for one or more of the curves plotted or calculated in step (c) with cut-off values being determined as described above.

Tumour marker proteins

The method of the invention is not limited to the detection of antibodies with any certain specificity. However, in one embodiment the method of the invention may be adapted for use in the detection of autoantibodies to a tumour marker protein. Herein the method may relate to the detection of autoantibodies to essentially any tumour marker protein for which a suitable antigen may be prepared, as a single marker assay or as a component of a panel assay.

In particular, the method may be adapted to detect/measure autoantibodies to the epidermal growth factor receptor protein EGFR (Downward et al (1984) Nature. 307: 521-527; Robertson et al. (2001) Archives of Pathology and Laboratory Medicine 126;177-81), the glycoprotein MUC1 (Batra, S. K. et al. (1992) Int. J. Pancreatology. 12: 271-283), the signal transduction/cell cycle regulatory proteins Myc (Blackwood, E. M. et al. (1994) Molecular Biology of the Cell 5: 597-609), c-myc, L-myc2 (Nau et al., Nature, 318(6041), 69-73), p53 (Matlashewski, G. et al. (1984) EMBO J. 3: 3257-3262; Wolf, D. et al. (1985) Mol. Cell. Biol. 5: 1887-1893) and ras (or Ras) (Capella, G. et al. (1991) Environ Health Perspectives. 93: 125-131), and also BRCA1 (Scully, R. et al. (1997) PNAS 94: 5605-10), BRCA2 (Sharan, S. K. et al. (1997) Nature. 386: 804-810), APC (Su, L. K. et al. (1993) Cancer Res. 53: 2728-2731; Munemitsu, S. et al. (1995) PNAS 92: 3046-50), CA125 (Nouwen, E. J. et al. (1990) Differentiation. 45: 192-8), PSA (Rosenberg, R. S. et al. (1998) Biochem Biophys Res Commun. 248: 935-939), carcinoembryonic antigen CEA (Duffy, M.J. (2001) Clin Chem, Apr 47(4):624-30), CA19.9 (Haga, Y. et al (1989) Clin Biochem (1989) Oct 22(5): 363-8), NY-ESO-1, (cancer/testis antigen; Chen, Y.-T. et al., Proc. Nat. Acad. Sci. 94: 1914-1918, 1997), PSMA (prostate specific membrane antigen; Israeli, R. S. et al., Cancer Res. 53: 227-230, 1993), PSCA (prostate stem cell antigen; Reiter, R. E. et al., Proc. Nat. Acad. Sci. 95: 1735-1740, 1998) and EpCam (epithelial cellular adhesion molecule; Szala, S. et al., Proc. Nat. Acad. Sci. 87: 3542-3546, 1990), HER2 (also known as c-erbB2 Coussens, L. et al., Science 230: 1132-1139, 1985), CAGE (Jager D, et al., Cancer Res. 1999 Dec 15;59(24):6197-204; Mashino K, et al., Br J Cancer. 2001 Sep 1;85(5):713-20), CAGE1, CAGE2, cytokeratins (Moll R, et al., Cell. 1982 Nov;31(1):11-24; Braun S, et al., N Engl J Med. 2000; 342: 525-533), recoverin (Maeda A, et al., Cancer Res. 2000 Apr 1;60(7):1914-20, kallikreins (Kim H, et al., Br J Cancer 2001;84:643-650; Yousef GM, et al., Tumor Biol 2002;23:185-192); annexins (Hudelist G, et al., Breast Cancer Res Treat. 2004 Aug;86(3):281-91), $\alpha$-fetoprotein (AFP; Stiller D, et al., Acta Histochem Suppl. 1986;33:225-31), GRP78 (Block TM, et al., Proc Natl Acad Sci USA. 2005 Jan 18;102(3):779-84; Hsu WM, et al., Int J Cancer. 2005 Mar 1;113(6):920-7), mammoglobin (Zehentner BK, et al., Clin Chem. 2004 Nov;50(11):2069-76; Zehentner BK, Carter D. Clin Biochem. 2004 Apr;37(4):249-57), raf (Callans LS. et al., Ann Surg Oncol. 1995 Jan;2(1):38-42; Pratt MA, et al., Mol Cell Biochem. 1998 Dec;189(1-2):119-25), beta-human chorionic gonadotropin b-HCG (Ayala AR, et al., Am J Reprod Immunol. 1983 Apr-May;3(3):149-51; Gregory JJ Jr, et al., Drugs. 1999 Apr;57(4):463-7), or 4-5 antigen (Krause P, et al., J Immunol Methods. 2003 Dec;283(1-2):261-7), SOX2 (Stevanovic et al., Mammalian genome : official journal of the International Mammalian Genome Society, 1994, 5(10), 640-2), GBU4-5 (Krause et al., J Immunol Methods 2003; 283: 261-7), MAGE (Serrano, et al., International journal of cancer. Journal international du cancer, 1999, 83(5), 664-9), HuD (Szabo et al., Cell, 1991, 67(2), 325-33), HE4, SALL4 (Al-Baradie et al., American journal of human genetics, 2002, 71(5), 1195-9), alpha enolase (Lee et al., Arthritis and rheumatism, 2003, 48(7), 2025-35), p62 (Zhang et al., The Journal of experimental medicine, 1999, 189(7), 1101-10), RalA (Bhullar et al., FEBS Letters, 1990, 260(1), 48-52), Cyclin B1(Pines et al., The Journal of cell biology, 1991, 115(1), 1-17), Calreticulin R (Opas et al., Journal of cellular physiology, 1991,149(1), 160-71), IMP1(Nielsen et al., Molecular and cellular biology, 1999, 19(2), 1262-70), Vitronectins (e.g. VTN10; Hayman et al., Proceedings of the National Academy of Sciences of the United States of America, 1983, 80(13), 4003-7), SSX1 (Crew et al., The EMBO journal, 1995, 14(10), 2333-40), TMP21(Blum et al. Journal of Biological Chemistry, 1996, 271(29), 17183-17189), NPC1L1C (Davies et al., Genomics, 2000, 65(2), 137-45), TMOD1 (Sung et al., The Journal of biological chemistry, 1992, 267(4), 2616-21), CAMK1 (Haribabu et al., The EMBO journal, 1995, 14(15), 3679-86), RGS1 (Watson et al., Nature, 1996, 383(6596), 172-5), PACSIN1 (Qualmann et al., Molecular biology of the cell, 1999, 10(2), 501-13), RCV1 (Investigative ophthalmology & visual science, 34(1), 81-90), MAPKAPK3 (Sithanandam et al., Molecular and cellular biology, 1996, 16(3), 868-76), CyclinE2 (Lauper et al., Oncogene, 1998, 17(20), 2637-43), cytokeratin 8 (CK8), 18 (CK18), 19 (CK19), 20 (CK20), SCC (Kato et al., Cancer, 1977, 40(4), 1621-1628), proGRP, serum Amyloid A (Rosenthal et al., Journal of immunology (Baltimore, Md.: 1950), 1976,116(5), 1415-8), alpha-1-anti-trypsin (Laurell et al., Scandinavian Journal of Clinical & Laboratory Investigation, 1963, 15(2), 132-140), apolipoproteinCIII (Ginsberg *et al., 1986*), thymo$\beta$in $\beta$4 (Dalakas *et al., 1986*), HDJ1 (Ohtsuka, Biochemical and biophysical research communications, 1993, 197(1), 235-40) and HDGF (Zhou et al. (2010). "Overexpressed HDGF as an independent prognostic factor is involved in poor prognosis in Chinese patients with liver cancer." Diagn Pathol 5: 58). However, the invention is not intended to be limited to the detection of autoantibodies to these particular tumour markers.

Applications of methods of the invention

Assay methods according to the invention, and specifically methods which detect autoantibodies directed to tumour marker proteins (in single marker or panel assay form), may be employed in a variety of different clinical situations. In particular, the method may be used in the detection or diagnosis of cancer, in assessing the prognosis of a patient diagnosed with cancer, in predicting response to therapy, in monitoring the progress of cancer or other neoplastic disease in a patient, in detecting early neoplastic or early carcinogenic change in an asymptomatic human subject, in screening a population of asymptomatic human subjects in order either to identify those subjects who are at increased risk of developing cancer or to diagnose the presence of cancer, in predicting the response of a cancer patient to anti-cancer treatment (e.g. surgical resection, vaccination, anti-growth factor or signal transduction therapies, radiotherapy, endocrine therapy, human antibody therapy, chemotherapy), in monitoring the response of a cancer patient to anti-cancer treatment (e.g. surgical resection, vaccination, anti-growth factor or signal transduction therapies, radiotherapy, endocrine therapy, human antibody therapy chemotherapy), in the detection of recurrent disease in a patient previously diagnosed as having cancer who has undergone anti-cancer treatment to reduce the amount of cancer present, or in the selection of an anti-cancer therapy (e.g. surgical resection, vaccine, anti-growth factor or signal transduction therapies, radiotherapy, endocrine therapy, human antibody treatment chemotherapy), for use in a particular patient.

The inventors have generally observed that levels of cancer-associated autoantibodies show a positive correlation with disease state (see also WO 99/58979). In addition, the inventors have now observed that certain secondary curve parameters are associated with a disease state. Hence, when the method of the invention is used in clinical applications increased levels of anti-tumour marker autoantibodies or an altered secondary curve parameter, as compared to suitable controls, are generally taken as an indication of the cancer disease state.

For example, when the immunoassays are used in the diagnosis of cancer, the presence of an elevated level of autoantibodies or an altered secondary curve parameter showing the required directionality, as compared to "normal" control individuals, is taken as an indication that the individual has cancer. The "normal" control individuals will preferably be age-matched controls not having any diagnosis of cancer based on clinical, imaging and/or biochemical criteria.

When the immunoassays are used in predicting the response of a cancer patient to anti-cancer treatment (e.g. surgical resection, vaccination, anti-growth factor or signal transduction therapies, radiotherapy, endocrine therapy, human antibody therapy, chemotherapy), the presence of an elevated level of autoantibodies or an altered secondary curve parameter showing the required directionality, as compared to "normal" control individuals, may be taken as an indication of whether or not the individual is likely to respond to the anti-cancer treatment. The "normal" control individuals will preferably be age-matched controls not having any diagnosis of cancer based on clinical, imaging and/or biochemical criteria. A relationship between the level of autoantibodies or the secondary curve parameter, compared to controls, and likely success of treatment can be established by observation of the outcome of such treatment in patients whose autoantibody status and secondary curve parameter is monitored throughout treatment. The previously established relationship may then be used to predict the likelihood of success for each treatment in a given patient based on assessment of autoantibody status and secondary curve parameter.

When the immunoassays are used in monitoring the progression of cancer or other neoplastic disease in a patient, the presence of an elevated level of autoantibodies or an altered secondary curve parameter showing the required directionality, as compared to a "normal control", is taken as an indication of the presence of cancer in the patient. The "normal control" may be levels of autoantibodies and secondary curve parameter present in control individuals, preferably age-matched, not having any diagnosis of cancer based on clinical, imaging and/or biochemical criteria. Alternatively, the "normal control" may be a "base-line" level established for the particular patient under test. The "base-line" level may be, for example, the level of autoantibodies and secondary curve parameter present when either a first diagnosis of cancer or a diagnosis of recurrent cancer was made. Any increase above the base-line level of autoantibodies or any alteration in the secondary curve parameter showing the required directionality would be taken as an indication that the amount of cancer present in the patient has increased, whereas any decrease below the base-line level of autoantibodies or any alteration in the secondary curve parameter showing a reverse of the directionality required for a positive reading would be taken as an indication that the amount of cancer present in the patient has decreased. The "base-line" value may also be, for example, the level before a new treatment is commenced. A change in the level of autoantibodies or a change in the secondary curve parameter showing the required directionality would be taken as an indication of the effectiveness of the therapy. For any given treatment the direction of the "change" in autoantibody levels or secondary curve parameter indicating a positive result (i.e. whether a value above or below the cut-off is considered positive) will be determined upon calculating the cut-off values. The directionality required for a positive reading will depend upon the antigen and the secondary curve parameter.

When the immunoassays are used in screening a population of asymptomatic human subjects this may be to identify those subjects who are at increased risk of developing cancer. Individuals having an elevated level of autoantibodies or an altered secondary curve parameter, as compared to "normal" control individuals, are identified as being "at risk" of developing cancer. The "normal" control individuals will preferably be age-matched controls not identified as having any predisposition to developing cancer or any significant elevated risk of developing cancer. An exception to this may be where age itself is a major risk factor.

When the immunoassays are used in screening a population of asymptomatic human subjects this may be to diagnose cancer in those subjects who have already developed a cancer Individuals having an elevated level of autoantibodies or an altered secondary curve parameter as compared to "normal" control individuals being scored as having cancer or some form of neoplastic change. The "normal" control individuals will preferably be age-matched controls not identified as having any predisposition to developing cancer. An exception to this may be where age itself is a major risk factor. Alternatively, the "normal control" may be a "base-line" level established for the particular patient under test. The "base-line" level may be, for example, the level of autoantibodies and secondary curve parameter present when the patient was first tested and found to have levels not elevated above a "normal control" population. Any increase thereafter against this baseline measurement would be taken as an indication of the presence of cancer in that individual. Thus the individual could, through such a baseline test, become their own control for future autoantibody measurement.

When the immunoassays are used in monitoring the response of a cancer patient to anti-cancer treatment (e.g. surgical resection, vaccination, anti-growth factor or signal transduction therapies, radiotherapy, endocrine therapy, human antibody therapy, chemotherapy), the presence of an altered level of autoantibodies or an altered secondary curve parameter after treatment is taken as an indication that the patient has responded positively to the treatment. A base-line level of autoantibodies and a base-line secondary curve parameter taken before treatment is commenced may be used for comparison purposes in order to determine whether treatment results in an "increase or decrease" in autoantibody levels. A change in the level of autoantibodies or secondary curve parameter showing the required directionality would be taken as an indication of the effectiveness of the therapy. For any given treatment the direction of the "change" in autoantibody levels or secondary curve parameter indicating a positive result (i.e. whether a value above or below the cut-off is considered positive) will be determined upon calculating the cut-off values. The directionality required for a positive reading will depend upon the antigen and the secondary curve parameter.

The method of the invention may be used in predicting and/or monitoring response of an individual to essentially any known anti-cancer treatment. This includes, for example human antibody therapy wherein monoclonal or polyclonal antibodies are infused into the patient, a non-limiting specific example being treatment with the anti-growth factor antibody Herceptin™ (Baselga, J., D. Tripathy et al., J Clin Oncol., 14(3), 737-744, 1996). The presence of a natural autoantibody response may enhance or inhibit the effectiveness of treatment with artificially infused therapeutic antibodies. Using the method of the invention it is possible to correlate response to any anti-cancer treatment, including antibody therapy, with natural levels of autoantibodies and secondary curve parameter prior to and over the course of the treatment in any patient or group of patients. This knowledge may then in turn be used to predict how other patients (or the same patient in the case of repeated treatment) will respond to the same treatment.

When the immunoassays are used in detection of recurrent disease, the presence of an increased level of autoantibodies or an altered secondary curve parameter showing the required directionality in the patient, as compared to a "normal control", is taken as an indication that disease has recurred. The "normal control" may be levels of autoantibodies and secondary curve parameter present in control individuals, preferably age-matched not having any diagnosis of cancer based on clinical, imaging and/or biochemical criteria. Alternatively, the "normal control" may be a "base-line" level established for the particular patient under test. The "base-line" level may be, for example, the level of autoantibodies and secondary curve parameter present during a period of remission from disease based on clinical, imaging and/or biochemical criteria.

Throughout the various applications of the methods of the invention it has been indicated that the presence of an altered level of autoantibodies or an altered secondary curve parameter showing the required directionality are indicative of a positive result. For the avoidance of doubt it should be understood that in some instances the presence of an altered level of autoantibodies and an altered secondary curve parameter showing the required directionality may be required for a positive result.

The assay method of the invention may be applied in the detection of many different types of cancer, of which examples are breast, bladder, colorectal, prostate, lung, pancreatic, ovarian cancers, hepatocellular carcinoma (HCC), gastrointestinal and melanoma. The assays may complement existing methods of screening and surveillance. For example, in the case of primary breast cancer, immunoassays for autoantibodies could be used to alert clinicians to biopsy small lesions on mammograms which radiographically do not appear suspicious or to carry out breast imaging or to repeat imaging earlier than planned. In the clinic, the assay methods of the invention are expected to be more objective and reproducible compared to current imaging techniques (i.e. mammography and ultrasound), the success of which can be operator-dependent.

"Panel assays" may be tailored having regard to the particular clinical application. A panel of antigens for detection of autoantibodies to at least p53 and c-erbB2 is particularly useful for many types of cancer and can optionally be supplemented with other markers having a known association with the particular cancer, or a stage of the particular cancer, to be detected. For example for breast cancer the panel might include MUC 1 and /or c-myc and/or BRCA1 and/or BRCA2 and/or PSA whereas bladder cancer the panel might optionally include MUC 1 and/or c-myc, for colorectal cancer ras and/or APC, for prostate cancer PSA and/or BRCA 1 and/or BRCA2 or for ovarian cancer BRCA1 and/or BRCA2 and/or CA125, for lung cancer SOX2 and/or CAGE, for hepatocellular carcinoma alphafetoprotein. There are other preferred embodiments in which p53 or c-erbB2 are not necessarily essential.

The utility of the method of the invention is not limited to detection of anti-tumour autoantibodies, although the assay is particularly useful for this purpose. Cancer is just one example of a disease wherein detection of antibody levels and a secondary curve parameter may be used as a biological marker for disease state/disease susceptibility. The inventors have shown that substantial advantages are gained by the use of a titration approach to detect antibodies in patient samples, and subsequently calculate a secondary curve parameter in order to perform a double cut-off method. It is therefore reasonable to conclude that similar advantages will be gained by the use of this approach to detect autoantibodies that are biological markers for diseases other than cancer. The method is therefore applicable to detection of any autoantibody which serves as a biological marker for a disease state or disease susceptibility, in any disease which has been shown (or can be shown) to be associated with autoantibody production.

Other applications of the method of the invention include, but are not limited to, detection of autoantibodies that are biological markers of autoimmune disease, such as for example rheumatoid arthritis, systemic lupus erythematous (SLE), primary biliary cirrhosis (PBC), autoimmune thyroiditis (e.g. Hashimoto's thyroiditis), autoimmune gastritis (e.g. pernicious anaemia), autoimmune adrenalitis (e.g. Addison's disease), autoimmune hypoparathyriodism, autoimmune diabetes (e.g. Type 1 diabetes) or myasthenia gravis, paraneoplastic neurological disorders such as Lambert-Eaton Myasthenic Syndrome, inflammatory bowel disease, sarcoidosis and autoimmune hepatitis screening of patient samples for kidney or hepatic disease leading to insufficiency or failure of either organ, and for screening of patient samples post-transplantation to detect the presence of antibodies directed against either the diseased tissue (which has been left in-situ post-transplantation) or against the transplanted tissue.

Kits for performing the methods of the invention

The present invention encompasses uses of a kit suitable for performing any one of the methods of the invention, wherein the kit comprises one or more antigens and a reagent capable of detecting complexes of the antigens bound to antibodies present in a test sample comprising a bodily fluid from a mammalian subject.

In certain embodiments the kit may comprise two or more antigen variants, such as two, three, four, five, six, seven, eight, nine, ten or more antigen variants.

In further embodiments the kit may comprise means for contacting said one or more antigens with a sample of bodily fluids. The invention will be further understood with reference to the following non-limiting experimental examples.

**Examples**

*Example 1 - Development of secondary curve parameter metrics*

Sample set

For the development of secondary curve parameter metrics the training set comprised 337 serum or plasma samples from patients with primary lung cancer taken at diagnosis and prior to treatment (cancers). These were matched (for age, gender and smoking history) to 415 serum or plasma samples from individuals with no evidence of malignancy (normals).

Standard cut-off for antibody / antigen binding

Antibody / antigen binding was measured by ELISA as described elsewhere (WO06/126008 & Murray et al. Ann Oncol. 2010 Aug 21(8): 1687-93). Data was obtained for binding to a range of concentrations of a panel of seven tumour associated antigens (p53, SOX2, CAGE, NY-ESO-1, GBU4-5, MAGE A4 and HuD) plus a control (VOL) from 1.6 to 160nM to provide titration curves for autoantibody binding (Figure 2). The cut-off for antibody / antigen binding was determined from matched case control studies by plotting a curve of the amount of specific binding versus the amount of antigen used in the titration series for each antigen in the panel. The amount of binding for each antigen at 160 and 50nM was calibrated and converted to a reference unit (RU) as described in WO09/081165. Cut-off values for antibody binding at one or more points on the titration curve were chosen to maximise sensitivity (the proportion of cancer patient samples that were positive) while maintaining high specificity (the proportion of samples from normal individuals that were negative). A measurement was considered to be positive if it resulted in a sigmoidal curve plotted from the reaction of antibody in the sample with the antigen titration series and if the value determined for one or more points on the titration curve was above the pre-determined cut-off.

Secondary curve parameters

Following detection of the amount of antigen/antibody binding to each amount of antigen used in the titration series, and the plotting of a curve of the amount of specific binding versus the amount of antigen for each amount of antigen used in the titration series, a secondary curve parameter was calculated. The secondary curve parameter may be calculated from either a linear or logarithmic regression curve. Herein a secondary curve parameter is any calculated value which provides an indication of the nature of the curve. For example, the secondary curve parameter may be Slope, Intercept, Area Under the Curve (AUC), SlopeMax or dissociation constant (Kd).

Slope is calculated using the equation:

$$b = \frac{\sum (x - \bar{x})(y - \bar{y})}{\sum (x - \bar{x})^2}$$

where b is the Slope, x refers to the antigen concentration (nM), and y refers to the OD value in absorbance units (AU).

Slope may be calculated from either the linear or logarithmic regression curves, or from both the linear and logarithmic regression curves, for each sample.

Intercept of the regression line is the value of the line at the y-axis when x=0.

Intercept may be calculated from either the linear or logarithmic regression curves, or from both the linear and logarithmic regression curves, for each sample.

Area Under the Curve (AUC) may be calculated using the summed trapezoid rule, which may be accomplished by estimating the definite integral between each set of antigen concentrations following the formula:

$$\int_a^b f(x)\,dx \approx (b-a)\left[\frac{f(a)+f(b)}{2}\right].$$

This calculation is repeated for each pair of consecutive antigen concentrations and the resulting values summed to give a total value for Area Under the Curve (AUC).

Area Under the Curve (AUC) may be calculated from either the linear or logarithmic regression curves, or from both the linear and logarithmic regression curves, for each sample.

SlopeMax may be calculated using the same formula as the Slope, discussed above. However to determine the greatest possible value for the Slope for each sample, a Slope value is obtained for each pair of consecutive antigen concentrations, with the Slope value of the greatest magnitude representing the SlopeMax.

SlopeMax may be calculated from either the linear or logarithmic regression curves, or from both the linear and logarithmic regression curves, for each sample.

Dissociation constant (Kd) may be calculated by fitting a four parameter logistic curve to each set of titration points and an iterative solve method is used to give values for the minimum asymptote (A), slope factor (B), inflection point (C) and maximum asymptote (D) parameters using the formula F(x)=((A-D)/(1+((x/C)^B)))+D, whereby the sum of the squared residuals is minimised. The inflection point for this solve data corresponds to the Kd of the antigen-antibody binding.

Once a secondary curve parameter was obtained it was combined with the antigen/antibody binding data in order to determine the presence or absence of the antibody.

The threshold or cut-off for the secondary curve parameter was determined by plotting a curve of the amount of specific binding versus the amount of antigen for each amount of antigen used in the titration series. This was performed for both cancers and normals using the same measurements that were derived for quantification of antibody / antigen binding. Cut-off values for the secondary curve parameters were chosen that maximise specificity (the proportion of samples from normal individuals that are negative) when used in combination with the cut-off for antibody / antigen binding. Upon calculating the cut-off value for the secondary curve parameter, the directionality required for a positive reading, i.e. whether a value above or below the cut-off is considered positive, is also determined. The directionality required for a positive reading will depend upon the antigen and the secondary curve parameter.

A measurement was considered to be ultimately positive if it was both above the cut-off for antibody / antigen binding and showed an altered secondary curve parameter having the required directionality compared to the cut-off.

Results

Figure 3A-D shows the distribution of samples in the training sample set according to their antibody / antigen binding level versus each individual secondary curve parameter. The individual samples are coded according to the result that was obtained by applying only the standard antibody / antigen binding cut-off. Although this data was calculated for all seven antigens in the panel, p53 is shown as an example in order to demonstrate the effects of the different parameters. It can be seen that the distribution of samples differs between linear and log regression data reduction hence both may be useful for identifying false positive samples and thus improving the performance of the test as a whole.

Secondary curve parameter cut-offs (Table 1) were optimised for the reclassification of false positives to true negatives and applied simultaneously to both antibody / antigen binding levels and secondary curve parameter.

*Table 1: Development of secondary curve parameters*

| Antigen | Antibody / antigen binding single parameter cut-off (RU) | Antibody / Antigen binding two parameter cut-off (RU) | Secondary curve parameter | Log/linear regression | Secondary curve parameter cut-off |
|---|---|---|---|---|---|
| p53 | 8.35 | 6.00 | Intercept | Log | > 0.15 |

(continued)

| Antigen | Antibody / antigen binding single parameter cut-off (RU) | Antibody / Antigen binding two parameter cut-off (RU) | Secondary curve parameter | Log/linear regression | Secondary curve parameter cut-off |
|---------|---------|---------|---------|---------|---------|
| SOX2 | 8.26 | 8.26 | AUC | Linear | > 27.0 |
| CAGE | 2.86 | 2.86 | UC | Log | > 2.80 |
| NY-ESO-1 | 6.38 | 6.38 | Intercept | Log | > -0.12 |
| MAGE A4 | 9.29 | 9.29 | AUC | Linear | > 25.0 |
| HuD | 9.53 | 9.53 | Slopemax | Linear | > 0.13 |

The distribution of samples according to their antibody / antigen binding versus the optimal secondary curve parameter are shown in Figure 4A-F. The horizontal cut-off represents the standard antibody / antigen binding cut-off and the vertical cut-off represents the secondary curve parameter cut-off. The quadrant that is positive for both cut-offs is known as the zone of positivity and it is clear that there was enrichment of true positives within this zone whereas samples that were previously false positive were mainly excluded and reclassified as true negatives by the application of the secondary curve parameter cut-off.

The performance of the autoantibody panel with and without the application of the secondary curve parameter cut-off is shown in Table 2.

*Table 2: Performance characteristics of an autoantibody test with application of single and double cut-off*

| Cut-off | Specificity (%) | Sensitivity (%) | PPV (%) | False calls per 1000 tests run | |
|---------|---------|---------|---------|---------|---------|
| | | | | Normal | Cancer |
| Standard antibody / antigen binding cut-off | 90.1 | 29.7 | 5.8 | 97.0 | 14.1 |
| Double cut-off method | 98.1 | 22.0 | 19.1 | 18.6 | 15.6 |

Although sensitivity was reduced by the reclassification of a few true positive samples to false negative, the specificity was improved from 90% to 98%. This represented an improvement in positive predictive value (PPV) from 5.8% (1 in every 17.2 patients with a positive tests has cancer) to 19.1% (1 in every 5.2 patients with a positive test has cancer). In a disease such as lung cancer with a prevalence in a high risk population of around 2% (i.e. 1 in 50), an 8% gain in specificity would result in about 78 less people who do not have cancer having a positive test for every 1000 people tested. These patients will be saved the anxiety and risk of follow-up tests such as CT imaging and biopsy. However in the same population a 7% loss in sensitivity will result in only 1.5 more patients with cancer being mis-diagnosed (Table 2). Confirmation of these results in a second large data set was required in order to validate this approach.

*Example 2 - Confirmation of developmental results in an independent sample set*

Sample set

For confirmation of the improved assay performance afforded by application of the double cut-off method, the sample set comprised 235 serum or plasma samples from patients with primary lung cancer taken at diagnosis and prior to treatment (cancers). These were matched (for age, gender and smoking history) to 266 serum or plasma samples from individuals with no evidence of malignancy (normals). There was no cross over between samples in the training and confirmation sample sets.

Secondary curve parameters and cut-off determination

Antibody / antigen binding was measured by ELISA as described in Example 1. Secondary curve parameters and directionality required for a positive reading were also as described in Example 1. Both standard antibody / antigen binding and secondary curve parameter cut-offs were determined as described in example 1 with slight adjustment for optimisation across both sample sets.

Results

Comparison of the training set with the confirmation set is shown in Figure 5A-F which depicts the distribution of sample signals according to their antibody / antigen binding versus the optimal secondary curve parameter. The horizontal cut-off represents the standard antibody / antigen binding cut-off and the vertical cut-off represents the secondary curve parameter cut-off. There was enrichment of true positives within the zone of positivity whereas samples that were previously false positive were mainly excluded and reclassified as true negatives in both sample sets. Table 3 shows the performance of the autoantibody panel in both development and confirmatory sample sets with and without the application of the secondary curve parameter cut-off.

*Table 3: Comparison of the performance characteristics of an autoantibody test with application of single and double cut-off in two independent sample sets*

| Cut-off | Specificity (%) | Sensitivity (%) | PPV (%) | False calls per 100 tests run | |
|---|---|---|---|---|---|
| | | | | Normal | Cancer |
| **Development set:** Standard antibody / antigen binding cut-off | 90.1 | 29.7 | 5.8 | 97.0 | 14.1 |
| Double cut-off method | 98.1 | 22.0 | 19.1 | 18.6 | 15.6 |
| **Confirmation set:** Standard antibody / antigen binding cut-off | 90.6 | 41.5 | 8.3 | 92.1 | 11.7 |
| Double cut-off method | 97.0 | 27.9 | 16.0 | 29.4 | 14.4 |

Although the confirmatory sample set provided better performance data than the training set using the standard antibody / antigen binding cut-off approach, the improvement in specificity and hence PPV afforded by the application of the secondary curve parameter cut-off was maintained. This demonstrates that the approach is reproducible across two completely independent case controlled sample sets.

*Example 3 - Improvement in panel performance by incorporation of additional antigens*

Sample Set

In order to investigate the improvement in panel performance that could be achieved by incorporation of additional antigens, a sub set of the training set described in Example 1 was used. This set comprised 166 serum or plasma samples from patients with primary lung cancer taken at diagnosis and prior to treatment (cancers). These were matched (for age, gender and smoking history) to 147 serum or plasma samples from individuals with no evidence of malignancy (normals).

Additional antigens

An additional four antigens: alpha-enolase, cytokeratin 8 (CK8), cytokeratin 20 (CK20) and L-myc2 were investigated for their ability to improve the performance of the autoantibody panel when the double cut-off method was applied. Antibody / antigen binding was measured by ELISA as described in Example 1. Secondary curve parameters were also as described in Example 1. Both antibody / antigen binding and secondary curve parameter cut-offs were optimised for maximum assay performance for the new antigens (Table 4).

*Table 4: Incorporation of additional antigens. Optimal secondary curve parameters are given with cut-offs*

| Antigen | Antibody / antigen binding cut-off (RU) | Secondary curve parameter | Log / linear regression | Secondary curve parameter cut-off |
|---|---|---|---|---|
| Alpha enolase | 7.5 | Slope | Log | <0.24 |
| Cytokeratin 8 | 8.0 | Slope | Log | > 0.15 |
| Cytokeratin 20 | 7.0 | Slopemax | Linear | > 0.005 |
| Lmyc2 | 7.0 | Slopemax | Log | > 0.40 |

Results

The distribution of samples according to their antibody / antigen binding versus the optimal secondary curve parameter for the four new antigens are shown in Figure 6A-D. Individual samples are coded according to the result that was obtained for the seven antigen panel. When these four antigens had previously been investigated in an assay format that involved only application of the antibody / antigen binding cut-off they had resulted in slightly increased sensitivity but the specificity of the increased panel was 82.3% and therefore too low to be clinically useful (Table 5).

*Table 5: Effect of additional antigens on autoantibody panel performance*

| Cut-off | Specificity (%) | Sensivity (%) | PPV (%) | False calls per 1000 tests run | |
|---|---|---|---|---|---|
| | | | | Normal | Cancer |
| **7 Antigen Panel:** Standard antibody / | 90.1 | 29.7 | 5.8 | 97.0 | 14.1 |
| antigen binding cut-off mathodparameter cut-off | 98.1 | 22.0 | 19.1 | 18.6 | 15.6 |
| **11 Antigen Panel:** Standard antibody / antigen binding cut-off | 82.3 | 52.4 | 5.7 | 173.5 | 9.5 |
| Double cut-off method | 96.6 | 40.4 | 19.5 | 33.3 | 11.9 |

However, when the secondary curve parameter cut-offs were applied and both cut-offs for these antigens set to maximise on the current seven antigen panel performance, the PPV was improved from 5.8% (1 in every 17 positive tests is a cancer) to 19.1% (1 in every 5 positive tests is a cancer). With the addition of the four extra antigens the sensitivity of the panel was also improved from 29.7% to 40.4% (Table 4) with minimal loss in specificity. This provided proof of principle that addition of new antigens to a panel where the double cut-off method was applied can improve test sensitivity with little detrimental effect on specificity which had not been possible with the use of a single antibody / antigen binding cut-off.

*Example 4 - Improvement in panel performance by incorporation of antigenic variants and mutants*

When autoantibodies specific for several different variant forms of the same antigen (e.g. p53) are measured in a group of cancer patients, differences are observed between individual patients in the specificity of the autoantibodies they produce (Table 6).

| Sample number | p53 BirA | p53-EP C-BirA | p53 C-BirA | p53-EP BirA | p53-292 BirA | p53-95 BirA | p53-7 NLIC | p53-7 CLIC | p53 NLIC | Any variant |
|---|---|---|---|---|---|---|---|---|---|---|
| 3 | | Pos | | | | n.d. | | | | Pos |
| 49 | | | | | | | Pos | Pos | Pos | Pos |
| 87 | Pos | Pos | Pos | Pos | | Pos | | | Pos | Pos |
| 105 | | | Pos | | | | | | | Pos |
| 113 | n.d. | Pos | Pos | Pos | Pos | Pos | n.d. | n.d. | n.d. | Pos |
| 126 | Pos | Pos | Pos | Pos | Pos | Pos | Pos | Pos | Pos | Pos |
| 127 | Pos | Pos | Pos | Pos | Pos | Pos | Pos | Pos | Pos | Pos |
| 145 | Pos | | | | | | | | | Pos |
| 173 | | | | | | Pos | | | | Pos |
| 185 | | | | Pos | | | | | | Pos |
| 247 | Pos | | | | | | | | | Pos |
| 265 | | | | | | Pos | | | | Pos |
| 339 | Pos | Pos | Pos | | | Pos | | | Pos | Pos |
| 363 | | | Pos | | | | Pos | Pos | | Pos |
| 372 | | | | | | Pos | | | | Pos |
| 445 | | | | Pos | | | | Pos | | Pos |
| 487 | | Pos | Pos | | | | | | | Pos |
| 738 | Pos | Pos | Pos | | Pos | Pos | Pos | Pos | Pos | Pos |
| 766 | | | | | | Pos | | | Pos | Pos |
| 835 | n.d. | | Pos | | | | Pos | Pos | Pos | Pos |
| 984 | | Pos | | Pos | | | | | | Pos |
| 1437 | Pos | | | | | | | | | Pos |
| 1443 | Pos | | | | | | | | | Pos |
| positivity | 9/96 | 9/98 | 10/98 | 7/96 | 4/98 | 10/96 | 6/99 | 7/98 | 8/99 | 23/100 |
| sensitivity | 9.4% | 9.2% | 10.2% | 7.3% | 4.1% | 10.4% | 6.1% | 7.1% | 8.1% | 23.0% |

*Table 6: Autoantibody positivity of several different variants of p53 in 100 lung cancer patients. Pos = sample was positive for autoantibody binding. n.d. = no data. Only samples positive for one or more autoantibody are shown*

This would seem to suggest that not all patients produce the same altered forms of tumour associated antigens during the course of their disease and it may be possible to use different variant forms or mutations in order to improve the performance of autoantibody assays. However normal individuals also show evidence of autoantibody production against different variant forms of tumour associated antigens (Table 7) presumably due to the fact that they have mounted a successful immune response against a malignant transformation process that has eradicated the cancer before it has had a chance to establish itself.

| Sample number | p53 BirA | p53-EP C-BirA | p53 C-BirA | p53-EP BirA | p53-292 BirA | p53-95 BirA | p53-7 NLIC | p53-7 CLIC | p53 NLIC | Any variant |
|---|---|---|---|---|---|---|---|---|---|---|
| 1711 | n.d. | Pos | Pos | Pos | n.d. | n.d. | Pos | Pos | Pos | Pos |
| 4951 | | | | Pos | | | | | | Pos |
| 4824 | | Pos | | | | | | | | Pos |
| 3988 | | | | | | | Pos | Pos | | Pos |
| 2575 | | | | Pos | | | | | | Pos |
| 3028 | | | | | | | Pos | | Pos | Pos |
| 4584 | | | Pos | | Pos | Pos | | | | Pos |
| 3064 | Pos | | | | | | | | | Pos |
| 2215 | | | | | | Pos | | | | Pos |
| 3227 | Pos | Pos | Pos | | | Pos | | Pos | | Pos |
| 2524 | | | | | | | | | Pos | Pos |
| 4440 | | | | Pos | | | | | | Pos |
| 3599 | Pos | | | | Pos | | | | | Pos |
| positivity | 3/98 | 3/98 | 3/99 | 3/99 | 3/97 | 3/98 | 3/100 | 3/100 | 3/100 | 13/100 |
| specificity | 96.9% | 96.9% | 97.0% | 97.0% | 96.9% | 96.9% | 97.0% | 97.0% | 97.0% | 87.0% |

*Table 7: Autoantibody positivity of several different variants of p53 in 100 individuals with no evidence of malignancy. Pos = sample was positive for autoantibody binding. n.d. = no data. Only samples positive for one or more autoantibody are shown*

The background level of autoantibody production in apparently normal individuals makes the use of these biomarkers for early detection of cancer challenging unless a method can be found for distinguishing those whose malignant assault has been eradicated by the immune response (false positives) from those who have active tumours (true positives). The following example describes a study performed in order to investigate the application of the double cut-off method to autoantibody panels including variant or mutated forms of tumour associated antigens.

Samples

The sample set used for this study was a subset of the training set and comprised 151 serum or plasma samples from patients with primary lung cancer taken at diagnosis and prior to treatment (cancers). These were matched (for age, gender and smoking history) to 104 serum or plasma samples from individuals with no evidence of malignancy (normals).

Antigenic variants and mutations

A list of antigenic variants and mutations used is given in Table 8.

*Table 8: Antigenic variants and mutations*

| Construct Name | Variant type | Amino acids |
|---|---|---|
| **p53** | | |
| p53-BirA | Full length tumour suppressor | 1-393 |
| p53 C-BirA | Full length tumour suppressor | 1-393 |
| p53-Cterm BirA | C terminal fragment, DNA binding and protein binding regions | 221-393 |
| p53-EP C-BirA | N terminal epitope, transcription activation region | |
| p53-95 BirA | N terminal fragment, transcription activation & ligand binding region | 1-95 |
| p53-292 BirA | N terminal fragment, transcription activation, ligand binding region and DNA binding domain | 1-292 |

EP 3 158 336 B1

(continued)

| EGFR | | |
|---|---|---|
| EGFR1 KD C-BirA | Isoform 1 Kinase domain truncation | 645-998 |
| EGFR1 EP C-BirA | Isoform 1 Epitope fragment truncation | 286-462 |
| EGFR vIII BirA | Partial extracellular domain bearing an N-terminal sequence splice variant | LEEKKG-274-605 |
| EGFR L858R C-BirA | Lung cancer associated mutation L858R of isoform 1 kinase domain | 645-998 |
| EGFR2 C-BirA | Isoform 2 like truncation | 2-379 |
| **Kras** | | |
| Kras G12C/Q61H BirA | Full length isoform 2b; double mutant | 1-188 |
| Kras G13C/Q61H BirA | Full length isoform 2b; double mutant | 1-188 |
| Kras Q61H BirA | Full length isoform 2b; single lung cancer associated mutation | 1-188 |

Antibody / antigen binding was measured by ELISA as described in Example 1. Secondary curve parameters and determination of the directionality required for a positive reading were also as described in Example 1. Net Reclassification Index (NRI) was used to choose the best antigens for addition to the final panel from all tested including all antigens described in Examples 1-3 and all variants and mutations.

Results

The distribution of samples according to their antibody / antigen binding versus the optimal secondary curve parameter for the variants and mutations selected for inclusion in the final panel are shown in Figure 7A-G Individual samples are coded according to the result that was obtained for the seven antigen panel. The NRI was used to rank the performance of antigens and chose the best antigens for the panel (Table 9).

*Table 9: Results of Net Reclassification Index. Sensitivity and specificity are expressed as %. Conc = antigen concentration at which the standard antibody / antigen binding result was calculated*

| Antigen rank | Antigen | Conc (nM) | NRI | Antigen Performance | | Panel Performance | |
|---|---|---|---|---|---|---|---|
| | | | | Specificity | Sensivity | Specificity | Sensivity |
| 1 | NY-ESO-1 | 160 | 0.113 | 100.0 | 11.3 | 100.0 | 11.3 |
| 2 | Alpha enolase | 50 | 0.099 | 100.0 | 9.9 | 100.0 | 21.2 |
| 3 | Cytokeratin 20 | 160 | 0.073 | 100.0 | 9.3 | 100.0 | 28.5 |
| 4 | p53-C term | 50 | 0.046 | 100.0 | 6.6 | 100.0 | 33.1 |
| 5 | Kras G12C/Q61H | 50 | 0.026 | 100.0 | 5.3 | 100.0 | 35.8 |
| 6 | MAGE A4 | 160 | 0.020 | 100.0 | 4.6 | 100.0 | 37.7 |
| 7 | SOX2 | 160 | 0.020 | 100.0 | 2.6 | 100.0 | 39.7 |
| 8 | Cytokeratin 8 | 160 | 0.020 | 100.0 | 2.6 | 100.0 | 41.7 |
| 9 | Lmyc2 | 160 | 0.013 | 100.0 | 2.6 | 100.0 | 43.0 |
| 10 | p53 | 160 | 0.010 | 99.0 | 8.6 | 99.0 | 45.0 |
| 11 | p53 C-BirA | 160 | 0.007 | 100.0 | 4.6 | 99.0 | 45.7 |
| 12 | Kras Q61H | 50 | 0.007 | 100.0 | 4.0 | 99.0 | 46.4 |
| 13 | EGFR1-EP | 160 | 0.007 | 100.0 | 3.3 | 99.0 | 47.0 |
| 14 | Kras G13C/Q61H | 50 | 0.007 | 100.0 | 2.6 | 99.0 | 47.7 |

25

(continued)

| Antigen rank | Antigen | Conc (nM) | NRI | Antigen Performance | | Panel Performance | |
|---|---|---|---|---|---|---|---|
| | | | | Specificity | Sensivity | Specificity | Sensivity |
| 15 | CAGE | 160 | 0.007 | 100.0 | 2.6 | 99.0 | 48.3 |
| 16 | GBU4-5 | 50 | 0.007 | 100.0 | 0.7 | 99.0 | 49.0 |
| 17 | p53-95 | 160 | 0.007 | 99.0 | 6.6 | 99.0 | 49.7 |
| 18 | Cytokeratin 20 | 50 | 0.007 | 100.0 | 5.3 | 99.0 | 50.3 |

The final panel consisted of 18 antigens and the performance of this panel is shown in Table 10.

*Table 10: Effect of additional antigens, variants and mutations on autoantibody panel performance*

| Cut-off | Specificity (%) | Sensitivity (%) | PPV (%) | False calls per 1000 tests run | |
|---|---|---|---|---|---|
| | | | | Normal | Cancer |
| Standard antibody / antigen binding cut-off | 90.4 | 31.8 | 6.3 | 94.1 | 13.6 |
| Double cut-off method | 99.0 | 50.3 | 50.7 | 9.8 | 9.9 |

No further improvement in performance could be obtained by addition of any of the other antigens against which autoantibodies were measured in this Example.

*Example 5 - Application of the double cut-off method in hepatocellular carcinoma*

Hepatocellular carcinoma (HCC) is a malignant disease of the liver. 80-90% of cases occur in individuals with a pre-disposing liver disease such as Hepatitis B or C infection or alcoholic cirrhosis. Early detection while the malignant lesions are still small enough to be removed by surgery or when liver transplant can still be curative is the key to improving mortality. HCC has a 40-70% 5 year survival rate in patients diagnosed with early stage (stage 0 and A) disease compared with 5% in patients diagnosed with advanced stage disease. Current surveillance methods involve abdominal ultrasound and measurement of serum alphafeto protein (AFP) levels. The accuracy of abdominal ultrasound is relatively low (especially for small lesions) and dependent on operator skill. Patient compliance is also poor therefore there is an urgent need for a simple blood test that can add to the performance of AFP for early detection of HCC in high risk groups.

Sample set

In order to investigate the improvement in assay performance afforded by application of the double cut-off method to a panel of AAb for detection of HCC, the sample set comprised 193 serum or plasma samples from patients with primary HCC taken at diagnosis and prior to cancer treatment. These were matched as closely as possible (for age and gender) to 190 serum or plasma samples from individuals with no evidence of malignancy (normals). A second control group consisted of 176 samples from patients with benign liver disease such as Hepatitis B, C or cirrhotic liver.

Secondary curve parameters and cut-off determination

Antibody / antigen binding was measured by ELISA as described in Example 1. Secondary curve parameters and directionality required for a positive reading were also as described in Example 1. Both standard antibody / antigen binding and secondary curve parameter cut-offs were determined as described in Example 1. Autoantibodies measured and secondary curve parameters applied in the assay for the early detection of HCC are shown in Table 11. Both antibody / antigen binding and secondary curve parameter cut-offs were optimised for maximum assay performance.

*Table 11: Development of secondary curve parameters for the assay for early detection of HCC.*

| Antigen | Antigen concentration (nM) | Antibody / antigen binding single parameter cut-off (OD) | Antibody / antigen binding two parameter cut-off (OD) | Secondary curve parameter | Log / linear regression | Secondary curve parameter cut-off |
|---|---|---|---|---|---|---|
| p53 | 50 | 1.29 | 1.29 | SlopeMax | Linear | 0.03 |
| p53 | 160 | 1.5 | 1.5 | SlopeMax | Linear | 0.03 |
| SOX2 | 50 | 0.5 | 0.5 | SlopeMax | Linear | 0.02 |
| SOX2 | 160 | 0.5 | 0.5 | SlopeMax | Linear | 0.02 |
| CAGE | 50 | 0.35 | 0.35 | SlopeMax | Linear | 0.012 |
| CAGE | 160 | 0.7 | 0.7 | SlopeMax | Linear | 0.01 |
| NY-ESO-1 | 50 | 0.3 | 0.3 | SlopeMax | Linear | 0.05 |
| NY-ESO-1 | 160 | 0.75 | 0.75 | SlopeMax | Linear | 0.005 |
| MAGE A4 | 50 | 0.7 | 0.7 | SlopeMax | Linear | 0.025 |
| MAGE-A4 | 160 | 0.7 | 0.7 | SlopeMax | Linear | 0.025 |
| Cytokeratin 8 | 50 | 0.5 | 0.45 | Slope | Linear | 0.004 |
| Cytokertin 8 | 160 | 0.75 | 0.75 | SlopeMax | Linear | 0.01 |
| AFP | 50 | 1.25 | 1.25 | SlopeMax | Linear | 0.04 |
| AFP | 160 | 1.1 | 1.1 | SlopeMax | Linear | 0.03 |
| p62 | 50 | 1.0 | 0.9 | Intercept | Linear | 0.25 |
| p62 | 160 | 1.1 | 0.85 | Intercept | Linear | 0.25 |
| SSX1 | 50 | 0.9 | 0.9 | SlopeMax | Linear | 0.025 |
| SSX1 | 160 | 1 | 1 | SlopeMax | Linear | 0.025 |
| HDGF | 50 | 0.56 | 0.5 | Intercept | Linear | 0.25 |
| HDGF | 160 | 0.5 | 0.35 | Intercept | Linear | 0.25 |
| VTN10 | 50 | 0.68 | 0.25 | Intercept | Linear | 0.1 |
| VTN10 | 160 | 0.5 | 0.25 | Intercept | Linear | 0.1 |
| IMP1 | 50 | 1.6 | 1.2 | Intercept | Linear | 0.8 |
| IMP1 | 160 | 1.6 | 0.8 | Intercept | Linear | 0.8 |

Results

The distribution of samples according to their antibody / antigen binding versus the optimal secondary curve parameter for the initial HCC panel antigens are shown in Figure 8A-F and the distribution for the six additional antigens are shown in Figure 9A-F. The horizontal cut-off represents the standard antibody / antigen binding cut-off and the vertical cut-off represents the secondary curve parameter cut-off. There was enrichment of true positives within the zone of positivity whereas samples that were previously false positive were mainly excluded and reclassified as true negatives in both sample sets. Table 12 shows the performance of the autoantibody with and without the application of the secondary curve parameter cut-off as well as the improvement that was afforded by incorporation of an additional six antigens for measurement of autoantibodies. It can be seen that application of the double cut-off was able to increase specificity in the benign cohort to 100%, with only a small reduction in sensitivity, and addition of a further 6 antigens was able to give an expanded panel with both improved specificity and sensitivity compared to the initial 6 antigen panel. This resulted in an assay that would give only six false positive calls per thousand patients tested.

*Table 12: Effect of applying secondary parameter cut-offs and inclusion of additional antigens on autoantibody panel performance for the detection of HCC.*

| Cut-off | Specificity* (%) | Sensivity (%) | PPV (%) | False calls per 1000 tests run | |
|---|---|---|---|---|---|
| | | | | Normal | Cancer |
| **6 Antigen Panel:** Standard antibody / antigen binding cut-off | 97.7% | 19.7% | 23.2% | 22 | 27 |
| Double cut-off method | 100.0% | 17.1% | 100.0% | 0 | 28 |
| **12 Antigen Panel:** Standard antibody / antigen binding cut-off | 96.6% | 21.2% | 18.0% | 33 | 27 |
| Double cut-off method | 99.4% | 20.2% | 54.2% | 6 | 27 |
| * Specificity is measured using the benign patient cohort because this is the high risk group that would undergo surveillance using a test such as this. | | | | | |

**Claims**

1. A method of detecting an antibody in a test sample comprising a bodily fluid from a mammalian subject, the method comprising the steps of:

   (a) contacting said test sample with a plurality of different amounts of an antigen specific for said antibody;
   (b) detecting the amount of specific binding between said antibody and said antigen;
   (c) plotting or calculating a curve of the amount of said specific binding versus the amount of antigen for each amount of antigen used in step (a);
   (d) calculating a secondary curve parameter from the curve plotted or calculated in step (c); and
   (e) determining the presence or absence of said antibody based upon a combination of:

      (i) the amount of specific binding between said antibody and said antigen determined in step (b); and
      (ii) said secondary curve parameter determined in step (d).

2. A method of detecting two or more antibodies in a test sample comprising a bodily fluid from a mammalian subject, the method comprising the steps of:

   (a) contacting said test sample with a panel comprising two or more sets of antigens, wherein each of said antigens in said panel is specific for at least one of said two or more antibodies;
   (b) detecting complexes of said antigens bound to antibodies present in said test sample;
   (c) for each antigen plotting or calculating a separate curve of the amount of said specific binding versus the amount of antigen for each amount of antigen used in step (a);
   (d) calculating a secondary curve parameter for each curve plotted or calculated in step (c); and
   (e) determining the presence or absence of said two or more antibodies based upon a combination of:

      (i) the amount of specific binding between said antibody and said antigen determined in step (b); and
      (ii) said secondary curve parameter determined in step (d),

   for each of the curves plotted or calculated in step (c).

3. The method of claim 2, wherein:

   (a) the two or more antigens of the panel are distinct antigens, and wherein the panel optionally comprises two or more antigen variants of one or more of the distinct antigens; or
   (b) the two or more antigens of the panel are antigen variants.

4. The method of any one of claims 1 to 3, wherein the secondary curve parameter is selected from the group consisting of Slope, Intercept, Area Under the Curve (AUC), SlopeMax or dissociation constant (Kd), and wherein the secondary curve parameter is optionally calculated from either a linear or logarithmic regression curve

5. The method of any one of claims 1 to 3, wherein the secondary curve parameter is Maximum Asymptote, Minimum Asymptote, Hill Slope (or Slope Factor) or Inflection Point of a logistic curve fitted to each curve plotted or calculated in step (c).

6. The method according to any one of claims 1 to 5 wherein the antigen is a naturally occurring protein or polypeptide, a recombinant protein or polynucleotide, a synthetic protein or polypeptide, synthetic peptide, peptide mimetic, polysaccharide or nucleic acid.

7. The method of any one of claims 1-6 wherein the antibody is a biological marker of a disease state or a disease susceptibility.

8. The method of any one of claims 1 to 7, wherein the antibody is an autoantibody.

9. The method of claim 8 wherein the autoantibody is specific for a tumour marker protein, and wherein the tumour marker protein is optionally selected from the group consisting of EGFR, MUC1, the signal transduction/cell cycle regulatory proteins Myc, c-myc, L-myc2, p53, ras (or Ras), BRCA1, BRCA2, APC, CA125, PSA, CEA, CA19.9, NY-ESO-1, PSMA (prostate specific membrane antigen), PSCA (prostate stem cell antigen), EpCam (epithelial cellular adhesion molecule), HER2 (also known as c-erbB2), CAGE, CAGE1, CAGE2, cytokeratins, recoverin, kallikreins, annexins, $\alpha$-fetoprotein (AFP), GRP78, mammoglobin, raf, beta-human chorionic gonadotropin b-HCG, 4-5 antigen, SOX2, GBU4-5, MAGE, HuD, HE4, SALL4, alpha enolase, p62, RalA, Cyclin B1, Calreticulin R, IMP1(, Vitronectins (e.g. VTN10), SSX1, TMP21, NPC1L1C, TMOD1, CAMK1, RGS1, PACSIN1, RCV1, MAPKAPK3, CyclinE2, cytokeratin 8 (CK8), 18 (CK18), 19 (CK19), 20 (CK20), SCC, proGRP, serum Amyloid A, alpha-1-anti-trypsin, apolipoproteinCIII, thymo$\beta$in $\beta$4, HDJ1 and HDGF.

10. The method of claim 8 wherein the autoantibody is:

(a) characteristic of or associated with an autoimmune disease, such as rheumatoid arthritis, systemic lupus erythematous (SLE), primary biliary cirrhosis (PBC), autoimmune thyroiditis, Hashimoto's thyroiditis, autoimmune gastritis, pernicious anaemia, autoimmune adrenalitis, Addison's disease, autoimmune hypoparathyriodism, autoimmune diabetes, myasthenia gravis, paraneoplastic neurological disorders such as Lambert-Eaton Myasthenic Syndrome, inflammatory bowel disease, sarcoidosis and autoimmune hepatitis; or
(b) characteristic of or associated with kidney or hepatic disease leading to insufficiency or failure of either organ.

11. The method of any one of claims 1-8 wherein the antibody is directed towards an epitope present on tissue transplanted into the mammalian subject.

12. Use of a kit for performing the method of any one of the preceding claims, wherein said kit comprises one or more antigens and a reagent capable of detecting complexes of said antigens bound to antibodies present in a test sample comprising a bodily fluid from a mammalian subject, and wherein the kit optionally comprises:

(a) two or more antigen variants; and/or
(b) means for contacting said one or more antigens with a sample of bodily fluid.

13. Use of the method of claim 9 in:

(a) the diagnosis, prognosis or monitoring of cancer;
(b) the detection of early neoplastic or early carcinogenic change in asymptomatic patients;
(c) the detection of recurrent disease in a patient previously diagnosed as carrying tumour cells, which patient has undergone treatment to reduce the number of said tumour cells;
(d) the identification of those individuals which are at increased risk of developing cancer, such as lung cancer, breast cancer, bladder cancer, prostate cancer, colorectal cancer, ovarian cancer or hepatocellular carcinoma, in a population of asymptomatic individuals;
(e) determination of the tumour marker profile of an individual suffering from cancer, wherein said tumour marker profile is optionally determined sequentially in said individual as an indication of the course of disease;
(f) predicting the response of an individual with cancer to anti-cancer treatment, such as surgical resection, hormone therapy, chemotherapy, radiotherapy, anti-growth factor therapy, immune therapy or vaccination;

(g) the diagnosis, prognosis or monitoring of cancer;

(h) screening a population of asymptomatic human subjects to identify those subjects who are at increased risk of developing cancer, wherein the samples to be tested using the method are samples of bodily fluid taken from the subjects, and wherein subjects having an elevated level of autoantibodies and an altered secondary curve parameter, as compared to normal control individuals, are identified as being at risk of developing cancer;

(i) detecting early neoplastic or early carcinogenic change in an asymptomatic human subject, wherein the sample to be tested using the method is a sample of bodily fluid taken from the subject, and wherein the presence of an elevated level of autoantibodies and an altered secondary curve parameter, as compared to normal control individuals, is taken as an indication of early neoplastic or early carcinogenic change in the subject;

(j) screening a population of asymptomatic human subjects to identify those subjects who have developed a cancer, wherein the samples to be tested using the method are samples of bodily fluid taken from the subjects, and wherein subjects having an elevated level of autoantibodies and an altered secondary curve parameter, as compared to normal control individuals, are diagnosed as having a cancer;

(k) testing a population of symptomatic human subjects to identify those subjects who have developed a cancer, wherein the samples to be tested using the method are samples of bodily fluid taken from the subjects, and wherein subjects having an elevated level of autoantibodies and an altered secondary curve parameter, as compared to normal control individuals, are diagnosed as having a cancer;

(l) monitoring the progress of cancer or other neoplastic disease in a patient, wherein the sample to be tested using the method is a sample of bodily fluid taken from a human patient, and wherein the presence of an elevated level of autoantibodies and an altered secondary curve parameter, as compared to a normal control, is taken as an indication of the presence of cancer in the patient;

(m) the detection of recurrent disease in a human patient previously diagnosed as having cancer, which patient has undergone anti-cancer treatment to reduce the amount of cancer present, wherein the sample to be tested using the method is a sample of bodily fluid taken from the patient, and wherein the presence of an increased level of autoantibodies in the patient and an altered secondary curve parameter, as compared to a normal control, is taken as an indication that disease has recurred; or

(n) assessment of prognosis from cancer, wherein the sample to be tested using the method is a sample of bodily fluid taken from a human patient, and wherein the presence of an elevated level of autoantibodies and an altered secondary curve parameter, as compared to a normal control, is taken as an indication of the prognosis of the patient from their cancer.

14. Use of the method of claim 7 or claim 8 in the detection of cancer, preferably lung, breast, bladder, colorectal, prostate ovarian cancer or hepatocellular carcinoma.

15. Use of the method of claim 7 or claim 8 in monitoring the response of a human cancer patient to anti-cancer treatment, wherein the sample to be tested using the method is a sample of bodily fluid taken from the patient, and wherein a change in the level of autoantibodies and a change in the secondary curve parameter after treatment is taken as an indication of whether or not the patient has responded to the treatment, wherein the treatment is optionally surgical resection, vaccination, anti-growth factor or signal transduction therapy, radiotherapy, endocrine therapy, human antibody therapy or chemotherapy and a change in the level of autoantibodies and a change in the secondary curve parameter after treatment is taken as an indication that the patient has responded positively to the treatment.

## Patentansprüche

1. Verfahren zum Erfassen eines Antikörpers in einer Versuchsprobe, die eine Körperflüssigkeit eines säugetierartigen Wesens umfasst, wobei das Verfahren die Schritte umfasst:

(a) Inkontaktbringen der Versuchsprobe mit einer Mehrzahl unterschiedlicher Mengen eines für den Antikörper spezifischen Antigens;

(b) Erfassen der Menge an spezifischer Bindung zwischen dem Antikörper und dem Antigen;

(c) Auftragen oder Berechnen einer Kurve der Menge der spezifischen Bindung in Abhängigkeit von der Menge an Antigen für jede in Schritt (a) verwendete Menge an Antigen;

(d) Berechnen eines sekundären Kurvenparameters aus der in Schritt (c) aufgetragenen oder berechneten Kurve; und

(e) Bestimmen des Vorhandenseins oder Nichtvorhandenseins des Antikörpers basierend auf einer Kombination aus

(i) der in Schritt (b) erfassten Menge an spezifischer Bindung zwischen dem Antikörper und dem Antigen; und
(ii) dem in Schritt (d) )bestimmten sekundären Kurvenparameter.

2. Verfahren zum Erfassen von zwei oder mehr Antikörpern in einer Versuchsprobe, die eine Körperflüssigkeit eines säugetierartigen Wesens umfasst, wobei das Verfahren die Schritte umfasst:

(a) Inkontaktbringen der Versuchsprobe mit einem Panel , das zwei oder mehr sätze von Antigenen umfasst, wobei jedes der Antigene in dem Panel für wenigstens einen der zwei oder mehr Antikörper spezifisch ist;
(b) Erfassen von Komplexen der an in der Versuchsprobe enthaltene Antikörper gebundenen Antigene;
(c) für jedes Antigen, Auftragen oder Berechnen einer eigenen Kurve der Menge der spezifischen Bindung in Abhängigkeit von der Menge an Antigen für jede in Schritt (a) verwendete Menge an Antigen;
(d) Berechnen eines sekundären Kurvenparameters für jede in Schritt (c) aufgetragene oder berechnete Kurve; und
(e) Bestimmen des Vorhandenseins oder Nichtvorhandenseins der zwei oder mehr Antikörpers basierend auf einer Kombination aus

(i) der in Schritt (b) erfassten Menge an spezifischer Bindung zwischen dem Antikörper und dem Antigen; und
(ii) dem in Schritt (d) )bestimmten sekundären Kurvenparameter

für jede der in Schritt (c) aufgetragenen oder berechneten Kurven.

3. Verfahren nach Anspruch 2, bei dem

(a) die zwei oder mehr Antigene des Panels unterschiedliche Antigene sind und das Panel optional zwei oder mehr Antigenvarianten eines oder mehrerer der unterschiedlichen Antigene umfasst; oder

die zwei oder mehr Antigene des Panels Antigenvarianten sind.

4. Verfahren nach einem der Ansprüche 1 bis 3, bei dem der sekundäre Kurvenparameter ausgewählt ist aus der Gruppe bestehend aus Steigung, Achsenabschnitt, Fläche unter Kurve (AUC), MaxSteigung oder Dissoziationskonstante (Kd), und wobei der sekundäre Kurvenparameter optional aus einer linearen oder logarithmischen Regressionskurve berechnet wird.

5. Verfahren nach einem der Ansprüche 1 bis 3, bei dem der sekundäre Kurvenparameter Maximumasymptote, Minimumasymptote, Hill-Steigung (oder Steigungsfaktor) oder Wendepunkt einer an jede in Schritt (c) aufgetragene oder berechnete Kurve angepassten logistischen Kurve ist.

6. Verfahren nach einem der Ansprüche 1 bis 5, bei dem das Antigen ein natürlich vorkommendes Protein oder Polypeptid, ein rekombinantes Protein oder Polynukleotid, ein synthetisches Protein oder Polypeptid, ein synthetisches Peptid, ein Peptidmimetikum, ein Polysaccharid oder eine Nukleinsäure ist.

7. Verfahren nach einem der Ansprüche 1 bis 6, bei dem der Antikörper ein biologischer Marker einer Erkrankung oder einer Erkrankungsneigung ist.

8. Verfahren nach einem der Ansprüche 1 bis 7, bei dem der Antikörper ein Autoantikörper ist.

9. Verfahren nach Anspruch 8, bei dem der Autoantikörper für ein Tumormarkerprotein spezifisch ist, und wobei das Tumormarkerprotein optional ausgewählt ist aus der Gruppe bestehend aus EGFR, MUC1, den Signaltransduktions-/zellzyklusregulatorischen Proteinen Myc, c-myc, L-myc2, p53, ras (oder Ras), BRCA1, BRCA2, APC, CA125, PSA, CEA, CA19.9, NY-ESO-1, PSMA (Prostataspezifisches Membranantigen), PSCA (Prostatastammzellantigen), EpCam (Epithelzelladhäsionsmolekül), HER2 (auch bekannt als c-erbB2), CAGE, CAGE1, CAGE2, Cytokeratine, Recoverin, Kallikreine, Annexine, $\alpha$-Fetoprotein (AFP), GRP78, Mammoglobin, raf, beta-Humanchoriongonadotropin b-HCG, 4-5-Antigen, SOX2, GBU4-5, MAGE, HuD, HE4, SALL4, alpha-Enolase, p62, RalA, Cyclin Bl, Calreticulin R, IMP1(, Vitronektine (z.B. VTN10), SSX1, TMP21, NPC1L1C, TMOD1, CAMK1, RGS1, PACS1N1, RCV1, MAPKAPK3, CyclinE2, Cytokeratin 8 (CK8), 18 (CK18), 19 (CK19), 20 (CK20), SCC, proGRP, Serum-Amyloid A, alpha-1-Antitrypsin, Apolipoprotein CIII, Thymoßin $\beta$4, HDJ1 und HDGF.

10. Verfahren nach Anspruch 8, bei dem der Autoantikörper

(a) charakteristisch für oder assoziiert ist mit einer Autoimmunerkrankung wie etwa rheumatoider Arthritis, systemischem Lupus erythematodes (SLE), primär biliärer Zirrhose (PBC), autoimmuner Thyroiditis, Hashimoto-Thyroiditis, Autoimmungastritis, perniziöser Anämie, Autoimmunadrenalitis, Morbus Addison, Autoimmun-Hypoparathyriodismus, Autoimmun-Diabetes, Myasthenia gravis, paraneoplastischen neurologischen Störungen wie Lambert-Eaton-Syndrom, Inflammatory Bowel Disease, Sarkoidose und Autoimmun-Hepatitis; oder
(b) charakteristisch für oder assoziiert ist mit einer Nieren- oder Lebererkrankung ist, die zu Insuffizienz oder Versagen des Organs führt.

11. Verfahren nach einem der Ansprüche 1 bis 8, bei dem der Antikörper gegen ein Epitop gerichtet ist, das auf in das säugetierartige Wesen transplantiertem Gewebe vorhanden ist.

12. Verwendung eines Kits zur Durchführung des Verfahrens nach einem der vorhergehenden Ansprüche, wobei der Kit ein oder mehrere Antigene und ein Reagens umfasst, das geeignet ist zum Erfassen von Komplexen der an in einer Versuchsprobe, die eine Körperflüssigkeit eines säugetierartigen Wesens umfasst, vorhandene Antikörper gebundenen Antigene, und wobei der Kit optional umfasst:

(a) zwei oder mehr Antigenvarianten;
(b) Mittel zum Inkontaktbringen der ein oder mehreren Antigene mit einer Probe einer Körperflüssigkeit.

13. Verwendung des Verfahrens nach Anspruch 9 zur

(a) Diagnose, Prognose oder Überwachung von Krebs;
(b) Erfassung früher neoplastischer oder früher karzinogener Veränderungen in symptomfreien Patienten;
(c) Erfassung eines Erkrankungsrückfalls bei einem zuvor als Träger von Tumorzellen erkannten Patienten, wobei der Patient sich einer Behandlung zur Verringerung der Zahl der Tumorzellen unterzogen hat;
(d) Identifizierung, in einer Population von symptomfreien Individuen, von Individuen, die ein erhöhtes Risiko zur Ausbildung von Krebs, wie etwa Lungenkrebs, Brustkrebs, Blasenkrebs, Prostatakrebs, Dickdarmkrebs, Eierstockkrebs oder Leberzellkarzinom tragen;
(e) Bestimmung des Tumormarkerprofils eines an Krebs leidenden Individuums, wobei das Tumormarkerprofil bei dem Individuum optional sequentiell als Hinweis auf den ablauf der Erkrankung bestimmt wird;
(f) Vorhersage der Reaktion eines Individuums mit Krebs auf deine Antikrebsbehandlung wie etwa chirurgische Resektion, Hormontherapie, Chemotherapie, Strahlentherapie, Antiwachstumsfaktortherapie, Immuntherapie oder Impfung;
(g) Diagnose, Prognose oder Überwachung von Krebs;
(h) Screening einer Population von symptomfreien menschlichen Individuen, um diejenigen Individuen zu ermitteln, die ein erhöhtes Risiko zur Ausbildung von Krebs tragen, wobei die unter Verwendung des Verfahrens zu untersuchenden Proben Proben von den Individuen entnommener Körperflüssigkeit sind, und wobei Individuen, die einen erhöhten Pegel an Autoantikörpern und einen abweichenden Sekundärkurvenparameter im Vergleich zu normalen Kontrollindividuen aufweisen, als zur Ausbildung von Krebs gefährdet identifiziert werden;
(i) Erfassung früher neoplastischer oder früher karzinogener Veränderungen an einem symptomfreien menschlichen Individuum, wobei die unter Verwendung des Verfahrens zu untersuchende Probe eine Probe von dem Individuum entnommener Körperflüssigkeit ist, und wobei das Vorliegen eines erhöhten Pegels an Autoantikörpern und ein abweichender Sekundärkurvenparameter im Vergleich zu normalen Kontrollindividuen aufweisen, als zur Hinweis auf eine frühe neoplastische oder frühe karzinogene Veränderung des Individuums bewertet wird;
(j) Screening einer Population von symptomfreien menschlichen Individuen, um diejenigen Individuen zu identifizieren, die einen Krebs entwickelt haben, wobei die unter Verwendung des Verfahrens zu untersuchenden Proben Proben von den Individuen entnommener Körperflüssigkeit sind, und wobei Individuen, die einen erhöhten Pegel an Autoantikörpern und einen abweichenden Sekundärkurvenparameter im Vergleich zu normalen Kontrollindividuen aufweisen, als an Krebs erkrankt diagnostiziert werden;
(k) Testen einer Population von symptomatischen menschlichen Individuen, um diejenigen Individuen zu identifizieren, die einen Krebs entwickelt haben, wobei die unter Verwendung des Verfahrens zu untersuchenden Proben Proben von den Individuen entnommener Körperflüssigkeit sind, und wobei Individuen, die einen erhöhten Pegel an Autoantikörpern und einen abweichenden Sekundärkurvenparameter im Vergleich zu normalen Kontrollindividuen aufweisen, als an Krebs erkrankt diagnostiziert werden;
(l) Überwachen des Fortschreitens von Krebs oder einer anderen neoplastischen Erkrankung in einem Patienten, wobei die unter Verwendung des Verfahrens zu untersuchende Probe eine Probe von dem Patienten entnommener Körperflüssigkeit ist, und wobei das Vorliegen eines erhöhten Pegels an Autoantikörpern und ein abweichender Sekundärkurvenparameter im Vergleich zu einer normalen Kontrolle, als Hinweis auf das Vorhandensein von Krebs bei dem Patienten gewertet wird;

(m) Erfassung eines Erkrankungsrückfalls bei einem menschlichen Patienten, bei dem früher Krebs diagnostiziert worden ist, und der sich einer Antikrebsbehandlung unterzogen hat, um die Menge des vorhandenen Krebs zu verringern, wobei die unter Verwendung des Verfahrens zu untersuchende Probe eine Probe von dem Patienten entnommener Körperflüssigkeit ist, und wobei das Vorliegen eines erhöhten Pegels an Autoantikörpern und ein abweichender Sekundärkurvenparameter im Vergleich einer normalen Kontrolle als Hinweis auf einen Erkrankungsrückfall gewertet wird; oder

(n) Beurteilung einer Krebsprognose, wobei die unter Verwendung des Verfahrens zu untersuchende Probe eine Probe von einem menschlichen Patienten entnommener Körperflüssigkeit ist, und wobei das Vorliegen eines erhöhten Pegels an Autoantikörpern und ein abweichender Sekundärkurvenparameter im Vergleich zu einer normalen Kontrolle als zur Hinweis auf einen Erkrankungsrückfall gewertet wird;

14. Verwendung des Verfahrens nach Anspruch 7 oder 8 zur Erkennung von Krebs, vorzugsweise Lungen-, Brust-. Blasen-, Dickdarm-, Prostata-, Eierstockskrebs oder Leberzellkarzinom.

15. Verwendung des Verfahrens nach Anspruch 7 oder 8 zur Überwachung der Reaktion eines menschlichen Patienten auf eine Antikrebsbehandlung, wobei die unter Verwendung des Verfahrens zu untersuchende Probe eine Probe von dem Patienten entnommener Körperflüssigkeit ist, und wobei eine Veränderung des Pegels an Autoantikörpern und eine Veränderung des Sekundärkurvenparameters nach der Behandlung als ein Hinweis gewertet wird, ob der Patient auf die Behandlung angesprochen hat, wobei optional die Behandlung chirurgische Resektion, Impfung, Antiwachstumsfaktor- oder Signaltransduktionstherapie, Strahlentherapie, Endokrintherapie, Humanantikörpertherapie oder Chemotherapie ist und eine Veränderung des Pegels an Autoantikörpern und eine Veränderung des Sekundärkurvenparameters nach der Behandlung als ein Hinweis gewertet wird, dass der Patient positiv auf die Behandlung angesprochen hat.

**Revendications**

1. Procédé de détection d'un anticorps dans un échantillon de test comprenant un fluide corporel d'un sujet mammifère, le procédé comprenant les étapes consistant à :

(a) mettre en contact ledit échantillon de test avec une pluralité de quantités différentes d'un antigène spécifique pour ledit anticorps ;
(b) détecter la quantité de liaison spécifique entre ledit anticorps et ledit antigène ;
(c) tracer ou calculer une courbe de la quantité de ladite liaison spécifique versus la quantité d'antigène pour chaque quantité d'antigène utilisée à l'étape (a) ;
(d) calculer un paramètre de courbe secondaire à partir de la courbe tracée ou calculée à l'étape (c) ; et
(e) déterminer la présence ou l'absence dudit anticorps en se basant sur une combinaison de :

(i) la quantité de liaison spécifique entre ledit anticorps et ledit antigène déterminée à l'étape (b) ; et
(ii) ledit paramètre de courbe secondaire déterminé à l'étape (d).

2. Procédé de détection de deux anticorps ou plus dans un échantillon de test comprenant un fluide corporel d'un sujet mammifère, le procédé comprenant les étapes consistant à :

(a) mettre en contact ledit échantillon de test avec un panel comprenant deux ensembles d'antigènes ou plus, où chacun desdits antigènes dans ledit panel est spécifique pour au moins un desdits deux anticorps ou plus ;
(b) détecter des complexes desdits antigènes liés aux anticorps présents dans ledit échantillon de test ;
(c) pour chaque antigène, tracer ou calculer une courbe distincte de la quantité de ladite liaison spécifique versus la quantité d'antigène pour chaque quantité d'antigène utilisée à l'étape (a) ;
(d) calculer un paramètre de courbe secondaire pour chaque courbe tracée ou calculée à l'étape (c) ; et
(e) déterminer la présence ou l'absence desdits deux anticorps ou plus en se basant sur une combinaison de :

(i) la quantité de liaison spécifique entre ledit anticorps et ledit antigène déterminée à l'étape (b) ;
(ii) ledit paramètre de courbe secondaire déterminé à l'étape (d),

pour chacune des courbes tracées ou calculées à l'étape (c) .

**3.** Procédé selon la revendication 2, dans lequel :

(a) les deux antigènes ou plus du panel sont des antigènes distincts, et où le panel comprend facultativement deux variants antigéniques ou plus d'un ou plusieurs des antigènes distincts ; ou
(b) les deux antigènes ou plus du panel sont des variants antigéniques.

**4.** Procédé selon l'une quelconque des revendications 1 à 3, dans lequel le paramètre de courbe secondaire est sélectionné dans le groupe consistant en la pente, l'ordonnée à l'origine, l'aire sous la courbe (ASC), la pente maximum ou la constante de dissociation (Kd), et dans lequel le paramètre de courbe secondaire est facultativement calculé à partir d'une courbe de régression linéaire ou logarithmique.

**5.** Procédé selon l'une quelconque des revendications 1 à 3, dans lequel le paramètre de courbe secondaire est l'asymptote maximum, l'asymptote minimum, la pente (ou facteur de pente) ou le point d'inflexion d'une courbe logistique ajustée à chaque courbe tracée ou calculée à l'étape (c).

**6.** Procédé selon l'une quelconque des revendications 1 à 5, dans lequel l'antigène est une protéine ou un polypeptide survenant à l'état naturel, une protéine ou un polynucléotide recombinant(e), une protéine ou un polypeptide synthétique, un peptide synthétique, un peptidomimétique, un polysaccharide ou un acide nucléique.

**7.** Procédé selon l'une quelconque des revendications 1 à 6, dans lequel l'anticorps est un marqueur biologique d'un état pathologique ou d'une sensibilité à une maladie.

**8.** Procédé selon l'une quelconque des revendications 1 à 7, dans lequel l'anticorps est un auto-anticorps.

**9.** Procédé selon la revendication 8, dans lequel l'auto-anticorps est spécifique pour une protéine de marqueur tumoral, et où la protéine de marqueur tumoral est facultativement sélectionnée dans le groupe consistant en l'EGFR, MUC1, les protéines de transduction du signal/régulation du cycle cellulaire Myc, c-myc, L-myc2, p53, ras (ou Ras), BRCA1, BRCA2, APC, CA125, PSA, CEA, CA19.9, NY-ESO-1, le PSMA (antigène membranaire spécifique de la prostate), le PSCA (antigène des cellules souches de la prostate), EpCam (molécule d'adhésion des cellules épithéliales), HER2 (également connu en tant que c-erbB2), CAGE, CAGE1, CAGE2, des cytokératines, la recovérine, des kallikréines, des annexines, l'$\alpha$-foetoprotéine (AFP), GRP78, la mammaglobine, raf, la gonodotrophine chorionique humaine bêta b-HCG, l'antigène 4-5, SOX2, GBU4-5, MAGE, HuD, HE4, SALL4, l'alpha énolase, p62, RalA, la cycline B1, la calréticuline R, IMP1, des vitronectines (par ex. VTN10), SSX1, TMP21, NPC1L1C, TMOD1, CAMK1, RGS1, PACSIN1, RCV1, MAPKAPK3, la cycline E2, la cytokératine 8 (CK8), 18 (CK18), 19 (CK19), 20 (CK20), SCC, proGRP, l'amyloïde A sérique, l'alpha-1-antitrypsine, l'apolipoprotéine CIII, la thymosine $\beta$4, HDJ1 et HDGF.

**10.** Procédé selon la revendication 8, dans lequel l'auto-anticorps est :

(a) caractéristique de ou associé à une maladie auto-immune, telle que la polyarthrite rhumatoïde, le lupus érythémateux systémique (LES), la cirrhose biliaire primitive (CBP), la thyroïdite auto-immune, la thyroïdite de Hashimoto, la gastrite auto-immune, l'anémie pernicieuse, l'adrénalite auto-immune, la maladie d'Addison, l'hypoparathyroïdie auto-immune, le diabète auto-immun, la myasthénie grave, des troubles neurologiques paranéoplasiques tels que le syndrome myasthénique de Lambert-Eaton, une maladie intestinale inflammatoire, la sarcoïdose et l'hépatite auto-immune ; ou
(b) caractéristique de ou associé à une maladie rénale ou hépatique entraînant une insuffisance ou une défaillance de l'organe.

**11.** Procédé selon l'une quelconque des revendications 1-8, dans lequel l'anticorps est dirigé contre un épitope présent sur un tissu transplanté chez le sujet mammifère.

**12.** Utilisation d'un kit pour réaliser le procédé selon l'une quelconque des revendications précédentes, dans laquelle ledit kit comprend un ou plusieurs antigènes et un réactif capable de détecter des complexes desdits antigènes liés à des anticorps présents dans un échantillon de test comprenant un liquide corporel d'un sujet mammifère, et dans laquelle le kit comprend facultativement :

(a) deux variants antigéniques ou plus ; et/ou
(b) un moyen pour mettre en contact lesdits un ou plusieurs antigènes avec un échantillon de fluide corporel.

**13.** Utilisation du procédé selon la revendication 9 dans :

(a) le diagnostic, le pronostic ou la surveillance du cancer ;

(b) la détection d'une modification néoplasique précoce ou carcinogène précoce chez des patients asymptomatiques ;

(c) la détection d'une maladie récidivante chez un patient précédemment diagnostiqué comme porteur de cellules tumorales, lequel patient a reçu un traitement pour réduire le nombre desdites cellules tumorales ;

(d) l'identification de ces individus qui sont exposés à un risque augmenté de développer un cancer, tel que le cancer du poumon, le cancer du sein, le cancer de la vessie, le cancer de la prostate, le cancer colorectal, le cancer de l'ovaire ou un carcinome hépatocellulaire, dans une population d'individus asymptomatiques ;

(e) la détermination du profil des marqueurs tumoraux d'un individu souffrant d'un cancer, où ledit profil des marqueurs tumoraux est facultativement déterminé de manière séquentielle chez ledit individu en tant qu'indication de l'évolution de la maladie ;

(f) la prédiction de la réponse d'un individu souffrant d'un cancer à un traitement anticancéreux, tel qu'une résection chirurgicale, l'hormonothérapie, la chimiothérapie, la radiothérapie, une thérapie anti-facteur de croissance, l'immunothérapie ou la vaccination ;

(g) le diagnostic, le pronostic ou la surveillance du cancer ;

(h) le dépistage d'une population de sujets humains asymptomatiques pour identifier ces sujets qui sont exposés à un risque augmenté de développer un cancer, où les échantillons devant être testés en utilisant le procédé sont des échantillons de fluide corporel prélevés chez les sujets, et où les sujets présentant un taux élevé d'auto-anticorps et un paramètre de courbe secondaire altéré, par comparaison à des individus de contrôle normaux, sont identifiés comme exposés à un risque élevé de développer un cancer ;

(i) la détection d'une modification néoplasique précoce ou carcinogène précoce chez un sujet humain asymptomatique, où l'échantillon devant être testé en utilisant le procédé est un échantillon de fluide corporel prélevé chez le sujet, et où la présence d'un taux élevé d'auto-anticorps et d'un paramètre de courbe secondaire altéré, par comparaison à des individus de contrôle normaux, est considérée comme une indication d'une modification néoplasique précoce ou carcinogène précoce chez le sujet ;

(j) le dépistage d'une population de sujets humains asymptomatiques pour identifier ces sujets qui ont développé un cancer, où les échantillons devant être testés en utilisant le procédé sont des échantillons de fluide corporel prélevés chez les sujets, et où les sujets présentant un taux élevé d'auto-anticorps et un paramètre de courbe secondaire altéré, par comparaison à des individus de contrôle normaux, sont diagnostiqués comme souffrant d'un cancer ;

(k) le test d'une population de sujets humains symptomatiques pour identifier ces sujets qui ont développé un cancer, où les échantillons devant être testés en utilisant le procédé sont des échantillons de fluide corporel prélevés chez les sujets, et où les sujets présentant un taux élevé d'auto-anticorps et un paramètre de courbe secondaire altéré, par comparaison à des individus de contrôle normaux, sont diagnostiqués comme souffrant d'un cancer ;

(l) la surveillance de la progression du cancer ou d'une autre maladie néoplasique chez un patient, où l'échantillon devant être testé en utilisant le procédé est un échantillon de fluide corporel prélevé chez un patient humain, et où la présence d'un taux élevé d'auto-anticorps et d'un paramètre de courbe secondaire altéré, par comparaison à un contrôle normal, est considérée comme une indication de la présence du cancer chez le patient ;

(m) la détection d'une maladie récidivante chez un patient précédemment diagnostiqué comme souffrant d'un cancer, lequel patient a reçu un traitement anticancéreux pour réduire la quantité de cancer présent, où l'échantillon devant être testé en utilisant le procédé est un échantillon de fluide corporel prélevé chez le patient, et où la présence d'un taux augmenté d'auto-anticorps chez le patient et d'un paramètre de courbe secondaire altéré, par comparaison à un contrôle normal, est considérée comme une indication de récidive de la maladie ; ou

(n) l'évaluation du pronostic du cancer, où l'échantillon devant être testé en utilisant le procédé est un échantillon de fluide corporel prélevé chez un patient humain, et où la présence d'un taux élevé d'auto-anticorps et d'un paramètre de courbe secondaire altéré, par comparaison à un contrôle normal, est considérée comme une indication du pronostic du patient par rapport à son cancer.

**14.** Utilisation du procédé selon la revendication 7 ou la revendication 8 dans la détection du cancer, de préférence le cancer du poumon, du sein, de la vessie, colorectal, de la prostate, de l'ovaire ou un carcinome hépatocellulaire.

**15.** Utilisation du procédé selon la revendication 7 ou la revendication 8 dans la surveillance de la réponse d'un patient cancéreux humain à un traitement anticancéreux, où l'échantillon devant être testé en utilisant le procédé est un échantillon de fluide corporel prélevé chez le patient, et où une modification du taux d'auto-anticorps et une modification du paramètre de courbe secondaire après le traitement est considérée comme une indication du fait que le patient a répondu ou non au traitement, où le traitement est facultativement une résection chirurgicale, la vaccination, une thérapie anti-facteur de croissance ou de transduction d'un signal, la radiothérapie, une thérapie endocrine, une thérapie par un anticorps humain ou la chimiothérapie, et une modification du taux d'auto-anticorps et une modification du paramètre de courbe secondaire après le traitement est considérée comme une indication du fait que le patient a répondu positivement au traitement.

Figure 1A

EP 3 158 336 B1

**Figure 1B**

Curve fitted using the formula: F(x) = ((A-D)/(1+((x/C)^B))) + D
In this example A = 0.000, B = 1.733, C = 2.763, D = 0.102

EP 3 158 336 B1

Figure 2

Figure 3

A

EP 3 158 336 B1

Figure 4

**B**

Antibody/Antigen Binding (RU)

Secondary Curve Parameter

EP 3 158 336 B1

C

**D**

Figure 5

**C**

Secondary Curve Parameter

Antibody/Antigen Binding (RU)

F

**Figure 6**

A

C

EP 3 158 336 B1

Figure 7

EP 3 158 336 B1

**B**

C

EP 3 158 336 B1

**Figure 8**

**Ai**

Aii

Disease_Code
○ HCC
□ Matched Normal
× Benign

Antibody/Antigen Binding (OD)

Secondary Curve Parameter

**Bii**

**Ci**

**Cii**

**Dii**

EP 3 158 336 B1

Disease_Code

○ HCC
□ Matched Normal
✕ Benign

0.0050

0.75

3.00

2.00

1.00

.00

-1.00

Antibody/Antigen Binding (OD)

-.020000   .000000   .020000   .040000   .060000

Secondary Curve Parameter

EP 3 158 336 B1

**Eii**

*Antibody/Antigen Binding (OD)*

**Secondary Curve Parameter**

Disease_Code
○ HCC
□ Matched Normal
× Benign

0.025

0.7

**Fi**

Disease_Code
○ HCC
□ Matched Normal
✕ Benign

Antibody/Antigen Binding (OD)

Secondary Curve Parameter

0.015

0.3

Fii

# Figure 9

**Ai**

Aii

**Bi**

**Bii**

EP 3 158 336 B1

Ci

Disease_Code
○ HCC
□ Matched Normal
× Benign

Secondary Curve Parameter

Antibody/Antigen Binding (OD)

**Cii**

EP 3 158 336 B1

**Di**

**Dii**

EP 3 158 336 B1

**Ei**

Eii

Disease_Code
○ HCC
□ Matched Normal
✕ Benign

Antibody/Antigen Binding (OD) vs Secondary Curve Parameter

**Fii**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9958978 A
- WO 2006126008 A
- WO 2004044590 A
- WO 9958979 A
- WO 06126008 A
- WO 09081165 A

### Non-patent literature cited in the description

- **E. DIAMANDIS ; T. CHRISTOPOULUS.** Immunoassay. Academic Press, Inc, 1996
- **DOWNWARD et al.** *Nature,* 1984, vol. 307, 521-527
- **ROBERTSON et al.** *Archives of Pathology and Laboratory Medicine,* 2001, vol. 126, 177-81
- **BATRA, S. K. et al.** *Int. J. Pancreatology.,* 1992, vol. 12, 271-283
- **BLACKWOOD, E. M. et al.** *Molecular Biology of the Cell,* 1994, vol. 5, 597-609
- **NAU et al.** *Nature,* vol. 318 (6041), 69-73
- **MATLASHEWSKI, G. et al.** *EMBO J.,* 1984, vol. 3, 3257-3262
- **WOLF, D. et al.** *Mol. Cell. Biol.,* 1985, vol. 5, 1887-1893
- **CAPELLA, G. et al.** *Environ Health Perspectives,* 1991, vol. 93, 125-131
- **SCULLY, R. et al.** *PNAS,* 1997, vol. 94, 5605-10
- **SHARAN, S. K. et al.** *Nature,* 1997, vol. 386, 804-810
- **SU, L. K. et al.** *Cancer Res.,* 1993, vol. 53, 2728-2731
- **MUNEMITSU, S. et al.** *PNAS,* 1995, vol. 92, 3046-50
- **NOUWEN, E. J. et al.** *Differentiation,* 1990, vol. 45, 192-8
- **ROSENBERG, R. S. et al.** *Biochem Biophys Res Commun.,* 1998, vol. 248, 935-939
- **DUFFY, M.J.** *Clin Chem,* April 2001, vol. 47 (4), 624-30
- **HAGA, Y. et al.** *Clin Biochem,* 1989, vol. 22 (5), 363-8
- **CHEN, Y.-T. et al.** *Proc. Nat. Acad. Sci.,* 1997, vol. 94, 1914-1918
- **ISRAELI, R. S. et al.** *Cancer Res.,* 1993, vol. 53, 227-230
- **REITER, R. E. et al.** *Proc. Nat. Acad. Sci.,* 1998, vol. 95, 1735-1740
- **SZALA, S. et al.** *Proc. Nat. Acad. Sci.,* 1990, vol. 87, 3542-3546
- **COUSSENS, L. et al.** *Science,* 1985, vol. 230, 1132-1139
- **JAGER D et al.** *Cancer Res.,* 15 December 1999, vol. 59 (24), 6197-204
- **MASHINO K et al.** *Br J Cancer.,* 01 September 2001, vol. 85 (5), 713-20
- **MOLL R et al.** *Cell,* November 1982, vol. 31 (1), 11-24
- **BRAUN S et al.** *N Engl J Med.,* 2000, vol. 342, 525-533
- **MAEDA A et al.** *Cancer Res.,* 01 April 2000, vol. 60 (7), 1914-20
- **KIM H et al.** *Br J Cancer,* 2001, vol. 84, 643-650
- **YOUSEF GM et al.** *Tumor Biol,* 2002, vol. 23, 185-192
- **HUDELIST G et al.** *Breast Cancer Res Treat,* August 2004, vol. 86 (3), 281-91
- **STILLER D et al.** *Acta Histochem Suppl.,* 1986, vol. 33, 225-31
- **BLOCK TM et al.** *Proc Natl Acad Sci USA.,* 18 January 2005, vol. 102 (3), 779-84
- **HSU WM et al.** *Int J Cancer,* 01 March 2005, vol. 113 (6), 920-7
- **ZEHENTNER BK et al.** *Clin Chem.,* November 2004, vol. 50 (11), 2069-76
- **ZEHENTNER BK ; CARTER D.** *Clin Biochem.,* April 2004, vol. 37 (4), 249-57
- **CALLANS LS et al.** *Ann Surg Oncol.,* January 1995, vol. 2 (1), 38-42
- **PRATT MA et al.** *Mol Cell Biochem.,* December 1998, vol. 189 (1-2), 119-25
- **AYALA AR et al.** *Am J Reprod Immunol.,* April 1983, vol. 3 (3), 149-51
- **GREGORY JJ JR et al.** *Drugs,* April 1999, vol. 57 (4), 463-7
- **KRAUSE P et al.** *J Immunol Methods.,* December 2003, vol. 283 (1-2), 261-7
- **STEVANOVIC et al.** *Mammalian genome : official journal of the International Mammalian Genome Society,* 1994, vol. 5 (10), 640-2

- **KRAUSE et al.** *J Immunol Methods,* 2003, vol. 283, 261-7
- **SERRANO et al.** *International journal of cancer. Journal international du cancer,* 1999, vol. 83 (5), 664-9
- **SZABO et al.** *Cell,* 1991, vol. 67 (2), 325-33
- **AL-BARADIE et al.** *American journal of human genetics,* 2002, vol. 71 (5), 1195-9
- **LEE et al.** *Arthritis and rheumatism,* 2003, vol. 48 (7), 2025-35
- **ZHANG et al.** *The Journal of experimental medicine,* 1999, vol. 189 (7), 1101-10
- **BHULLAR et al.** *FEBS Letters,* 1990, vol. 260 (1), 48-52
- **PINES et al.** *The Journal of cell biology,* 1991, vol. 115 (1), 1-17
- **OPAS et al.** *Journal of cellular physiology,* 1991, vol. 149 (1), 160-71
- **NIELSEN et al.** *Molecular and cellular biology,* 1999, vol. 19 (2), 1262-70
- **HAYMAN et al.** *Proceedings of the National Academy of Sciences of the United States of America,* 1983, vol. 80 (13), 4003-7
- **CREW et al.** *The EMBO journal,* 1995, vol. 14 (10), 2333-40
- **BLUM et al.** *Journal of Biological Chemistry,* 1996, vol. 271 (29), 17183-17189
- **DAVIES et al.** *Genomics,* 2000, vol. 65 (2), 137-45
- **SUNG et al.** *The Journal of biological chemistry,* 1992, vol. 267 (4), 2616-21
- **HARIBABU et al.** *The EMBO journal,* 1995, vol. 14 (15), 3679-86
- **WATSON et al.** *Nature,* 1996, vol. 383 (6596), 172-5
- **QUALMANN et al.** *Molecular biology of the cell,* 1999, vol. 10 (2), 501-13
- *Investigative ophthalmology & visual science,* vol. 34 (1), 81-90
- **SITHANANDAM et al.** *Molecular and cellular biology,* 1996, vol. 16 (3), 868-76
- **LAUPER et al.** *Oncogene,* 1998, vol. 17 (20), 2637-43
- **KATO et al.** *Cancer,* 1977, vol. 40 (4), 1621-1628
- **ROSENTHAL et al.** *Journal of immunology,* 1976, vol. 116 (5), 1415-8
- **LAURELL et al.** *Scandinavian Journal of Clinical & Laboratory Investigation,* 1963, vol. 15 (2), 132-140
- **OHTSUKA.** *Biochemical and biophysical research communications,* 1993, vol. 197 (1), 235-40
- **ZHOU et al.** Overexpressed HDGF as an independent prognostic factor is involved in poor prognosis in Chinese patients with liver cancer. *Diagn Pathol,* 2010, vol. 5, 58
- **BASELGA, J. ; D. TRIPATHY et al.** *J Clin Oncol.,* 1996, vol. 14 (3), 737-744
- **MURRAY et al.** *Ann Oncol.,* 21 August 2010, vol. 8, 1687-93